(19) Europäisches Patentamt
European Patent Office
Office européen des brevets

(11) EP 3 981 777 A1

(12) EUROPEAN PATENT APPLICATION

(43) Date of publication:
**13.04.2022 Bulletin 2022/15**

(21) Application number: **20200905.6**

(22) Date of filing: **08.10.2020**

(51) International Patent Classification (IPC):
***C07H 15/26*** (2006.01) ***A61P 31/12*** (2006.01)
***A61P 31/16*** (2006.01) ***A61P 31/18*** (2006.01)

(52) Cooperative Patent Classification (CPC):
**C07H 15/26; A61P 31/12; A61P 31/16; A61P 31/18**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Universität Basel**
  **4001 Basel (CH)**
- **Ernst, Beat**
  **4312 Magden (CH)**

(72) Inventors:
- **Ernst, Beat**
  **4312 Magden (CH)**
- **Aliu, Butrint**
  **4057 Basel (CH)**
- **Cramer, Jonathan**
  **4123 Allschwil (CH)**

(74) Representative: **Vossius & Partner Patentanwälte Rechtsanwälte mbB**
**Siebertstrasse 3**
**81675 München (DE)**

# (54) ANTIVIRAL COMPOUNDS AND POLYMERS

(57) The present invention refers to a compound having the formula (I):

$$OS\text{-}A\text{-}B\text{-}(CH_2)_q\text{-}SH \qquad (I)$$

to a polymer comprising a multitude of compounds according to formula (I) as well as pharmaceutical compositions comprising the same that can be used in treatment, prevention and/or alleviation of viral infection. The instant compounds, polymers and pharmaceutical compositions are particularly useful in treating, preventing and alleviating a viral infection caused by SARS-Coronavirus-2.

EP 3 981 777 A1

## Description

### Field of the invention

The present invention relates to novel compounds, polymers and pharmaceutical compositions comprising the same that can be used in treatment, prevention and alleviation of viral infections. The instant compounds, polymers and compositions are particularly useful in treating, preventing and alleviating viral infections caused by SARS-Coronavirus-2.

### Background information

Envelope glycoproteins of a variety of viruses are densely covered with host-derived carbohydrates. These glycan structures shield viruses from antibody-mediated immune responses and enable attachment to the host's lectins. One of these lectins is the C-type lectin receptor (CLR) DC-SIGN (dendritic cell-specific intercellular adhesion molecule 3 grabbing non-integrin), a surface receptor expressed by innate immune cells. It has been demonstrated that DC-SIGN acts as an entry receptor for pathogens and plays a detrimental role in the pathology of many viral infections by promoting virus dissemination and immune evasion. High-mannose glycan epitopes on envelope glycoproteins of epidemic and pandemic viruses such as HIV, Ebola, influenza A, hepatitis C, SARS, zika, dengue, and others, have been shown to enable DC-SIGN mediated attachment and internalization, either for *cis*-infection of DC-SIGN$^+$ cells or for *trans*-infection of other cell types.

In the context of the ongoing SARS-CoV-2 pandemic, increasing evidence points to a possible involvement of DC-SIGN in virus attachment to innate immune cells. The SARS-CoV-2 spike protein is heavily glycosylated with high mannose and complex N-glycans, which potentially could serve the attachment to host lectins. In fact, several groups have demonstrated that these glycans are indeed recognized with picomolar affinity by various CLRs such as DC-SIGN and the closely related DC-SIGNR. The interaction of the viral spike glycoprotein leads to internalization in DC-SIGN-presenting cells, strongly implicating DC-SIGN as an ACE2-independent entry receptor for SARS-CoV-2. Infection of innate immune cells by this mechanism could contribute to the exuberant immune response in severe COVID-19. This is consistent with the fact that DC-SIGN expression levels were increased in severe COVID-19 patients with elevated amounts of proinflammatory monocyte-derived macrophages, inflammatory cytokines and chemokines. Differences in SARS-CoV-2 virulence due to mutation in the viral spike glycoprotein, such as the prominent D614G mutation, have been attributed to potential variations in glycosylation, resulting in differences in the interaction with DC-SIGN and DC-SIGNR. Furthermore, a recent study concluded that genetic variants in the *ABO* gene locus correlate with DC-SIGN expression levels and that increased DC-SIGN expression represents a genetic risk factor for severe COVID-19, indicating a central role of the innate immune system in this disease.

Inhibition of DC-SIGN-mediated attachment of viral particles to innate immune cells, thus, represents a promising strategy for the development of antiviral drugs. In the past, this strategy has been investigated for the treatment of HIV, Ebola, zika, and dengue infections. In view of the recent emergence of the SARS-CoV-2 pandemic, DC-SIGN-targeted antivirals could represent a promising host-directed treatment option for COVID-19. Importantly, metabolic pathways responsible for glycosylation of viral proteins, as well as the targeted glycan receptors are evolutionarily conserved in the host genome. It can therefore be assumed, that the pharmacological interference in this mechanism with antiviral therapeutics is non-sensitive to viral resistance mutations and additionally provides the possibility for an early containment of infections from newly emerging virus strains with pandemic potential.

### Summary of the invention

It was an object of the present invention to provide novel compounds for treating and/or preventing and/or alleviating viral infection in a subject in need thereof. In particular, the compounds should exhibit broad antiviral activity and be safe for application in subjects over extended periods of time.

The problem is solved by the embodiments described herein and as characterized in the claims.

The present inventors have surprisingly found that the compounds of the present invention, the polymers of the present invention and the pharmaceutical compositions of the present invention efficiently block the interaction of DC-SIGN$^+$ cells with viral glycoproteins, and are thus suitable for treating viral infections, in particular caused by HIV, Ebola virus, and SARS-CoV-2 (see Examples 3 and 4). The present inventors have further shown that the compounds, the polymers and the pharmaceutical compositions of the present invention are readily cleared by the target cells and CD206$^+$ macrophages, and thus, show great potential for compatibility with prolonged application in a therapeutic setting (see Example 5 and Example 6).

The present invention can be summarized in the following embodiments.

In a first embodiment, the present invention relates to a compound according to the formula (I):

$$OS\text{-}A\text{-}B\text{-}(CH_2)_q\text{-}SH \qquad (I)$$

or a tautomer, pharmaceutically acceptable salt, hydrate, solvate or polymorph thereof, wherein:

OS is a glycan moiety comprising up to 9 mannose monomers, wherein OS is attached to A preferably through the reducing end of one of its monomers, preferably through an O, NH, S, or $CH_2$ group;

As understood in the present invention, the definition of "OS" preferably includes the O, NH, S, or $CH_2$ group through which the glycan moiety comprising up to 9 mannose monomers is bound to A.

A is $C_1$-$C_7$-alkylene, $C_1$-$C_4$-alkylene-$(OCH_2CH_2)_p$O-$C_1$-$C_4$-alkylene, or $C_1$-$C_7$-alkylene-$R^b$-, wherein -$R^b$- is an optionally substituted arylene or an optionally substituted heteroarylene, and wherein p is 0 to 6, preferably p is 1, 2, or 3, and more preferably p is 1;
B is -NHC(O)-, -S-, -$CH_2$-, -O-, -NH- or

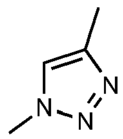,

and
q is 0 to 6, preferably q is 1, 2, 3 or 4, and further preferably q is 1 or 2.

In a further embodiment, the present invention relates to a compound of formula (I), wherein the OS moiety is selected from:

α/β-D-mannopyranosyl,
α-D-mannopyranosyl-(1→2)-α/β-D-mannopyranosyl,
α-D-mannopyranosyl-(1→3)-α/β-D-mannopyranosyl,
α-D-mannopyranosyl-(1→4)-α/β-D-mannopyranosyl,
α-D-mannopyranosyl-(1→6)-α/β-D-mannopyranosyl,
α-D-mannopyranosyl-(1→2)-α-D-mannopyranosyl-(1→2)-α/β-D-mannopyranosyl,
α-D-mannopyranosyl-(1→2)-α-D-mannopyranosyl-(1→3)-α/β-D-mannopyranosyl,
α-D-mannopyranosyl-(1→2)-α-D-mannopyranosyl-(1→6)-α/β-D-mannopyranosyl,
α-D-mannopyranosyl-(1→3)-α-D-mannopyranosyl-(1→6)-α/β-D-mannopyranosyl,
α-D-mannopyranosyl-(1→3)-[α-D-mannopyranosyl-(1→6)]-α/β-D-mannopyranosyl,
α-D-mannopyranosyl-(1→2)-α-D-mannopyranosyl-(1→2)-α-D-mannopyranosyl-(1→3)-α/β-D-mannopyranosyl,
α-D-mannopyranosyl-(1→2)-α-D-mannopyranosyl-(1→3)-α-D-mannopyranosyl-(1→6)-α/β-D-mannopyranosyl,
α-D-mannopyranosyl-(1→2)-α-D-mannopyranosyl-(1→6)-α-D-mannopyranosyl-(1→6)-α/β-D-mannopyranosyl,
α-D-mannopyranosyl-(1→2)-α-D-mannopyranosyl-(1→6)-[α-D-mannopyranosyl-(1→3)]-α/β-D-mannopyranosyl,
α-D-mannopyranosyl-(1→2)-α-D-mannopyranosyl-(1→3)-[α-D-mannopyranosyl-(1→6)]-α/β-D-mannopyranosyl,
α-D-mannopyranosyl-(1→6)-α-D-mannopyranosyl-(1→6)-[α-D-mannopyranosyl-(1→3)]-α/β-D-mannopyranosyl,
α-D-mannopyranosyl-(1→3)-α-D-mannopyranosyl-(1→6)-[α-D-mannopyranosyl-(1→3)]-α/β-D-mannopyranosyl,
and
α-D-mannopyranosyl-(1→6)-[α-D-mannopyranosyl-(1→3)]-α-D-mannopyranosyl-(1→6)-α/β-D-mannopyranosyl.

It is to be understood that in these examples, the mannopyranosyl is bound to A via an -O- group.

In a further embodiment, the present invention relates to a compound according to formula (Ib):

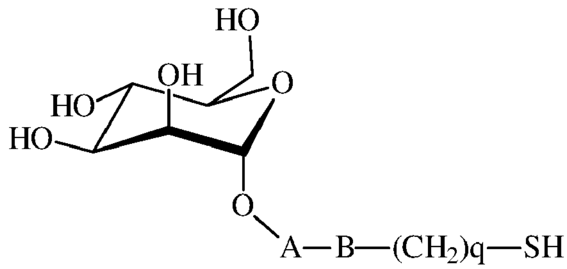

(Ib)

or an anomer, tautomer, pharmaceutically acceptable salt, hydrate, solvate or polymorph thereof, wherein:

A is $C_1$-$C_7$-alkylene, $C_1$-$C_4$-alkylene-$(OCH_2CH_2)_pO$-$C_1$-$C_4$-alkylene, or $C_1$-$C_7$-alkylene-$R^b$-, wherein -$R^b$- is an optionally substituted arylene or an optionally substituted heteroarylene, and wherein p is 0 to 6, preferably p is 1, 2, or 3, and more preferably p is 1;

B is -NHC(O)-, -S-, -$CH_2$-, -O-, -NH- or

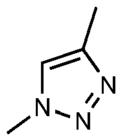

,

and

q is 0 to 6, preferably q is 1, 2, 3 or 4, and further preferably q is 1 or 2.

In a further particular embodiment, the present invention relates to a compound of formula (I), or a tautomer, pharmaceutically acceptable salt, hydrate, solvate or polymorph thereof, wherein A is $C_1$-$C_7$-alkylene, preferably -$CH_2CH_2CH_2$-.

In again a further particular embodiment, the present invention relates to a compound of formula (I), or a tautomer, pharmaceutically acceptable salt, hydrate, solvate or polymorph thereof, wherein B is -NHC(O)-.

In again a further particular embodiment, the present invention relates to a compound of formula (I), or a tautomer, pharmaceutically acceptable salt, hydrate, solvate or polymorph thereof, wherein q is 3.

In a further embodiment, the present invention relates to a compound according to formula (Ic):

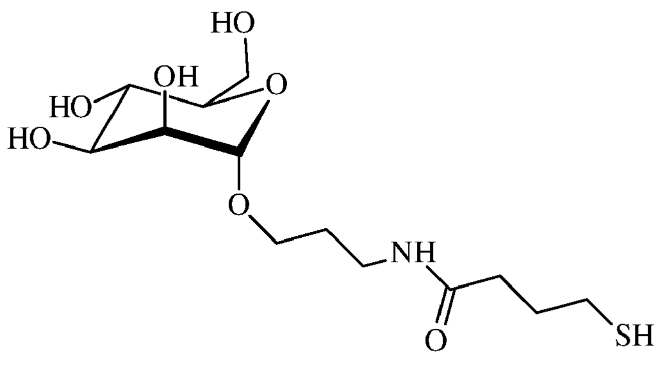

(Ic)

or an anomer, tautomer, pharmaceutically acceptable salt, hydrate, solvate or polymorph thereof.

In another embodiment, the present invention relates to a polymer comprising a polymer backbone and a multitude of compounds according to formula (I), wherein said compounds are connected to the polymer backbone through the -SH group.

In a further particular embodiment, the present invention relates to a polymer comprising a polymer backbone and a multitude of compounds according to formula (I), wherein the polymer backbone is an α-amino acid polymer, an acrylic acid or methacrylic acid polymer or copolymer, an N-vinyl-2-pyrrolidone-vinyl alcohol copolymer, a chitosan polymer, or a polyphosphazene polymer.

In again a further particular embodiment, the present invention relates to a polymer comprising a polymer backbone and a multitude of compounds according to formula (I), wherein the polymer backbone is an α-amino acid polymer, and wherein said α-amino acid is lysine, ornithine, glutamic acid, aspartic acid, histidine, cysteine, or serine, and wherein the polymer is optionally PEGylated. It is to be understood that the α-amino acid polymer may be a homopolymer or copolymer of any of the mentioned amino acids.

In a particular embodiment, the present invention relates to a polymer comprising a multitude of compounds according to formula (I), wherein the polymer backbone is poly-lysine.

In a further particular embodiment, the present invention relates to a polymer comprising a multitude of compounds according to formula (I), wherein the weight average molecular weight of the polymer is at least 30 000 Da, preferably wherein the weight average molecular weight of the polymer is at least 90 000 Da, more preferably wherein the weight average molecular weight of the polymer is between 90 000 Da and 500 000 Da.

Unless indicated otherwise, any average molecular weights mentioned herein are weight average molecular weights determined by NMR method, as defined herein.

In again a further particular embodiment, the present invention relates to a polymer comprising a multitude of compounds according to formula (I), wherein the percentage of loading of the compound according to formula (I) on the polymer backbone is between 10 and 90%, preferably between 20 and 80%, more preferably between 30 and 70%. Preferably, the remainder of the polymer backbone (i.e. more specifically the side chains of the amino acid repeating units) is capped with a group selected from 2,3-dihydroxy-propylthioacetyl, 2-thioacetic acid, 3-thiopropionic acid, 2-thioethanesulfonic acid, mercaptosuccinic acid, cysteine, cysteamine, N,N-dimethyl cysteamine and $(HOCH_2)_3CNHC(O)(CH_2)_{1-6}SH$.

In a further embodiment, the present invention relates to a polymer comprising a multitude of compounds according to formula (I), wherein the polymer exhibits a hydrodynamic radius of at least 3 nm as measured by analytical ultracentrifugation, preferably wherein the polymer exhibits a hydrodynamic radius of at least 10 nm as measured by analytical ultracentrifugation,

and/or

wherein the polymer exhibits a hydrodynamic diameter of at least 9 nm as measured by dynamic light scattering, preferably wherein the polymer exhibits a hydrodynamic diameter of at least 19 nm as measured by dynamic light scattering.

In a further particular embodiment, the present invention relates to a polymer comprising a multitude of compounds according to formula (I), the polymer comprising x repeating units having the following formula:

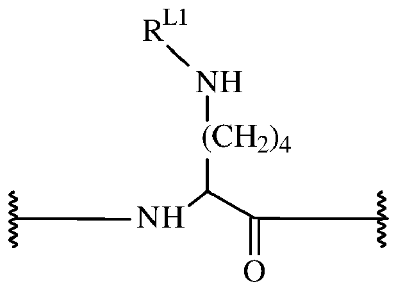

or an anomer, tautomer, pharmaceutically acceptable salt, hydrate, solvate or polymorph thereof, wherein $R^{L1}$ is:

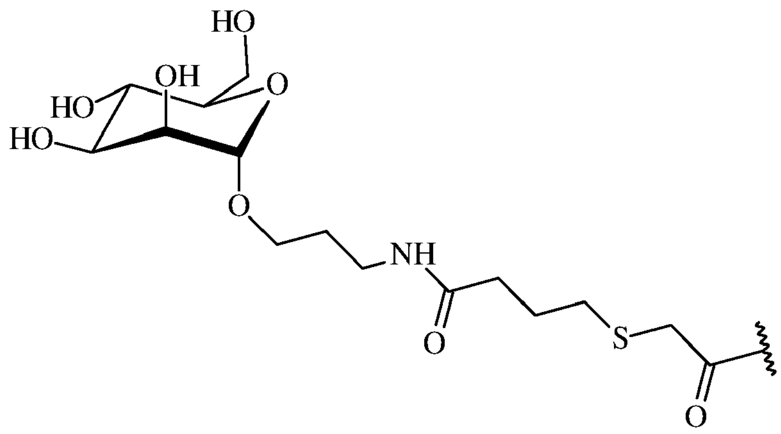

wherein the total number of repeating units n is at least 100, preferably n is at least 250, more preferably n is in the range from 250 to 800; and

wherein x/n is between 10 and 90%, preferably between 20 and 80%, more preferably between 30 and 70%. It is to be understood that the polymer is preferably a polyamide.

In another embodiment, the present invention relates to a pharmaceutical composition comprising the compound of the present invention, or a polymer of the present invention, and at least one pharmaceutically acceptable carrier.

In another embodiment, the present invention relates to the compound of the present invention, the polymer of the present invention, or the pharmaceutical composition of the present invention, for use as a medicament.

In a particular embodiment, the present invention relates to the compound of the present invention, the polymer of the present invention, or the pharmaceutical composition of the present invention for use in the treatment and/or prevention and/or alleviation of a viral infection.

In a further embodiment, the present invention relates to the compound for use of the present invention, the polymer for use of the present invention, or the pharmaceutical composition for use of the present invention, wherein the viral infection is caused by a virus characterized by mannose-comprising glycan epitopes on envelope glycoproteins or glycolipids.

In a further particular embodiment, the present invention relates to the compound for use of the present invention, the polymer for use of the present invention, or the pharmaceutical composition for use of the present invention, wherein the viral infection is caused by a virus selected from SARS-Coronavirus 2, SARS-Coronavirus, MERS-Coronavirus, HIV, influenza A virus, hepatitis C virus, zika virus, dengue virus, Ebola virus, west-Nile virus, Marburg virus, chikungunya virus, and Japanese encephalitis virus.

In again a further embodiment, the present invention relates to the compound for use of the present invention, the polymer for use of the present invention, or the pharmaceutical composition for use of the present invention, wherein the compound, the polymer, or the pharmaceutical composition interferes with DC-SIGN or DC-SIGNR-mediated attachment and/or internalization of viral particles.

In again a further particular embodiment, the present invention relates to the compound for use of the present invention, the polymer for use of the present invention, or the pharmaceutical composition for use of the present invention, wherein the compound of the invention, the polymer of the invention, or the pharmaceutical composition of the invention is administered through pulmonary administration route or intravenous administration route.

## Definitions

Within the meaning of the present application the following definitions apply:

As understood herein, an $\alpha$-amino acid is a proline or a compound containing both - $NH_2$ group and -COOH group. Preferably, the compound containing both -$NH_2$ group and -COOH group is a compound containing a -$CH(NH_2)COOH$ moiety. Preferably, the compound containing -$CH(NH_2)COOH$ moiety is one of twenty standard amino acids, known to the skilled person, or an ornithine.

As understood herein, an "anomer" is an epimer at the hemiacetal/hemiketal carbon in a cyclic saccharide. The said carbon atom is also referred to as anomeric carbon atom. As understood herein, an "epimer" is a stereoisomer that differs in configuration at any single one stereogenic center.

As understood herein, the term "alkyl" refers to a saturated straight or branched organic moiety consisting of carbon and hydrogen atoms. Examples of suitable alkyl groups have 1 to 6 carbon atoms, preferably 1 to 4 carbon atoms, and include methyl, ethyl, propyl, isopropyl, n-butyl, t-butyl and isobutyl. An alkyl group referred to as $C_{1-4}$ alkyl is an alkyl group having 1 to 4 carbon atoms.

As understood herein, "alkylene" is a bivalent radical corresponding to an alkyl group as defined herein, from which one hydrogen radical has been removed.

The term "aryl" preferably refers to an aromatic monocyclic ring containing 5 or 6 carbon atoms, an aromatic bicyclic ring system containing 10 carbon atoms or an aromatic tricyclic ring system containing 14 carbon atoms. Examples are phenyl, naphthyl or anthracenyl, preferably phenyl. As defined herein, aryl can be optionally substituted. Suitable optional substituent is selected from halogen, hydroxy, alkyl and haloalkyl. Aryl as defined herein may be substituted with one or more optional substituents.

The term "arylene" relates to a bivalent radical obtained from the aryl group as defined herein, from which a hydrogen radical was removed. Thus, a preferred example of an arylene is phenylene.

The expression "between ... to ..." is herein understood as also encompassing the numbers referred to. For example, between 1 and 10 also includes the values 1 and 10.

The term "comprising" as used in the context of a "polymer comprising a polymer backbone and a multitude of compounds according to formula (I)" as used herein refers to a situation, wherein, formally, for each compound according to formula (I), a hydrogen radical in the -SH group of each compound according to formula (I) and typically one hydrogen radical of the polymer backbone are removed, and the resulting forms (i.e. radicals) of the compound according to formula (I) and the polymer backbone are combined together.

As understood herein, "glycan" relates to a mono-, oligo-, or polysaccharide, that is a compound comprising one/several monosaccharide monomers linked by a glycosidic bond. The term glycan may also refer to an oligo/polysaccharide portion of a glycoconjugate. Glycosidic bond is understood herein as a bond formed between anomeric carbon of one monosaccharide moiety and an -O- moiety of another monosaccharide moiety. An oligo- or polysaccharide is herein understood as a compound comprising more than one monosaccharide moiety linked by glycosidic bond(s).

As understood herein, the term "pyranose" refers to a structure adopted by a monosaccharide comprising a six membered ring made of five carbon atoms and one oxygen atom. A non-limiting example of pyranose is mannopyranose.

Unless indicated otherwise, any average molecular weights mentioned herein are preferably weight average molecular weights determined by SEC-MALS or NMR. In the case of polymer backbones, the weight average molecular weight is preferably determined by SEC-MALS. In the case of polymers of the present invention, the weight average molecular weight is preferably determined by NMR, as defined herein.

The term "haloalkyl" is herein understood as an alkyl wherein one or more hydrogens are substituted with halogen(s).

The term "halogen" is understood herein as F, Cl, Br or I.

The term "heteroaryl" preferably refers to a five or six-membered aromatic ring containing 1, 2, 3, or 4 (for the five membered ring) or 1, 2, 3, 4, or 5 (for the six membered ring) of the same or different heteroatoms, wherein the heteroatoms are selected from O, N and S. Examples of the heteroaryl group include furanyl, thiophenyl, pyrrolyl, oxazolyl, isoxazolyl, isothiazolyl, thiazolyl, pyrazolyl, imidazolyl, 1,2,4-triazolyl, 1,2,3-triazolyl, 1,2,5-oxadiazolyl, 1,2,3-oxadiazolyl, 1,3,4-thiadiazolyl, 1,2,5-thiadiazolyl, tetrazolyl, pyridinyl, pyridazinyl, pyrimidinyl and pyrazinyl.

As understood herein, the term "heteroarylene" relates to a bivalent radical obtained from the heteroaryl group as defined herein, from which a hydrogen radical was removed. Thus, a preferred example of a "heteroarylene" is a 1,2,3-triazolylene.

As used herein, the term "multitude" is to be understood as plurality, in other words more than one.

As understood herein, the term "optionally substituted" relates to a situation, wherein one or more hydrogen radical(s) in, for example, alkyl, aryl or heteroaryl, is formally removed and replaced with another substituent. The substituent as defined herein may for example be a halogen, a hydroxy group, a cyano group, a carboxylic group, an alkyl group, an aryl group, a heteroaryl group.

As understood herein, the term "reducing end of saccharide/sugar" relates to an anomeric carbon atom in a monosaccharide or an oligosaccharide or a polysaccharide. When referring to the reducing end of (mono/oligo/poly)saccharide-yl moiety, an anomeric carbon that is not substituted with a saccharide moiety is meant.

Compounds of the present invention having one or more optically active carbons can exist as racemates and racemic mixtures, stereoisomers (including diastereomeric mixtures and individual diastereomers, enantiomeric mixtures and single enantiomers, mixtures of conformers and single conformers), tautomers, atropisomers, and rotamers. All isomeric forms are included in the present invention. Compounds described in this invention containing olefinic double bonds include E and Z geometric isomers, unless stated otherwise. Also included in this invention are all salt forms, polymorphs, hydrates and solvates.

If a compound or moiety is referred to as being "optionally substituted" it can in each instance include one or more of the indicated substituents, wherein the substituents can be the same or different.

The term "polymorphs" refers to the various crystalline structures of the compounds of the present invention. This may include, but is not limited to, crystal morphologies (and amorphous materials) and all crystal lattice forms. Salts of the present invention can be crystalline and may exist as more than one polymorph.

Solvates, hydrates as well as anhydrous forms of the salt or the free compound are also encompassed by the invention. The solvent included in the solvates is not particularly limited and can be any pharmaceutically acceptable solvent. Examples include water and $C_{1-4}$ alcohols (such as methanol or ethanol).

"Pharmaceutically acceptable salts" are defined as derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as, but not limited to, hydrochloric, hydrobromic, sulfuric, sulfamic, phosphoric, nitric and the like; and the salts prepared from organic acids such as, but not limited to, acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Organic solvents include, but are not limited to, nonaqueous media like ethers, ethyl acetate, ethanol, isopropanol, or acetonitrile. Lists of suitable salts can be found in Remington's Pharmaceutical Sciences, 18th ed., Mack Publishing Company, Easton, PA, 1990, p. 1445, the disclosure of which is hereby incorporated by reference.

"Pharmaceutically acceptable" is defined as those compounds, materials, compositions, and/or dosage forms which are, within the scope of sound medical judgment, suitable for use in contact with the tissues of human beings and animals without excessive toxicity, irritation, allergic response, or other problem or complication commensurate with a reasonable benefit/risk ratio.

The terms "DC-SIGN" means the C-type lectin receptor DC-SIGN, as well as all related and analogous receptors, such as DC-SIGNR.

**Brief description of figures**

**Figure 1** represents normalized scattering intensity distribution for Man-PLL **1a-d** as determined by dynamic light scattering.

**Figure 2** Normalized diffusion-deconvoluted differential sedimentation coefficient distributions (c(s) distributions) for **1a-d** determined by sedimentation velocity AUC experiments. The magnitude of c(s) at a given S value gives the proportion of the total material in that sample having that S value.

**Figure 3** depicts inhibition of viral glycoprotein attachment to DC-SIGN$^+$ B-THP-1 cells. Cells were incubated with Man-PLL **1a-d** in the presence of A) Cy5 labeled SARS-CoV-2 spike glycoprotein S1 subunit (10 nM), B) ebola glycoprotein (50 nM), and C) HIV gp120 (10 nM). Mean fluorescence intensity determined by flow cytometry was normalized before global fitting to a four parameter Hill model. Error bars represent the standard deviation from three independent experiments.

**Figure 4** shows inhibition of the DC-SIGN receptor on B-THP-1 cells by **1d.** A) Incubation of the cells with 100 nM **1d** lead to a sustained blocking of the DC-SIGN receptor for at least 6 hours, thereby successfully preventing the binding of Cy5 labeled SARS-CoV-2 spike glycoprotein S1 subunit (10 nM). B) Pre-treatment of the cells with 100 nM **1d** for 30 min showed a time dependent recovery of the receptor with a significantly decreased binding availability for at least 6h. Results are shown as mean with standard deviation from three independent experiments (one-way ANOVA, $*P \leq 0.05$, $**P \leq 0.01$, $***P \leq 0.001$, $****P \leq 0.0001$).

**Figure 5** demonstrates DC-SIGN mediated uptake and degradation of fluorescently labeled Man-$PLL_{400}$ **13** into B-THP-1 cells. DC-SIGN$^+$ B-THP-1 cells and control B-THP-1 cells were incubated with 100 nM rhodamine 6G labeled Man-$PLL_{400}$ **13** or 100 nM rhodamine 6G labeled $PLL_{400}$ **14.** A) The internalization was quantified by measuring the pH-dependent mean fluorescence intensity with flow cytometry, showing the highly specific and time-dependent uptake of **13** by DC-SIGN$^+$ B-THP-1 cells in comparison to control B-THP-1 cells and control **14.** Furthermore, after increasing fluorescence intensity over time, the signal disappeared after 24 h, highlighting the targeted uptake and degradation. B) Representative images recorded with confocal microscopy reveal the time dependent uptake of **13** in acidic cell compartments of DC-SIGN$^+$ B-THP-1 cells

and the effective removal after 24 h. Error bars represent the standard deviation from three independent experiments.

**Figure 6** A-D: $^1$H NMR spectra of compounds **1a-d**, respectively. The signals due to protons relevant for calculations of polymer loading with compound according to formula (I) are shown.

**Detailed description of the invention**

The compounds of the present invention will be described in the following. It is to be understood that all possible combinations of the following definitions are also envisaged.

In one embodiment, the present invention relates to a compound according to formula (I):

$$OS\text{-}A\text{-}B\text{-}(CH_2)_q\text{-}SH \qquad (I)$$

or a tautomer, pharmaceutically acceptable salt, hydrate, solvate or polymorph thereof.

OS is an oligosaccharide-yl moiety. In certain embodiments, OS is attached to A through the reducing end of one of its monomers. In other words, A is attached to the anomeric carbon of the sugar moiety of OS, preferably through a -O-, -NH-, -S-, or - $CH_2$-group. In preferred embodiments of the present invention, A is attached to the anomeric carbon of the sugar moiety of OS, preferably through a -O- group.

In certain embodiments of the present invention, the OS moiety is a substructure (obtained by replacing selected mono- or oligosaccharide-yl moieties with H radical) of the oligosaccharide-yl according to the formula (II):

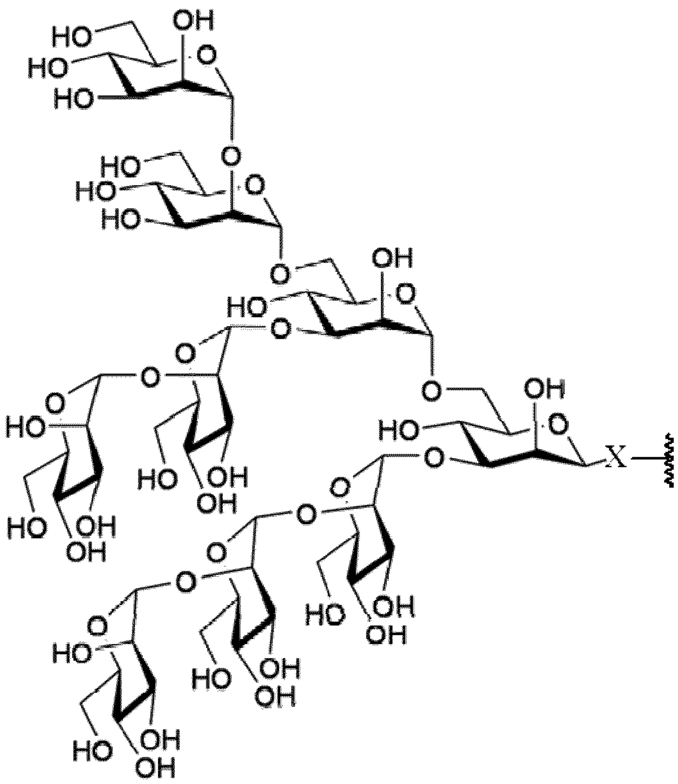

(II)

or the oligosaccharide-yl obtained by anomerization of the mannopyranose ring at the right side of the formula. X is -O-, -NH-, -S-, or -$CH_2$-. Preferably, X is -O-.

In certain embodiments of the present invention, the OS moiety is selected from α-D-mannopyranosyl-(1→2)-α/β-D-mannopyranosyl, α-D-mannopyranosyl-(1→3)-α/β-D-mannopyranosyl, α-D-mannopyranosyl-(1→4)-α/β-D-mannopyranosyl, α-D-mannopyranosyl-(1→6)-α/β-D-mannopyranosyl, α-D-mannopyranosyl-(1→2)-α-D-mannopyranosyl-(1→2)-α/β-D-mannopyranosyl, α-D-mannopyranosyl-(1→2)-α-D-mannopyranosyl-(1→3)-α/β-D-mannopyranosyl, α-D-mannopyranosyl-(1→2)-α-D-mannopyranosyl-(1→6)-α/β-D-mannopyranosyl, α-D-mannopyranosyl-(1→3)-α-D-mannopyranosyl-(1→6)-α/β-D-mannopyranosyl, α-D-mannopyranosyl-(1→3)-[α-D-mannopyranosyl-(1→6)]-α/β-D-mannopyranosyl, α-D-mannopyranosyl-(1→2)-α-D-mannopyranosyl-(1→2)-α-D-mannopyranosyl-(1→3)-α/β-D-mannopyranosyl, α-D-mannopyranosyl-(1→2)-α-D-mannopyranosyl-(1→3)-α-D-mannopyranosyl-(1→6)-α/β-D-mannopyranosyl, α-D-mannopyranosyl-(1→2)-α-D-mannopyranosyl-(1→6)-α-D-mannopyranosyl-(1→6)-α/β-D-mannopyranosyl, α-D-mannopyranosyl-(1→2)-α-D-mannopyranosyl-(1→6)-[α-D-mannopyranosyl-(1→3)]-α/β-D-mannopyranosyl, α-D-mannopyranosyl-(1→2)-α-D-mannopyranosyl-(1→3)-[α-D-mannopyranosyl-(1→6)]-α/β-D-mannopyranosyl, α-D-mannopyranosyl-(1→6)-α-D-mannopyranosyl-(1→6)-[α-D-mannopyranosyl-(1→3)]-α/β-D-mannopyranosyl, α-D-mannopyranosyl-(1→3)-α-D-mannopyranosyl-(1→6)-[α-D-mannopyranosyl-(1→3)]-α/β-D-mannopyranosyl, and α-D-mannopyranosyl-(1→6)-[α-D-mannopyranosyl-(1→3)]-α-D-mannopyranosyl-(1→6)-α/β-D-mannopyranosyl.

In certain embodiments of the invention, the OS moiety comprises 1 mannose monomer. In certain embodiments of the invention, the OS moiety comprises α/β-D-mannopyranosyl. In certain embodiments of the invention, the OS moiety is α/β-D-mannopyranosyl.

In certain embodiments of the invention, the OS moiety comprises 2 mannose monomers. In certain embodiments of the invention, the OS moiety is selected from α-D-mannopyranosyl-(1→2)-α/β-D-mannopyranosyl, α-D-mannopyranosyl-(1→3)-α/β-D-mannopyranosyl, α-D-mannopyranosyl-(1→4)-α/β-D-mannopyranosyl, and α-D-mannopyranosyl-(1→6)-α/β-D-mannopyranosyl. In certain embodiments of the invention, the OS moiety comprises disaccharide-yl selected from α-D-mannopyranosyl-(1→2)-α/β-D-mannopyranosyl, α-D-mannopyranosyl-(1→3)-α/β-D-mannopyranosyl, α-D-mannopyranosyl-(1→4)-α/β-D-mannopyranosyl, and α-D-mannopyranosyl-(1→6)-α/β-D-mannopyranosyl. In certain embodiments of the invention, the OS moiety is α-D-mannopyranosyl-(1→2)-α/β-D-mannopyranosyl. In certain embodiments of the invention, the OS moiety is α-D-mannopyranosyl-(1→3)-α/β-D-mannopyranosyl. In certain embodiments of the invention, the OS moiety is α-D-mannopyranosyl-(1→4)-α/β-D-mannopyranosyl. In certain embodiments of the invention, the OS moiety is α-D-mannopyranosyl-(1→6)-α/β-D-mannopyranosyl.

In certain embodiments of the invention, the OS moiety comprises 3 mannose monomers. In certain embodiments of the invention, the OS moiety is selected from α-D-mannopyranosyl-(1→2)-α-D-mannopyranosyl-(1→2)-α/β-D-mannopyranosyl, α-D-mannopyranosyl-(1→2)-α-D-mannopyranosyl-(1→3)-α/β-D-mannopyranosyl, α-D-mannopyranosyl-(1→2)-α-D-mannopyranosyl-(1→6)-α/β-D-mannopyranosyl, α-D-mannopyranosyl-(1→3)-α-D-mannopyranosyl-(1→6)-α/β-D-mannopyranosyl, and α-D-mannopyranosyl-(1→3)-[α-D-mannopyranosyl-(1→6)]-α/β-D-mannopyranosyl. In certain embodiments of the invention, the OS moiety is a trisaccharide-yl selected from α-D-mannopyranosyl-(1→2)-α-D-mannopyranosyl-(1→2)-α/β-D-mannopyranosyl, α-D-mannopyranosyl-(1→2)-α-D-mannopyranosyl-(1→3)-α/β-D-mannopyranosyl, α-D-mannopyranosyl-(1→2)-α-D-mannopyranosyl-(1→6)-α/β-D-mannopyranosyl, α-D-mannopyranosyl-(1→3)-α-D-mannopyranosyl-(1→6)-α/β-D-mannopyranosyl, and α-D-mannopyranosyl-(1→3)-[α-D-mannopyranosyl-(1→6)]-α/β-D-mannopyranosyl. In certain embodiments of the invention, the OS moiety is α-D-mannopyranosyl-(1→2)-α-D-mannopyranosyl-(1→2)-α/β-D-mannopyranosyl. In certain embodiments of the invention, the OS moiety is α-D-mannopyranosyl-(1→2)-α-D-mannopyranosyl-(1→3)-α/β-D-mannopyranosyl. In certain embodiments of the invention, the OS moiety is α-D-mannopyranosyl-(1→2)-α-D-mannopyranosyl-(1→6)-α/β-D-mannopyranosyl. In certain embodiments of the invention, the OS moiety is α-D-mannopyranosyl-(1→3)-α-D-mannopyranosyl-(1→6)-α/β-D-mannopyranosyl. In certain embodiments of the invention, the OS moiety is α-D-mannopyranosyl-(1→3)-[α-D-mannopyranosyl-(1→6)]-α/β-D-mannopyranosyl.

In certain embodiments of the invention, the OS moiety comprises 4 mannose monomers. In certain embodiments of the invention, the OS moiety is selected from α-D-mannopyranosyl-(1→2)-α-D-mannopyranosyl-(1→2)-α-D-mannopyranosyl-(1→3)-α/β-D-mannopyranosyl, α-D-mannopyranosyl-(1→2)-α-D-mannopyranosyl-(1→3)-α-D-mannopyranosyl-(1→6)-α/β-D-mannopyranosyl, α-D-mannopyranosyl-(1→2)-α-D-mannopyranosyl-(1→6)-α-D-mannopyranosyl-(1→6)-α/β-D-mannopyranosyl, α-D-mannopyranosyl-(1→2)-α-D-mannopyranosyl-(1→6)-[α-D-mannopyranosyl-(1→3)]-α/β-D-mannopyranosyl, α-D-mannopyranosyl-(1→2)-α-D-mannopyranosyl-(1→3)-[α-D-mannopyranosyl-(1→6)]-α/β-D-mannopyranosyl, α-D-mannopyranosyl-(1→6)-α-D-mannopyranosyl-(1→6)-[α-D-mannopyranosyl-(1→3)]-α/β-D-mannopyranosyl, α-D-mannopyranosyl-(1→3)-α-D-mannopyranosyl-(1→6)-[α-D-mannopyranosyl-(1→3)]-α/β-D-mannopyranosyl, and α-D-mannopyranosyl-(1→6)-[α-D-mannopyranosyl-(1→3)]-α-D-mannopyranosyl-(1→6)-α/β-D-mannopyranosyl. In certain embodiments of the invention, the OS moiety is comprises a tetrasaccharide moiety selected from α-D-mannopyranosyl-(1→2)-α-D-mannopyranosyl-(1→2)-α-D-mannopyranosyl-(1→3)-α/β-D-mannopyranosyl, α-D-mannopyranosyl-(1→2)-α-D-mannopyranosyl-(1→3)-α-D-mannopyranosyl-(1→6)-α/β-D-mannopyranosyl, α-D-mannopyranosyl-(1→2)-α-D-mannopyranosyl-(1→6)-α-D-mannopyranosyl-(1→6)-α/β-D-mannopyranosyl, α-D-mannopyranosyl-(1→2)-α-D-mannopyranosyl-(1→6)-[α-D-mannopyranosyl-(1→3)]-α/β-D-mannopyranosyl, α-D-mannopyranosyl-(1→2)-α-D-mannopyranosyl-(1→3)-[α-D-mannopyranosyl-(1→6)]-α/β-D-mannopyranosyl, α-D-mannopyranosyl-(1→6)-α-D-mannopyranosyl-(1→6)-[α-D-mannopyranosyl-(1→3)]-α/β-D-mannopyranosyl, α-D-mannopyranosyl-(1→3)-α-D-mannopyranosyl-(1→6)-[a-D-mannopyranosyl-(1→3)]-α/β-D-mannopyranosyl, and α-D-mannopyranosyl-(1→6)-[α-D-mannopyranosyl-(1→3)]-α-D-mannopyranosyl-(1→6)-α/β-D-mannopyranosyl. In certain embodiments of the invention, the OS moiety is α-D-mannopyranosyl-(1→2)-α-D-mannopyranosyl-(1→2)-α-D-mannopyranosyl-(1→3)-α/β-D-mannopyranosyl. . In certain embodiments of the invention, the OS moiety is α-D-mannopyranosyl-(1→2)-α-D-mannopyranosyl-(1→3)-α-D-mannopyranosyl-(1→6)-α/β-D-mannopyranosyl. In certain embodiments of the invention, the OS moiety is α-D-mannopyranosyl-(1→2)-α-D-mannopyranosyl-(1→6)-α-D-mannopyranosyl-(1→6)-α/β-D-mannopyranosyl. . In certain embodiments of the invention, the OS moiety is α-D-mannopyranosyl-(1→2)-α-D-mannopyranosyl-(1→6)-[α-D-mannopyranosyl-(1→3)]-α/β-D-mannopyranosyl. In certain embodiments of the invention, the OS moiety is α-D-mannopyranosyl-(1→2)-α-D-mannopyranosyl-(1→3)-[α-D-mannopyranosyl-(1→6)]-α/β-D-mannopyranosyl. In certain embodiments of the invention, the OS moiety is α-D-mannopyranosyl-(1→6)-α-D-mannopyranosyl-(1→6)-[α-D-mannopyranosyl-(1→3)]-α/β-D-mannopyranosyl. In certain embodiments of the invention, the OS moiety is α-D-mannopyranosyl-(1→3)-α-D-mannopyranosyl-(1→6)-[α-D-mannopyranosyl-(1→3)]-α/β-D-mannopyranosyl. In certain embodiments of the invention, the OS moiety is α-D-mannopyranosyl-(1→6)-[α-D-mannopyranosyl-(1→3)]-α-D-mannopyranosyl-(1→6)-α/β-D-mannopyranosyl.

A is $C_1$-$C_7$-alkylene, $C_1$-$C_4$-alkylene-$(OCH_2CH_2)_pO$-$C_1$-$C_4$-alkylene, or $C_1$-$C_7$-alkylene-$R^b$-, wherein -$R^b$- is an optionally substituted arylene or an optionally substituted heteroarylene, and wherein p is 0 to 6, preferably p is 1, 2, or 3, and more preferably p is 1. Preferably, A is $C_1$-$C_7$-alkylene. More preferably, A is $-CH_2CH_2CH_2-$.

B is -NHC(O)-, -S-, $-CH_2-$, -O-, -NH- or

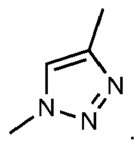

Preferably, B is -NHC(O)-.

q is 0 to 6. Preferably q is 1, 2, 3 or 4. Further preferably, q is 3.

Optionally, there may be an additional $C_1$-$C_7$-alkylene between A and B.

In certain embodiments of the invention, OS comprises up to 9 mannose monomers, and is attached to A through the reducing end of one of its monomers. In other words, A is attached to the anomeric carbon of the sugar moiety of OS, preferably through an -O-, -NH-, -S-, or $-CH_2$-group. Therefore, in certain embodiments of the present invention, the compound according to formula (I) is a compound according to formula (Ia):

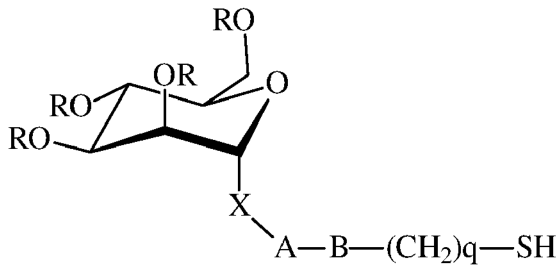

(Ia)

or a tautomer, pharmaceutically acceptable salt, hydrate, solvate, polymorph thereof, as well as an anomer at the carbon atom attached to -X-. A, B, and q are as defined herein. Each R is independently an oligosaccharide-yl moiety, preferably comprising up to 3 mannose monomers, or H. X is -O-, -NH-, -S-, or $-CH_2-$. Preferably, X is -O-.

In certain embodiments of the present invention, OS comprises a pyranose-yl moiety, preferably fucose-yl moiety.

In certain embodiments of the present invention, OS comprises a moiety selected from Lewis x antigen (β-D-galactopyranosyl-(1-4)-[α-D-fucopyranosyl-(1-3)]-α/β-D-N-acetylglucopyranosylamine), Lewis a antigen (β-D-galactopyranosyl-(1-3)-[α-D-fucopyranosyl-(1-4)]-α/β-D-N-acetylglucopyranosylamine), ABO A antigen (α-D-galactopyranosyl-(1-3)-[α-D-fucopyranosyl-(1-2)]-β-D-galactopyranosyl-(1-3/4)-α/β-D-N-acetylglucopyranosylamine; ABO B antigen (α-D-N-acetylgalactopyranosylamine-(1-3)-[α-D-fucopyranosyl-(1-2)]-β-D-galactopyranosyl-(1-3/4)-α/β-D-N-acetylglucopyranosylamine, ABO H antigen (α-D-fucopyranosyl-(1-2)-β-D-galactopyranosyl-(1-3/4)-α/β-D-N-acetylglucopyranosylamine), and Lacto-N-fucopentaose III (*LNFP III*): (β-D-galactopyranosyl-(1-4)-[α-D-fucopyranosyl-(1-3)]-β-D-galactopyranosyl-(1-3)-α/β-D-galactopyranose).

In certain embodiments of the present invention, OS is derived from a naturally occurring mannose-containing glycan. For example, in certain embodiments OS is an oligosaccharide-yl according to formula (IIa):

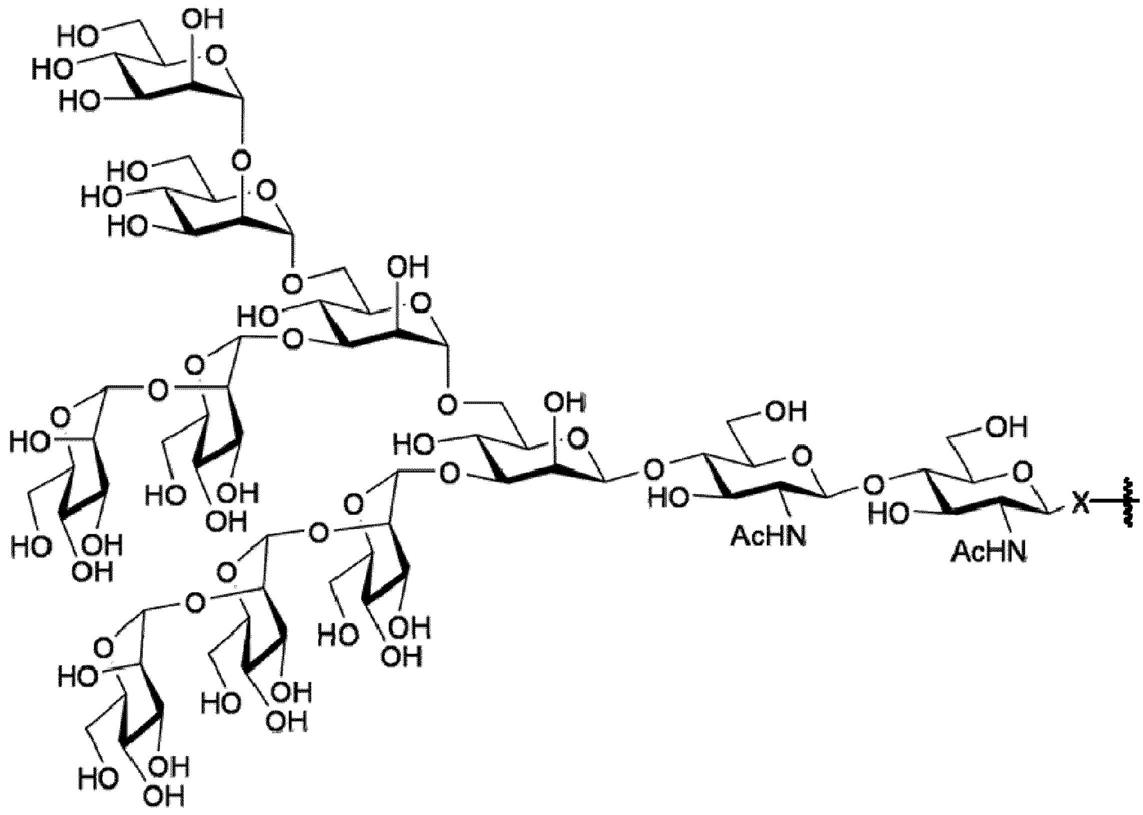

(IIa)

or oligosaccharide-yl obtained by anomerization of the carbon atom attached to -X-. Ac is an acetyl group ($CH_3C(O)$-). X is -O-, -NH-, -S-, or -$CH_2$-. Preferably, X is -O-.

In certain embodiments, the compound of formula (I) is a compound of formula (Ib)

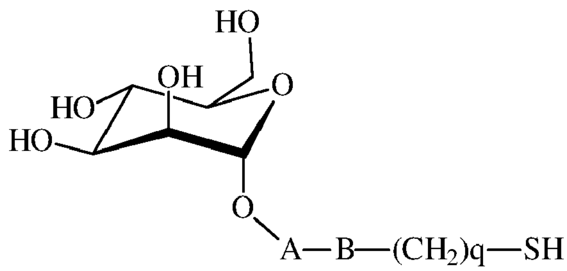

(Ib)

or an anomer, tautomer, pharmaceutically acceptable salt, hydrate, solvate or polymorph thereof.

A is $C_1$-$C_7$-alkylene, $C_1$-$C_4$-alkylene-$(OCH_2CH_2)_pO$-$C_1$-$C_4$-alkylene, or $C_1$-$C_7$-alkylene-$R^b$-, wherein -$R^b$- is an optionally substituted arylene or an optionally substituted heteroarylene, and wherein p is 0 to 6, preferably p is 1,2, or 3, and more preferably p is 1. Preferably, A is $C_1$-$C_7$-alkylene. More preferably, A is -$CH_2CH_2CH_2$-.

B is -NHC(O)-, -S-, -$CH_2$-, -O-, -NH- or

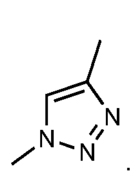

Preferably, B is -NHC(O)-.

q is 0 to 6. Preferably q is 1, 2, 3 or 4. Further preferably, q is 3.

Preferably, in certain embodiments of the present invention, the compound of formula (Ia) is a compound of formula (Ic):

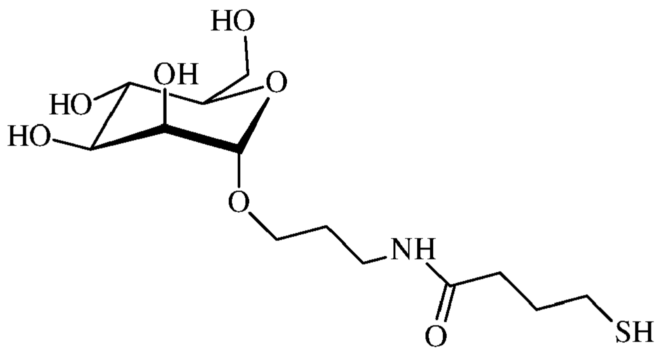

(Ic)

or an anomer, tautomer, pharmaceutically acceptable salt, hydrate, solvate or polymorph thereof.

In certain embodiments of the present invention, the compound of formula (I) is a compound according to formula (Id):

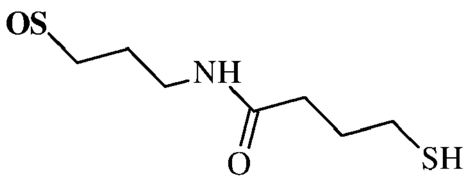

(Id)

or a tautomer, pharmaceutically acceptable salt, hydrate, solvate or polymorph thereof.

OS is as defined herein.

The -A-B-$(CH_2)_q$- moiety of the compound of formula (I) or formula (Ib) is preferably selected from any one of the following moieties:

i. $-(CH_2)_{2-7}-NHC(O)-(CH_2)_{2-6}-$
ii. $-(CH_2)_2-O-(CH_2CH_2O)_{1-6}-CH_2CH_2-NHC(O)-(CH_2)_{2-6}-$
iii. $-(CH_2)_{2-7}-S-(CH_2)_{2-6}-$
iv. $-(CH_2)_2-O-(CH_2CH_2O)_{1-6}-CH_2CH_2-S-(CH_2)_{2-6}-$
v.

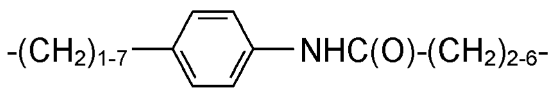

vi.

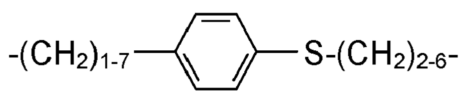

vii.

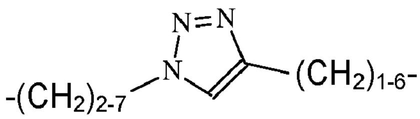

viii.

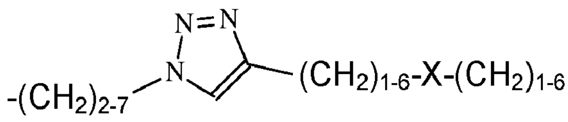

(wherein X is -O-, -NH-, - NH(CO)-, -NH($SO_2$)-, or -S-)

According to the present invention, preferred are the moieties according to formulas i, vii, and viii.

Preferably, the -A-B-$(CH_2)_q$- substructure is a substructure according to formula (Ie):

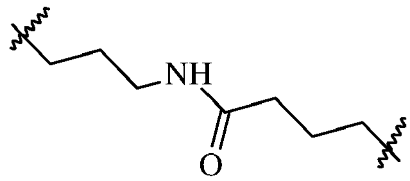

(Ie)

In one embodiment, the present invention relates to a polymer comprising a polymer backbone and a multitude of compounds of formula (I) as defined herein. The polymer backbone is not particularly limited and any polymer that is suitable for administration to patients can be used with the invention. It is understood that the polymer backbone comprises repeating units. In certain embodiments, the polymer backbone comprises an α-amino acid polymer, an acrylic acid or methacrylic acid polymer or copolymer, an N-vinyl-2-pyrrolidone-vinyl alcohol copolymer, a chitosan polymer, or a polyphosphazene polymer. Preferably, the polymer backbone comprises an α-amino acid polymer, preferably poly-L-lysine.

According to the present invention, the multitude of compounds of formula (I) are connected to the polymer backbone through the -SH group It is further understood that preferably each compound according to formula (I) is connected to one repeating unit of the polymer backbone through the -SH group. The term "connected through the -SH group" as used herein refers to a situation wherein an H radical is formally removed from the -SH group, yielding an -S· group for attachment. The phrase "a polymer comprising a polymer backbone and a multitude of compounds according to formula (I)" as used herein refers to a situation, wherein, formally, for each of a multitude of the compounds according to formula (I), the hydrogen radical in the -SH group of the compound according to formula (I) and typically one hydrogen radical of the polymer backbone (in particular of a repeating unit of the polymer backbone) are removed, and the resulting forms (i.e. hypothetical radicals) of the compound according to formula (I) and the polymer backbone are linked together. It is to be understood that this hypothetical consideration is merely intended to illustrate the meaning of the expression "a polymer comprising a polymer backbone and a multitude of compounds according to formula (I)" and in no way limits the manner in which the polymers of the present invention are/can be prepared.

According to the present invention, each repeating unit of the polymer backbone preferably comprises an acetyl moiety. Preferably, each of the compounds of the multitude of compounds according to formula (I) is connected through the -SH group to an acetyl moiety of the polymer backbone (in particular of a repeating unit of the polymer backbone; even more specifically of a side chain of a repeating unit of the polymer backbone in the case where the polymer backbone comprises an α-amino acid polymer). Herein, the polymer backbone preferably comprises an α-amino acid polymer, preferably a poly-L-lysine. As defined herein, the polymer backbone preferably comprises a poly-L-lysine wherein preferably each lysine side chain amino group is amidated with a substituent comprising an acetyl moiety. In other words, the polymer backbone comprises preferably N-acetylated poly-L-lysine, wherein an acetyl moiety is attached to each of its repeating units preferably through an amino group of lysine side chain. As understood herein, the phrase "a polymer comprising a polymer backbone and a multitude of compounds according to formula (I)" as used herein refers to a situation, wherein, formally, for each of a multitude of the compounds according to formula (I), a hydrogen radical in the -SH group of the compound according to formula (I) and typically one hydrogen radical of the polymer backbone (in

particular of a repeating unit of the polymer backbone, in particular of acetyl group) are removed, and the resulting forms of the compound according to formula (I) and the polymer backbone are linked together.

Preferably, the attachment of the compound according to formula (I) to the polymer backbone can thus involve reacting the prepolymer that comprises the polymer backbone, wherein some or all of its repeating units comprise an acetyl moiety, which is comprised within a chloroacetyl moiety, with the multitude of compounds according to formula (I). In such a case, the phrase "a polymer comprising a polymer backbone and a multitude of compounds according to formula (I)" as used herein refers to a situation, wherein, formally, for each of a multitude of the compounds according to formula (I), the proton in the -SH group of the compound according to formula (I) and typically one chloride ion of the prepolymer (such as a chloride ion of a chloroacetyl residue) are removed, and the resulting forms of the compound according to formula (I) and the polymer backbone are linked together.

The polymer backbone may also comprise, instead of acetyl moiety, a maleimide moiety according to the following formula:

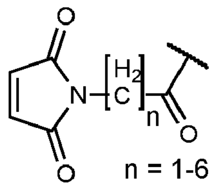

n = 1-6

If the polymer backbone comprises an $\alpha$-amino acid polymer, preferably a poly-L-lysine, the maleimide moiety can be connected to the amino group of the lysine side chain through an amide bond (the amide bond typically including the carbonyl group shown at the right-hand side of the above formula and the amine group of the side chain of the lysine). Herein, each of the multitude of compounds according to formula (I) can be connected to the polymer backbone (in particular to its repeating units, more particularly to the side chain of the repeating units in the case of amino acid polymers) through the -SH group, through the formal addition of the thiol to the double bond of maleimide.

In certain embodiments of the present invention, the polymer backbone comprises a poly-$\alpha$-amino acid, wherein the amino acid carries a side chain aminoalkyl function, such as in poly-lysine, in particular poly-L-lysine or poly-D-lysine, or in poly-ornithine. The poly-amino acid can be linear, hyperbranched, or dendritic, as described by Z. Kadlecova et al. Biomacromolecules 2012, 13:3127-3137, for poly-lysine as follows:

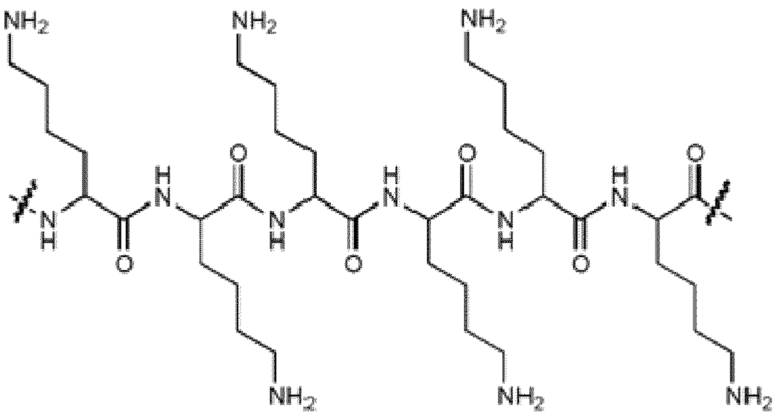

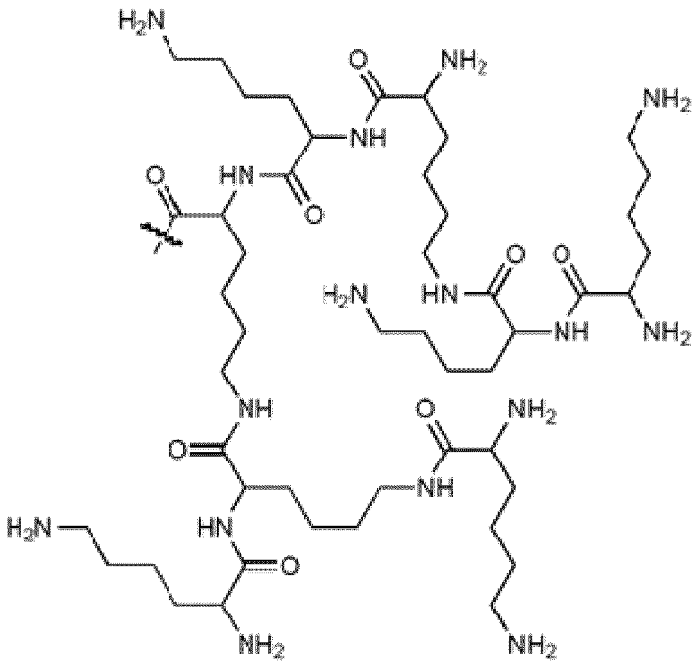

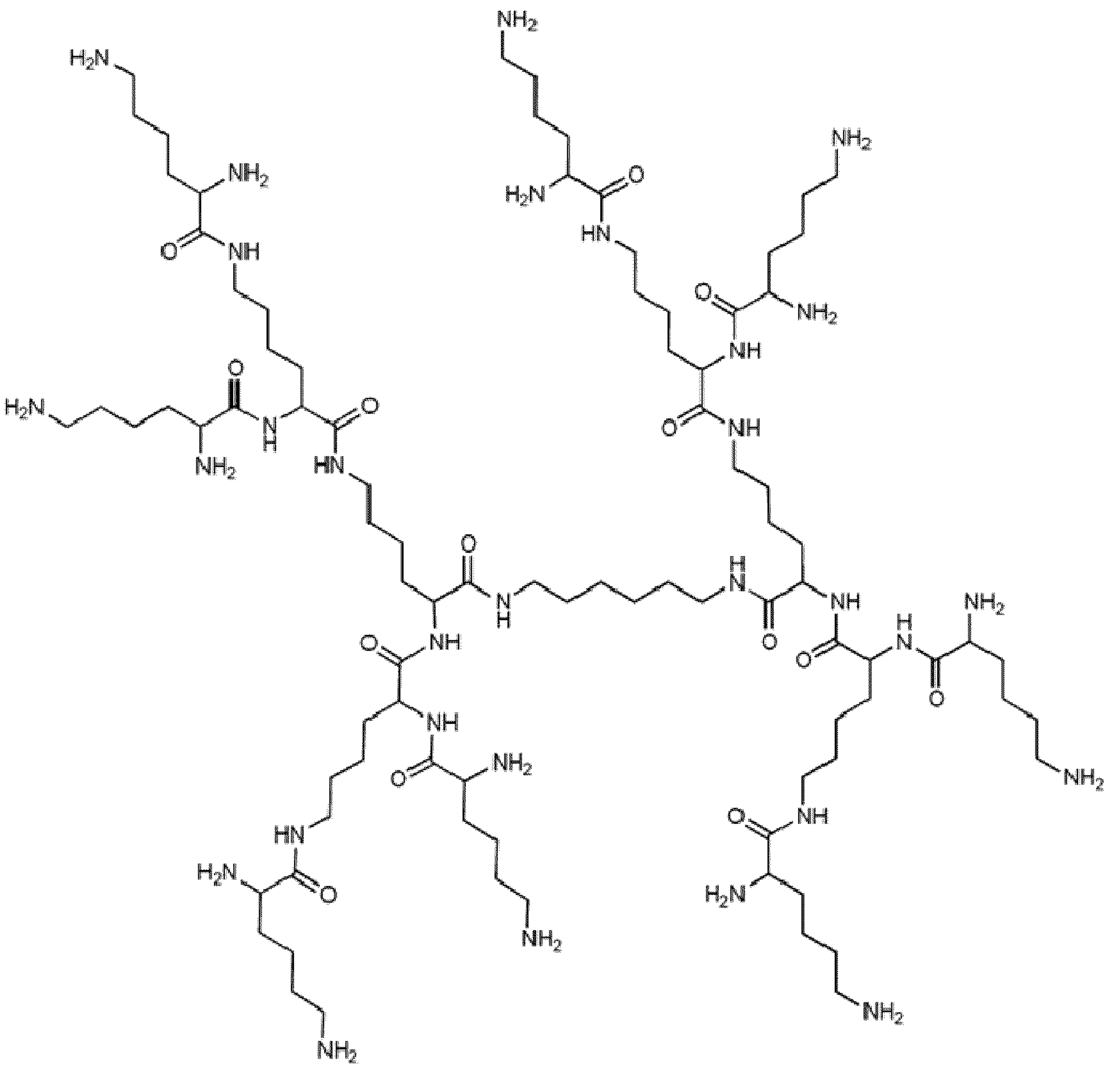

In certain embodiments of the present invention, the polymer comprises x repeating units having the following formula (IIIa):

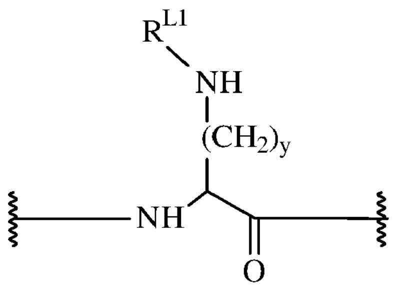

(IIIa)

wherein y is 3 in the case of poly-ornithine (preferably poly-L-ornithine), and y is 4 in the case of poly-lysine (preferably poly-L-lysine). As defined herein, $R^{L1}$ comprises the compound according to formula (I) attached through its -SH group, preferably $R^{L1}$ comprises the substructure $-CH_2C(O)-$, which is attached to the amino group of poly-lysine or poly-ornithine through an amide bond, and to the compound according to formula (I) through the -SH group of the latter. As herein understood, an attachment to -SH group involves, formally, the removal of an H radical from the -SH group.

Preferably, the polymer also comprises repeating units according to formula (IIIa), wherein $R^{L1}$ is 2,3-dihydroxypropylthioacetyl. The 2,3-dihydroxypropylthio-moiety can be replaced with another moiety that positively impacts solubility of the protein. As defined herein, the total number of repeating units n is at least 100, preferably n is at least 250, more preferably n is in the range from 250 to 800; and x/n is between 10 and 90%, preferably between 20 and 80%, more preferably between 30 and 70%.

$R^{L1}$ in x repeating units of the polymer of the present invention can be according to the formula (IVa):

$$OS\text{-}A\text{-}B\text{-}(CH_2)_q\text{-}S\text{-}CH_2C(O)\text{-} \qquad (IVa)$$

OS, A, B, and q are as defined herein. In other words, in this formula, a compound of formula (I) is already linked to an acetyl group (such as derived from a chloro acetyl group). Preferably, $R^{L1}$ according to formula (IVa) is a substructure according to formula (IVb):

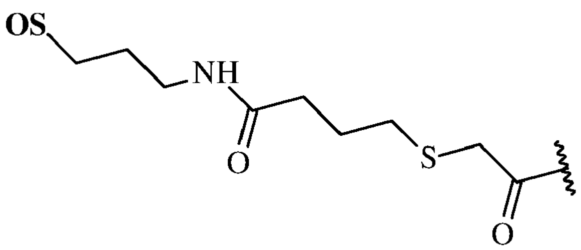

(IVb)

OS is as defined herein. Preferably, OS is a $\alpha/\beta$-D-mannopyranosyl moiety. Therefore, in a preferred embodiment, $R^{L1}$ according to formula (IVa) or (IVb) is a substructure according to formula (IVc):

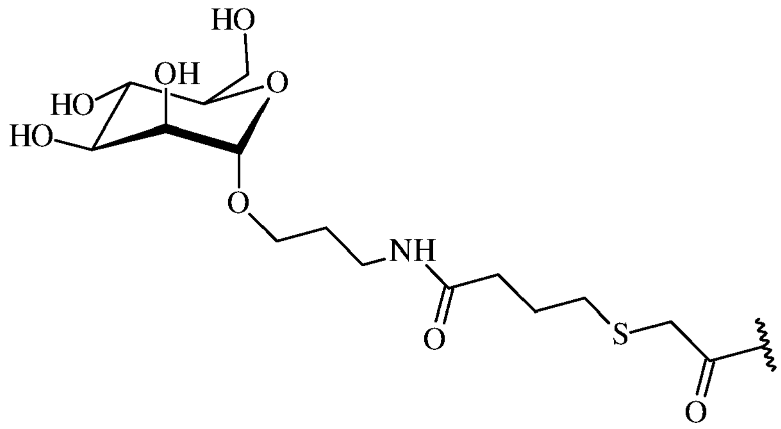

(IVc)

In certain embodiments of the present invention, the polymer comprises a substructure according to formula (IIIb):

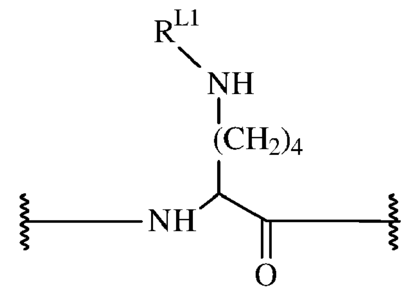

(IIIb)

$R^{L1}$ is:

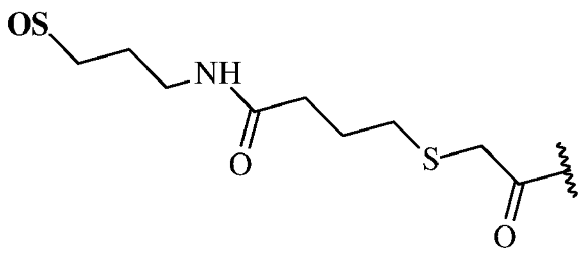

OS is as defined herein. Preferably, OS is selected from α/β-D-mannopyranosyl, α-D-mannopyranosyl-(1→2)-α/β-D-mannopyranosyl, α-D-mannopyranosyl-(1→3)-α/β-D-mannopyranosyl, α-D-mannopyranosyl-(1→4)-α/β-D-mannopyranosyl, α-D-mannopyranosyl-(1→6)-α/β-D-mannopyranosyl, α-D-mannopyranosyl-(1→3)-[α-D-mannopyranosyl-(1→6)]-α/β-D-mannopyranosyl, and α-D-mannopyranosyl-(1→2)-α-D-mannopyranosyl-(1→2)-α-D-mannopyranosyl-(1→3)-α/β-D-mannopyranosyl. More preferably, OS is α/β-D-mannopyranosyl.

Therefore, in a preferred embodiment, $R^{L1}$ according to formula (IVa) or (IVb) is a substructure according to formula (IVc):

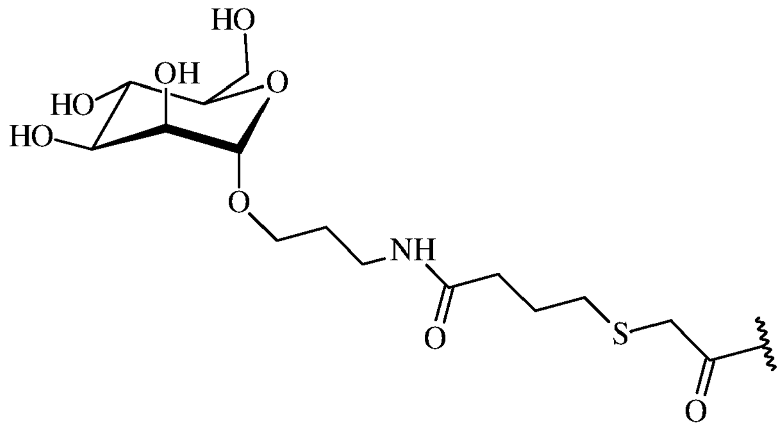

(IVc)

As defined herein, n is at least 100, preferably n is at least 250, more preferably n is in the range from 250 to 800; and
wherein x/n is between 10 and 90%, preferably between 20 and 80%, more preferably between 30 and 70%.

Preferably, the polymer also comprises repeating units according to formula (IIIb), wherein $R^{L1}$ is:

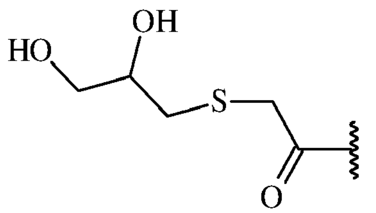

or another moiety positively impacting solubility of the polymer, for example a moiety comprising at least one -($CH_2CH_2O$)- repeating unit.

In certain embodiments of the present invention, the polymer backbone is a poly-α-amino acid, wherein the amino acid carries a side chain carbonylalkyl function, such as in poly-aspartic acid or poly-glutamic acid. The poly-amino acid can be linear, hyperbranched, or dendritic, as described by Z. Kadlecova et al. Biomacromolecules 2012, 13:3127-3137. In certain embodiments, the polymer comprises x repeating units having the formula (IIIc):

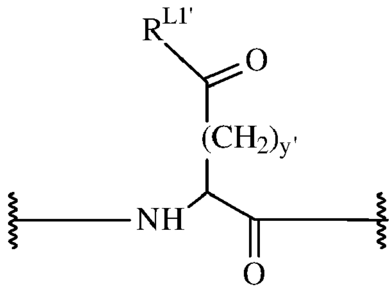

(IIIc)

wherein y' is 1 in the case of poly-aspartic acid, y' is 2 in the case of poly-glutamic acid. As defined herein, $R^{L1'}$ comprises the compound according to formula (I) attached through its -SH group, preferably $R^{L1'}$ comprises the substructure -$CH_2$-, which is directly attached to the carbonyl group of poly-aspartic acid or poly-glutamic acid, as well as to the compound according to formula (I) through the -SH group of the latter. As herein understood, attachment to -SH group involves, formally, removal of an H radical from the -SH group.

Preferably, the polymer further comprises repeating units according to formula (IIIc), wherein $R^{L1'}$ is 2,3-dihydroxypropylthiomethylen or another moiety that may lead to improved aqueous solubility.

As defined herein, the total number of repeating units n is at least 100, preferably n is at least 250, more

preferably n is in the range from 250 to 800; and x/n is between 10 and 90%, preferably between 20 and 80%, more preferably between 30 and 70%.

In certain embodiments of the present invention, the polymer backbone is poly-acrylic acid, poly-methacrylic acid or a copolymer of acrylic and methacrylic acid. In certain embodiments of the present invention, the polymer comprises x repeating units according to formula (IIId):

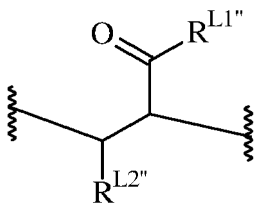

(IIId)

As defined herein, $R^{L1"}$ comprises the compound according to formula (I) attached through its -SH group, preferably $R^{L1"}$ comprises the substructure $-CH_2C(O)-NH-CH_2CH_2-NH-$, which is directly attached to the carbonyl group of the polymer through an amide bond to -NH- moiety, as well as to the -SH group of the compound according to formula (I) through the $-CH_2-$ of the $-CH_2C(O)-$ group. As herein understood, attachment to the -SH group involves, formally, the removal of an H radical from the -SH group.

Preferably, the polymer further comprises repeating units, wherein $R^{L1"}$ is a 2,3-dihydroxypropylthioacetylaminoalkylamino group. Dihydroxypropylthio group can be replaced with another moiety which positively impacts solubility of the polymer.

$R^{L2"}$ is methyl or H.

As defined herein, the total number of repeating units n is at least 100, preferably n is at least 250, more preferably n is in the range from 250 to 800; and

wherein x/n is between 10 and 90%, preferably between 20 and 80%, more preferably between 30 and 70%.

Alternatively, the polymer comprises x repeating units according to formula (IIId), wherein $R^{L1"}$ comprises the substructure $-CH_2C(O)-NH-CH_2CH_2-O-[CH_2CH_2-O]_{n"}-CH_2CH_2-NH-$, wherein n" is between 1 to 10, preferably wherein n" is between 1 to 4, which is directly attached to the carbonyl group of the polymer through an amide bond to -NH- moiety, as well as to the -SH group of the compound according to formula (I) through the $-CH_2-$ of $-CH_2C(O)-$ group. As herein understood, attachment to -SH group involves, formally, removal of H radical from the -SH group.

Preferably, the polymer further comprises repeating units, wherein $R^{L1"}$ is 2,3-dihydroxypropylthio group attached to the acetyl moiety in $-CH_2C(O)-NH-CH_2CH_2-O-[CH_2CH_2-O]_{n"}-CH_2CH_2-NH-$. Dihydroxypropylthio group can be replaced with another moiety which positively impacts solubility of the polymer.

$R^{L2"}$ is methyl or H.

As defined herein, the total number of repeating units n is at least 100, preferably n is at least 250, more preferably n is in the range from 250 to 800; and

wherein x/n is between 10 and 90%, preferably between 20 and 80%, more preferably between 30 and 70%.

Alternatively, the polymer comprises x repeating units according to formula (IIId), wherein $R^{L1"}$ comprises the substructure $-(CH_2)_r-NH-$, wherein r is between 1 and 6, preferably between 1 and 4, in particular 2.

Preferably, the polymer further comprises repeating units, wherein $R^{L1"}$ is $CH_3-NH-$.

$R^{L2"}$ is methyl or H.

As defined herein, the total number of repeating units n is at least 100, preferably n is at least 250, more preferably n is in the range from 250 to 800; and

wherein x/n is between 10 and 90%, preferably between 20 and 80%, more preferably between 30 and 70%.

In certain embodiments of the present invention, the polymer backbone is a copolymer of N-vinyl-2-pyrrolidone and vinyl alcohol, wherein the hydroxy group of the vinyl alcohol part of the copolymer is connected to the compound according to formula (I). In certain embodiments of the present invention, the polymer comprises x repeating units according to the formula (IIIe):

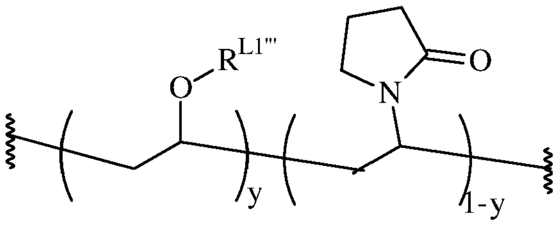

(IIIe)

Herein, $R^{L1'''}$ comprises a $-CH_2-$ moiety. y is either 0 or 1. Preferably, for repeating units, wherein y is 1, $R^{L1'''}$ comprises the $-C(O)NHCH_2CH_2NHC(O)-CH_2-$ substructure, which is attached to the polymer through the carbonyl moiety, and the compound according to formula (I) is attached through its -SH group to the $-CH_2-$ moiety of $C(O)-CH_2-$ of said substructure. As herein understood, attachment to the -SH group involves, formally, the removal of an H radical from the -SH group.

Preferably, the polymer further comprises repeating units according to formula (IIIe) wherein y = 1 and $R^{L1'''}$ is preferably $-C(O)NHCH_2CH_2NHC(O)-CH_2-S-CH_2-CHOH-CH_2-OH$.

Alternatively, the polymer comprises x repeating units according to the formula (IIIe) wherein y is 1, $R^{L1'''}$ preferably comprises the $-C(O)NHCH_2CH_2-O(CH_2CH_2O)_m-CH_2CH_2NHC(O)-CH_2-$ moiety, wherein m is between 1 and 10, preferably between 1 and 4, the polymer is attached to the carbonyl moiety, and the compound according to formula (I) is attached through its -SH group to the $-CH_2-$ moiety of the $-C(O)-CH_2-$moiety. As herein understood, attachment to -SH group involves, formally, removal of H radical from the -SH group.

Preferably, the polymer further comprises the repeating units according to formula (IIIe) wherein y = 1, $R^{L1'''}$ is preferably $-C(O)NHCH_2CH_2-O(CH_2CH_2O)_m-CH_2CH_2NHC(O)-CH_2-S-CH_2-CHOH-CH_2-OH$ wherein m is between 1 and 10, preferably between 1 and 4.

Alternatively, the polymer comprises x repeating units according to the formula (IIIe), $R^{L1'''}$ is according to formula $-C(O)NH-(CH_2)_{r'''}-$ wherein r''' is between 1 and 6, preferably between 1 and 4, more preferably 2, and wherein the polymer is attached to the carbonyl moiety, and the compound according to formula (I) is attached through its -SH group to the $-CH_2-$ moiety of the $-C(O)-CH_2-$ moiety.

Preferably the polymer further comprises repeating units according to formula (IIIe) wherein y = 1, $R^{L1'''}$ is preferably $-C(O)NHCH_3$.

As defined herein, n is at least 100, preferably n is at least 250, more preferably n is in the range from 250 to 800; and

wherein x/n is between 10 and 90%, preferably between 20 and 80%, more preferably between 30 and 70%.

In certain embodiments of the present invention, the polymer backbone is an α-amino acid polymer. Preferably, the α-amino acid polymer is a homopolymer. The amino acid is preferably selected from lysine, ornithine, glutamic acid, aspartic acid, histidine, cysteine, or serine. Therefore, in certain embodiments of the present invention, the polymer backbone is polyhistidine. In certain embodiments of the present invention, the polymer backbone is polycysteine. In certain embodiments of the present invention, the polymer backbone is polyserine.

In certain embodiments of the present invention, the polymer backbone is a chitosan polymer. Chitosan, as understood herein, is a linear polysaccharide composed of randomly distributed β-hnked D-glucosamine and N-acetyl-D-glucosamine. Preferably, the molar ratio of D-glucosoamine repeating units to the sum of D-glucosoamine and N-acetyl-D-glucosoamine repeating units is between 0 and 0.4.

In certain embodiments of the present invention, the polymer backbone is a polyphosphazene polymer.

In certain embodiments of the present invention, the polymer backbone is a copolymer comprising more than one type of monomers.

In certain embodiments of the present invention, the polymer backbone is PEGylated (i.e. a polymer or an oligomer comprising $-CH_2CH_2O-$ repeating units is attached thereto). The polymer backbone may be PEGylated on its one end or on its two ends, or in the case of branched or dendrimeric polymers, on each of its ends. For example, when the polymer backbone is an α-amino acid polymer, the polymer backbone can be PEGylated at its N-terminus and/or at its C-terminus. The polymer backbone may also be PEGylated at other positions. For example, when the polymer backbone is an α-amino acid polymer, the polymer backbone can be PEGylated at selected sidechains of the amino acid. The skilled person would know how to PEGylate different polymers as discussed in the present application. Typically, a PEG chain comprising between 1 and 1000 $-CH_2CH_2O-$ repeating units is attached at each position.

In certain embodiments of the present invention, the weight average molecular weight of the polymer is at least

30 000 Da. In certain embodiments of the invention, the weight average molecular weight of the polymer is at least 40 000 Da, at least 50 000 Da, at least 60 000 Da, at least 70 000 Da, at least 80 000 Da, or at least 90 000 Da. Preferably, the weight average molecular weight of the polymer is at least 90 000 Da. Further preferably the weight average molecular weight of the polymer is between 90 000 Da and 500 000 Da, more preferably between 90 000 Da and 200 000 Da. In certain embodiments of the present invention, the weight average molecular weight of the polymer backbone is at least 5000 Da, preferably at least 10000 Da. In certain embodiments of the invention, the weight average molecular weight of the polymer is between 10000 Da and 120 000 Da, As herein understood, the weight average molecular weight of the polymer relates to the weight average molecular weight of a polymer with the plurality of compounds according to formula (I) attached to the backbone. The weight average molecular weight is determined by using SEC-MALS or NMR. The weight average molecular weight is preferably measured by SEC-MALS, as known to the skilled person. Alternatively, the weight average molecular weight can be calculated based on the weight average molecular weight of polymer backbone and polymer loading with compound according to formula (I), as determined by NMR, as disclosed herein. For example, in the case of polymer **1a**, n is 100 (total number of repeating units) the weight average molecular weight of the poly-L-lysine polymer (hydrobromide) backbone according to the supplier is 21 000 Da as determined by SEC-MALS, and the polymer loading as determined by NMR is 44% (see Table 1). Therefore, the calculated weight average molecular weight of the polymer is 12 717 (corresponding to poly-L-lysine of 100 repeating units) + 100 · 44% · 380.43 (molecular weight of substructure according to formula (IVc)) + 100 · 56% · 149.18 (molecular weight of 2,3-dihydroxythioacetyl) with one hydrogen radical subtracted to be formally removed from each repeating unit of poly-L-lysin polymer backbone for attachment = 37 810 Da.

Attachment of the plurality of compounds according to formula (I) can also be described by using the term of polymer loading with the compound according to formula (I). As used herein, if the total number of monomers in the polymer is n, and number of monomers with the compound according to formula (I) attached is x, then the polymer loading with the compound according to formula (I) is defined as

$$\text{Polymer loading} = x/n \cdot 100\%$$

Polymer loading may also be referred to herein as x%. It is noted that in certain polymers, for example copolymers, it may be that not every monomer can be loaded with the compound according to formula (I). The polymer loading with the compound according to formula (I) can also be referred to as the loading (or the percentage loading) of the compound according to formula (I) onto the polymer backbone.

In certain embodiments of the present inventions, loading can be determined by NMR. As illustrated in the Examples, in certain embodiments loading can be determined by NMR by comparing the integrals of thioglycerol $CH_2$-SR and $CH_2$-SR/$CH_2$-CONH that are part of the attached compound according to formula (I).

According to the present invention, in certain embodiments the loading of the compound according to formula (I) on the polymer backbone is at least 10%, at least 20%, at least 30%, or at least 40%. The loading of the compound according to formula (I) on the polymer backbone of at least 10% is to be understood herein that at least 10% of monomers of the polymer backbone are attached to the compound according to formula (I). Preferably, in certain embodiments of the invention the loading of the compound according to formula (I) onto the polymer backbone is at least 20%, more preferably is at least 30%. In certain embodiments of the present invention, the loading of the compound according to formula (I) onto the polymer backbone is not more than 60%, not more than 70%, not more than 80%, or not more than 90%. In certain embodiments of the present invention, preferably the loading of the compound according to formula (I) onto the polymer backbone is not more than 80%, more preferably is not more than 70%. Thus, in certain embodiments of the present invention, the percentage of loading of the compound according to formula (I) onto the polymer backbone is between 10 and 90%, preferably between 20 and 80%, more preferably between 30 and 70%.

The polymer according to the present invention can also be described using biophysical parameters, in particular hydrodynamic radius as determined by analytical ultracentrifugation (AUC) (Example 2, Figure 2) and hydrodynamic diameter, as determined by dynamic light scattering (DLS) (Example 1, Figure 1). The present inventors have surprisingly found that large, elongated molecules (1b-d) were found to be more potent inhibitors of viral attachment compared with the smaller, globular polymer **1a**, probably because in elongated molecules the availability of epitopes for binding to a cell surface is spatially favored. The observed trend in affinity in the cellular assay correlates well with the solution properties of the Man-PLL polymers determined by DLS and AUC. The differences in size and shape are reflected by a significantly higher binding affinity (approximately a factor of 50) of elongated polymers **1b-d** in cellular assays compared with the globular **1a.** On the other side, between **1b-d**, the variations in the polymerization degree (**1b** → **1c**) and carbohydrate loading (**1c** → **1d**) influence affinity only by a factor of two. The observation that the elongated glycopolymers **1b-d** show much higher affinity compared to the globular **1a** is probably related to the fact that DC-SIGN has evolved to capture large pathogens, such as viruses (ca. 20-500 nm) or bacteria (1-2 μm), not soluble glycoproteins.

In certain embodiments of the present invention, the polymer exhibits a hydrodynamic radius of at least 3 nm

as measured by analytical ultracentrifugation. Preferably the polymer exhibits a hydrodynamic radius of at least 5 nm as measured by analytical ultracentrifugation. More preferably, the polymer exhibits a hydrodynamic radius of at least 10 nm as measured by analytical ultracentrifugation.

In certain embodiments of the present invention, the polymer exhibits a hydrodynamic diameter of at least 9 nm as measured by dynamic light scattering. Preferably, the polymer exhibits the hydrodynamic diameter of at least 14 nm as measured by dynamic light scattering. More preferably, the polymer exhibits the hydrodynamic diameter of at least 19 nm as measured by dynamic light scattering.

In one embodiment, the invention relates to a polymer which is obtainable by reacting two or more compounds according to formula (I) with a prepolymer containing two or more chloroacetyl moieties, as defined herein.

The compounds of the present invention as well as the polymers of the present administration can be administered to a patient in the form of a pharmaceutical composition which can optionally comprise one or more pharmaceutically acceptable excipient(s) and/or carrier(s). Pharmaceutically acceptable excipients, carriers, adjuvants, additives, surfactants, desiccants or diluents known to those well-skilled in the art, and can be suitably adapted for systemic or topical administration.

Without wishing to be bound by theory it is assumed that the compounds of the present invention, the polymers of the present invention and the pharmaceutical compositions of the present invention are capable of inhibiting the attachment of viral envelope glycoproteins to the cells containing DC-SIGN receptor. Envelope glycoproteins of many viruses are heavily glycosylated. Among other functions, virus glycans can mediate interactions with host receptors and contribute to internalization and virus dissemination. The C-type lectin receptor DC-SIGN, which is expressed by cells of the innate immune system, can act as an entry receptor for many pathogens, including pandemic viruses such as SARS-CoV-2, Ebola, and HIV. In the context of the recent SARS-CoV-2 pandemic, this mechanism has been linked to severe cases of COVID-19. Inhibition of the interaction between DC-SIGN and viral envelope glycoproteins, thus, has the potential to generate broad spectrum antivirulent agents. Moreover, the importance of this mechanism across many different infections and the conservation of the interaction partners in the host genome highlight the potential of DC-SIGN-targeted therapeutics not only for the treatment of existing infections, but also for the rapid response to future pandemics with newly emerging virus serotypes. With respect to the ongoing SARS-CoV-2 pandemic, DC-SIGN targeted compounds, polymers and pharmaceutical compositions are useful in treatment of severe COVID-19, which is characterized by an exuberant immune response with the release of proinflammatory cytokines. Furthermore, inhibition of the DC-SIGN-mediated infection of DC-SIGN$^+$ cells by pulmonary application of the present invention, the compound of the invention, the polymer of the invention or the pharmaceutical composition of the invention may be able to prevent progression of mild infections to severe forms of the disease.

The pharmaceutical compositions of the present invention are suitable for treating, preventing and/or alleviating viral infection in an individual in need thereof. The pharmaceutical compositions may be in any suitable form depending on the viral infection to be treated. Thus, the pharmaceutical composition may be formulated for systemic administration or for topical administration, depending on the viral infection.

In certain embodiments of the present invention, the compound of the invention, the polymer of the invention or the pharmaceutical composition of the invention is administered intravenously.

Among preferred forms of administration of compounds of the present invention are forms suitable for injectionable use and include sterile aqueous solutions or dispersions and sterile powders for the extemporaneous preparation of sterile injectable solutions or dispersion. In all cases the final solution or dispersion form must be sterile and fluid. Typically, such a solution or dispersion will include a solvent or dispersion medium, containing, for example, water-buffered aqueous solutions, e.g., biocompatible buffers (e.g., citrate buffer), ethanol, polyol, such as glycerol, propylene glycol, polyethylene glycol, suitable mixtures thereof, surfactants or vegetable oils. A compound of the invention can also be formulated into liposomes, in particular for parenteral administration. Liposomes provide the advantage of increased half-life in the circulation, if compared to the free drug and an even more prolonged release of the enclosed drug.

Sterilization of infusion or injection solutions can be accomplished by any number of art recognized techniques including but not limited to addition of preservatives like anti-bacterial or anti-fungal agents, e.g., parabene, chlorobutanol, phenol, sorbic acid or thiomersal, preferably by sterile filtration. Further, isotonic agents, such as sugars or salts, in particular sodium chloride may be incorporated in infusion or injection solutions.

Production of sterile injectable solutions containing one or several of the compounds of the invention is accomplished by incorporating the respective compound in the required amount in the appropriate solvent with various ingredients enumerated above as required followed by sterilization. To obtain a sterile powder the above solutions are vacuum-dried or freeze-dried as necessary. Preferred diluents of the present invention are water, physiologically acceptable buffers, physiologically acceptable buffer salt solutions or salt solutions. Preferred carriers are cocoa butter and vitebesole.

In certain embodiments of the present invention, the compound of the invention, the polymer of the invention or the pharmaceutical composition of the invention is administered through pulmonary administration route. Administration of the compound of the invention, the polymer of the invention or the pharmaceutical composition of the invention

directly to the lungs is particularly desired in the case of viral infections presents in the lungs, for example in the case of the viral infection caused by SARS-Coronavirus-2.

Pulmonary administration can be achieved by using aerosol sprays, non-aqueous inhalers, dry powder inhalers, as well as nebulizers. The compound of the invention or the polymer of the invention or the pharmaceutical composition of the invention can be administered in the form of a dry powder inhaler or an aerosol spray from a pressurized container, pump, spray or nebulizer with the use of a suitable propellant, e.g., dichlorodifluoromethane, trichlorofluoromethane, dichlorotetrafluoroethane, a hydrofluoroalkane such as 1,1,1,2-tetrafluoroethane (HFA 134A™) or 1,1,1,2,3,3,3-heptafluoropropane (HFA 227EA™), carbon dioxide, or another suitable gas. The pressurized container, pump, spray or nebulizer may contain a solution or suspension of the compound of the invention or the polymer of the invention, e.g., using a mixture of ethanol and the propellant as the solvent, which may additionally contain a lubricant, e.g., sorbitan trioleate.

It is to be understood that depending on the severity of the infection and the particular type which is treatable with one of the compounds of the invention, as well as on the respective patient to be treated, e.g., the general health status of the patient, etc., different doses of the respective compound can be required to elicit a therapeutic or prophylactic effect. The determination of the appropriate dose lies within the discretion of the attending physician. It is contemplated that the dosage of a compound of the invention in the therapeutic or prophylactic use of the invention should be in the range of about 0.1 mg to about 1 g of the active ingredient (i.e. compound of the invention) per kg body weight. However, in a preferred use of the present invention a compound of the invention can be administered to a subject in need thereof in an amount ranging from 1.0 to 500 mg/kg body weight, preferably ranging from 1 to 200 mg/kg body weight. The duration of therapy with a compound of the invention will vary, depending on the severity of the disease being treated and the condition and idiosyncratic response of each individual patient.

In certain embodiments of the present invention, the viral infection is caused by a virus characterized by a mannose-comprising glycan epitope on envelope glycoproteins. Such viruses include SARS-Coronavirus 2, SARS-Coronavirus, MERS-Coronavirus, HIV, influenza A virus, hepatitis C virus, zika virus, dengue virus, ebola virus, west-Nile virus, Marburg virus, chikungunya virus, and Japanese encephalitis virus.

Therefore, in certain embodiments of the present invention, the viral infection is caused by a virus selected from SARS-Coronavirus 2, SARS-Coronavirus, MERS-Coronavirus, HIV, influenza A virus, hepatitis C virus, zika virus, dengue virus, ebola virus, west-Nile virus, Marburg virus, chikungunya virus, and Japanese encephalitis virus. Preferably, the viral infection is caused by SARS-Coronavirus 2.

In certain embodiments of the present invention, the compound, the polymer, or the pharmaceutical composition interferes with DC-SIGN-mediated attachment and internalization of viral particles. It is known to the skilled person that DC-SIGN-mediated attachment and internalization of viral particles is a mechanism that is common and conserved among different viruses. For example, SARS-Coronavirus 2 that emerged only in 2019 and spread within months causing a pandemic with more than million deaths to date, shares this mechanism with other coronaviruses. Therefore, the compounds of the present invention, the polymers of the present invention and the pharmaceutical compositions of the present invention are useful in the treatment of infections caused by newly emerging viruses.

In certain embodiments of the present invention, the compounds of the invention, the polymers or the pharmaceutical compositions of the invention is administered to a subject treated with an antiviral agent.

## Synthesis of the compounds of the present invention

The compounds of the present invention can be synthesized by one of the general methods shown in the following scheme. These methods are only given for illustrative purposes and should not to be construed as limiting.

### Scheme 1

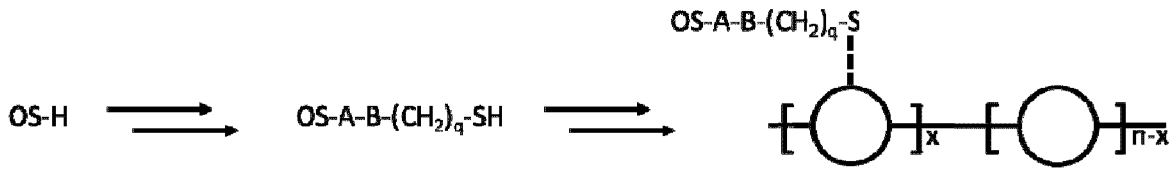

wherein circle represents a monomer of a chloroacetylated polymer backbone. Oligosaccharide (OS-H) is decorated in one or more synthetic steps with a moiety comprising thiol group, yielding compounds according to formula (I) OS-A-B-$(CH_2)_q$-SH. In further step(s), the compound according to formula (I) is attached to the chloroacetylated polymer backbone through its -SH group by nucleophilic substitution of a chlorine substituent. It is to be understood that Scheme

1 is not limited to block copolymers, but also encompasses statistical polymers. Instead it merely serves to illustrate that some, but not necessarily all, repeating units in the polymer backbone are modified by the attachment of the OS-containing group.

The invention is illustrated by the following examples which, however, should not be construed as limiting. Various modifications and variations of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art or the relevant fields are intended to be covered by the present invention.

## Examples

NMR spectra were recorded on a Bruker Avance III 500 MHz spectrometer. Assignment of $^1H$ and $^{13}C$ NMR spectra was achieved using 2D methods (COSY, HSQC, HMBC). Chemical shifts are expressed in ppm using residual $CHCl_3$, $CHD_2OD$ or HDO as references. Electron spray ionization mass spectra (ESI-MS) were obtained on a Waters micromass ZQ Mass Spectrometer. Reactions were monitored by TLC using glass plates coated with silica gel 60 $F_{254}$ (Merck, Darmstadt, Germany) and visualized by using UV light and/or by charring with a molybdate solution (a 0.02 M solution of ammonium cerium sulfate dihydrate and ammonium molybdate tetrahydrate in aqueous 10% $H_2SO_4$). MPLC separations were carried out on a CombiFlash Companion or $R_f$ from Teledyne Isco (Lincoln, NE, USA) equipped with RediSep flash columns. For purification of glycopolymers, Vivaspin ultrafiltration devices with a molecular weight cutoff of 50 kDa (Sartorius, Göttingen, Germany) were used. Commercially available reagents and dry solvents were purchased from Sigma-Aldrich, Alfa Aesar and Acros Organics. Poly-L-lysine HBr polymers were acquired from PtS (Valencia, Spain).

## General synthesis of Man-PLL glycopolymers

Mannose containing building block **4** according to formula (I), as well as polymers **1a-d** comprising poly-L-lysine block with said mannose containing building block attached, have been obtained according to the following scheme.

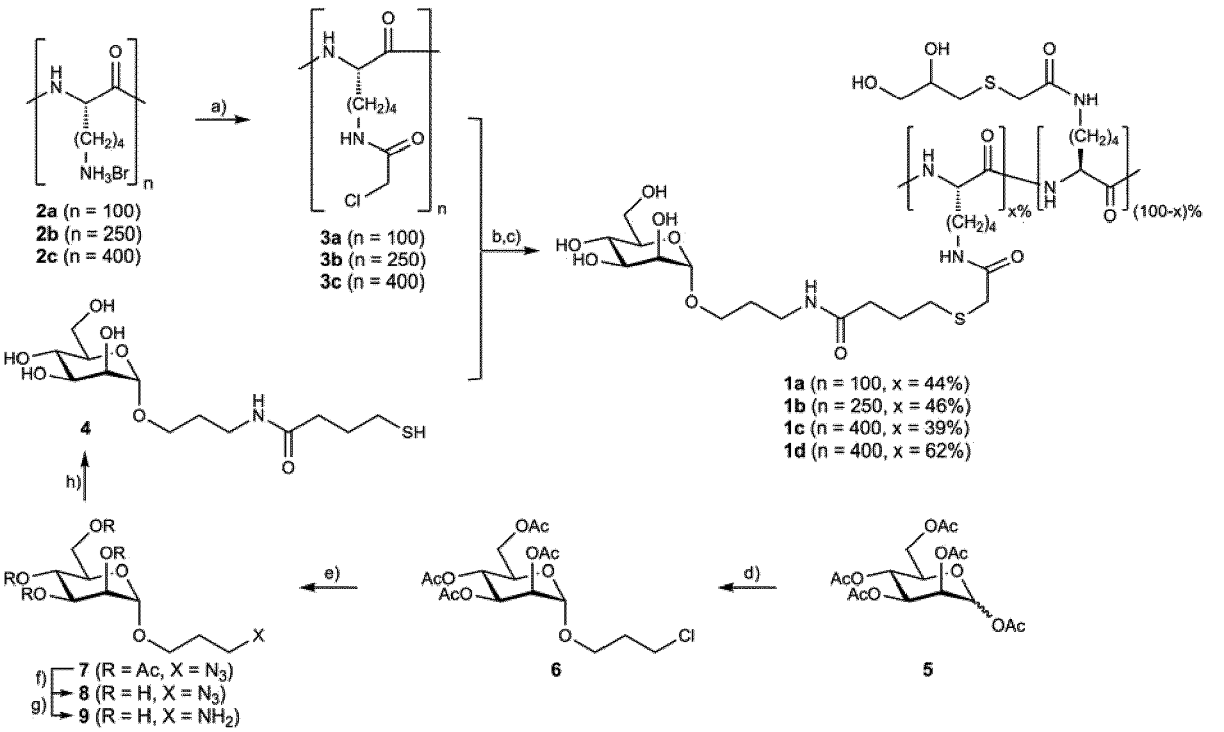


wherein (a) 2,6-lutidine, chloroacetic anhydride, 4°C, 16h (**3a** (92%), **3b** (62%), **3c** (96%)); b) 1,8-diazabicyclo(5.4.0)undec-7-ene/DMF, building block **4**, without purification c) thioglycerol, $Et_3N$, shaken overnight: **1a** (45%, loading 44%); **1b** (64%, loading 46%); **1c** (68%, loading 39%); **1d** (51%, loading 62%). d) 3-chloro-1-propanol, $BF_3$●$Et_2O/CH_2Cl_2$, rt, overnight, (65%); (e) $NaN_3$/DMF, 60°C, 2h, (95%); (g) MeONa/MeOH, rt, overnight, (83%); (h) $Pd(OH)_2$/C in MeOH, $H_2$,

(84%); (i) γ-thiobutyrolactone, MeOH/$Et_3N$, rt, overnight, (68%). The polymers **1a-d** in the scheme above are not to be understood as block-copolymers. Both bracketed entities are only meant to be present in the stoichiometric ratio as defined by the parameter x%, representing polymer loading as defined herein.

**Preparative example 1 - synthesis of mannose building block 4**

**3-Chloropropyl-2,3,4,6-tetracatate-α-D-mannopyranoside (6)**

To a mixture of D-mannose pentacetate (**5**, 532 mg, 1.36 mmol) and 3-chloro-1-propanol (98%) (114 μL, 1.36 mmol) in dry dichloromethane (DCM) (5.0 mL), $BF_3$ $Et_2O$ (671 μL, 4.0 mmol) was added at room temperature. The reaction mixture was stirred at rt overnight, then diluted with DCM, washed carefully with sat. aqueous $NaHCO_3$ and brine. The combined organic layers were dried over $Na_2SO_4$. The solvent was removed under reduced pressure and the residue was purified by chromatography on silica gel (petrol ether/ethyl acetate, 7:3-6:4) to provide **6** (378 mg, 65%) as colorless oil.
$^1H$ NMR (500 MHz, $CDCl_3$) δ 5.34 - 5.25 (m, 2H, H-5, H-4), 5.24 (dd, *J* = 3.2, 1.8 Hz, 1H, H-2), 4.83 (d, *J* = 1.8 Hz, 1H, H-1), 4.28 (dd, *J* = 12.2, 5.5 Hz, 1H, H-6b), 4.13 (dd, *J* = 12.2, 2.5 Hz, 1H, H-6a), 3.99 (ddd, *J* = 9.4, 5.4, 2.4 Hz, 1H, H-5), 3.92 (ddd, *J* = 9.8, 7.3, 4.9 Hz, 1H, $ClCH_2CH_2CH_2O$), 3.73 - 3.62 (m, 2H, $ClCH_2CH_2CH_2O$), 3.59 (ddd, *J* = 9.8, 6.1, 5.1 Hz, 1H, $ClCH_2CH_2CH_2O$), 2.16 (s, 3H, OAc), 2.10 (s, 3H, OAc), 2.08 (m, 2H, $ClCH_2CH_2CH_2O$), 2.05 (s, 3H, OAc), 2.00 (s, 3H, OAc),

**3-Azidopropyl-2,3,4,6-tetra-O-acetyl-α-D-mannopyranoside (7)**

To a solution of chloride **6** (373 mg, 0.87 mmol) in N,N-dimethylformamide (DMF) (4.0 mL), $NaN_3$ (342 mg, 5.26 mmol) was added at room temperature. The reaction mixture was stirred at 60°C for 2h and then the solvent was removed under reduced pressure. The residue was dissolved in EtOAc, washed with $H_2O$ and brine. The organic layer was dried over $Na_2SO_4$, the solvent removed under reduced pressure and the residue purified by chromatography on silica gel (petrol ether/ethyl acetate, 7:3-6:4) to provide azide **7** (258 mg, 95%) as colorless oil.
$^1H$ NMR (500 MHz, $CDCl_3$) δ 5.40 - 5.18 (m, 3H, H-4, H-3, H-2), 4.82 (d, *J* = 1.8 Hz, 1H, H-1), 4.28 (dd, *J* = 12.3, 5.4 Hz, 1H, H-6b), 4.13 (m, 1H, H-6a), 3.97 (ddd, *J* = 9.6, 5.4, 2.4 Hz, 1H, H-5), 3.82 (ddd, *J* = 9.9, 6.8, 5.5 Hz, 1H, $N_3CH_2CH_2CH_2O$), 3.53 (ddd, *J* = 9.9, 6.4, 5.4 Hz, 1H, $N_3CH_2CH_2CH_2O$), 3.43 (t, *J* = 6.5 Hz, 2H, $N_3CH_2CH_2CH_2O$), 2.16 (s, 3H, OAc), 2.11 (s, 3H, OAc), 2.05 (s, 3H, OAc), 2.00 (s, 3H, OAc), 1.90 (dtd, *J* = 12.1, 6.7, 5.4 Hz, 2H, $N_3CH_2CH_2CH_2O$).

**3-Azidopropyl-α-D-mannopyranoside (8)**

To a solution of azide **7** (587 mg, 1.96 mmol) in MeOH (10.0 mL), 0.155 mL of $CH_3ONa$/MeOH (0.437 N) was added at rt. The reaction mixture was stirred at rt overnight, then neutralized with Amberlyst-15 and filtered. The filtrate was concentrated *in vacuo* and the residue was purified by chromatography on silica gel (DCM/MeOH, 9:1-6:1) to provide **8** (298 mg, 83%) as colorless oil.
$^1H$ NMR (500 MHz, MeOD) δ 4.75 (d, *J* = 1.8 Hz, 1H, H-1), 3.86 - 3.81 (m, 3H, H-6a, $N_3CH_2CH_2CH_2O$, H-2), 3.74 - 3.66 (m, 2H, H-6b, H-3), 3.61 (t, *J* = 9.5 Hz, 1H, H-4), 3.54 - 3.47 (m, 2H, H-5, $N_3CH_2CH_2CH_2O$), 3.41 (td, *J* = 6.7, 4.5 Hz, 2H, $N_3CH_2CH_2CH_2O$), 1.95 - 1.65 (m, 2H, $N_3CH_2CH_2CH_2O$).

**3-Aminopropyl-α-D-mannopyranoside (9)**

A suspension of azide **8** (298 mg, 1.132 mmol) and $Pd(OH)_2$/C (38.0 mg, 10% Pd) in MeOH (10.0 mL) was hydrogenated with a $H_2$ balloon at room temperature overnight. The reaction mixture was filtered through celite and the solvent was removed under reduced pressure. The residue was purified by reverse-phase C18 chromatography ($H_2O$/MeOH, 1:0-9:1) to provide amine **9** (226 mg, 84%) as a white solid (highly hydroscopic).
$^1H$ NMR (500 MHz, $D_2O$) δ 4.88 (d, *J* = 1.7 Hz, 1H, H-1), 3.96 (dd, *J* = 3.4, 1.7 Hz, 1H, H-2), 3.91 (dd, *J* = 12.2, 1.8 Hz, 1H, H-6b), 3.86 - 3.72 (m, 3H), 3.69 - 3.54 (m, 3H), 2.86 - 2.74 (m, 2H $NH_2CH_2CH_2CH_2O$), 1.82 (tt, *J* = 14.1, 7.3 Hz, 2H, m, 2H, $NH_2CH_2CH_2CH_2O$).

**Synthesis of mannose building block 4**

Aminopropyl α-D-mannopyranoside (**9**, 20 mg, 84 μmol, 1 eq) was dissolved in 190 μL water. 1 M NaOH (8.4 μL, 84 μmol, 0.1 eq), γ-thiobutyrolactone (72.8 μL, 86 mg, 840 μmol, 10 eq), and methanol (300 μL) were added and the resulting mixture was stirred overnight. After removal of methanol under reduced pressure the aqueous phase was

washed three times with EtOAc. After removal of insoluble material by centrifugation and lyophilization, the building block **4** was obtained as a colorless solid (24 mg, 71 μmol, 84%).
$^1$H NMR (500 MHz, $D_2O$) δ 4.87 (d, *J* = 1.8 Hz, 1H, Man-*H1*), 3.97 (dd, *J* = 3.4, 1.8 Hz, 1H, Man-*H2*), 3.91 (dd, *J* = 12.1, 2.1 Hz, 1H, Man-*H6'*), 3.87 - 3.73 (m, 3H, Man-*H3,* Man-*H6*", O-C$H_2$'), 3.73 - 3.60 (m, 2H, Man-*H4*, Man-*H5*), 3.57 (m, 1H, RO-C$H_2$"), 3.31 (m, 2H, C$H_2$-NH(CO)), 2.57 (t, *J* = 7.1 Hz, 2H, C$H_2$-SH), 2.39 (t, *J* = 7.4 Hz, 2H, C$H_2$-CONH), 1.92 (m, 2H, C$H_2$-$CH_2$-SH), 1.89 - 1.81 (m, 2H, C$H_2$-$CH_2$-O). $^{13}$C NMR (126 MHz, $D_2O$) δ 176.0 (*C*ONH), 99.9 (Man-*C1*), 72.8 (Man-*C5*), 70.6 (Man-*C4*), 70.1 (Man-*C2*), 66.8 (Man-*C3*), 65.2 (*C*$H_2$-O), 60.9 (Man-*C6*), 36.4 (*C*$H_2$-NH(CO)), 34.5 (*C*$H_2$-CONH), 29.5 (*C*$H_2$-$CH_2$-SH), 28.1 (*C*$H_2$-$CH_2$-O), 23.1 (*C*$H_2$-SH). ESI$^+$-MS Calculated for $C_{13}H_{24}NNaO_7S$ $[M+Na]^+$: 362.39, found: 362.16.

**Preparative examples - synthesis of Man-PLL glycopolymers 1a-d.**

Table 1 summarizes the glycopolymers prepared within Preparative Examples 2 to 5 of the present application.

**Table 1.** Poly-L-lysine glycopolymers prepared for the study of DC-SIGN binding.

| Compound | Polymerization degree (number of Lys) | Absolute number of mannose residues by NMR | Carbohydrate loading x% | Average molecularweight [kDa] |
|---|---|---|---|---|
| Man-PLL$_{100}$ (1a) | 100 | 44 | 44% | 37.8 |
| Man-PLL$_{250}$ (1 b) | 250 | 115 | 46% | 95.0 |
| Man-PLL$_{400}$ (1 c) | 400 | 156 | 39% | 146.6 |
| Man-PLL$_{400}$ (1d) | 400 | 248 | 62% | 167.9 |

**Syntheses of chloroacetylated poly-L-lysine (3a-c)**

Poly-L-lysine$^{400}$ HBr (**2**, 100 mg, 0.48 mmol) obtained from Alamanda Polymers Inc. (Huntsville, USA) was suspended in a mixture of DMF (2 mL) and 2,6-lutidine (0.5 mL). Chloroacetic anhydride (244 mg, dissolved in 0.5 mL of DMF) was added over 10 min under cooling with ice. The reaction mixture was stirred at 4°C for 16 h. The product precipitated after addition to 40 mL of a vigorously stirred 1:1 mixture of ethanol and diethyl ether and subsequent centrifugation (1000 rpm, 2 min, 4°C). The supernatant was discarded and the precipitate was washed three times with ethanol/diethylether (1:1, 30 mL). The purified product was dried under vacuum to yield chloroacetylated poly-L-lysine$^{400}$ **3c** as a colorless solid (92 mg, 96%). NMR spectra were identical to previously reported data (G. Thoma, J. T. Patton, J. L. Magnani, B. Ernst, R. Ohrlein, R. O. Duthaler, J. Am. Chem. Soc. 1999, 121, 5919-5929.).
Chloroacetylated poly-L-lysine$^{100}$ **3a** was obtained using a similar process as for **3c** as a colorless solid (90 mg, 92%). $^1$H NMR (400 MHz, DMSO-*d$_6$*) δ 8.18 (s, 2H, 2 - *NH*(CO)-), 4.02 (s, 2H, -C$H_2$Cl), 3.82 (s, 1H, -(CO)*CH*-NH-), 3.05 (s, 2H, $CH_2$), 1.83 (s, 2H, $CH_2$), 1.42 (m, 4H, 2 $CH_2$).
Chloroacetylated poly-L-lysine$^{250}$ **3b** was obtained using a similar process as for **3c** as a colorless solid (30 mg, 62%). $^1$H NMR (400 MHz, DMSO-*d$_6$*) δ 8.18 (s, 2H, 2 - *NH*(CO)-), 4.03 (s, 3H, -(CO)*CH*-NH-, and -C$H_2$Cl), 3.06 (s, 2H, $CH_2$), 1.43 (br, 6H, 3 $CH_2$).

**Synthesis of Man-PLL$^{100}$ 1a**

To a solution of chloroacetylated L-polylysine$^{100}$ (**3a,** 4.7 mg, 22.96 μmol) in DMF (200 μL) a solution of **4** [35 μL (3.5 mg, 10 μmol) of an aqueous stock solution (8.0 mg in water 80 μL] was added under Argon. Then 1,8-diazabicyclo(5.4.0)undec-7-ene (3.4 μL, 22.96 μmol) was added. The reaction mixture was shaken in an Eppendorf tube at 25°C for 1 h with an Eppendorf thermomixer at 300 rpm. Then, thioglycerol (6.0 μL, 68.8 μmol) and $Et_3N$ (9.6 μL, 68.8 μmol) were added and the mixture was shaken overnight. Next, the reaction mixture was dropped into a stirred solution of ethyl acetate/ethanol (1:1; 3.0 mL), leading to precipitation of the product. The precipitate was collected by centrifugation, washed with ethanol (3.0 mL), and then dissolved in water (3.0 mL). The aqueous solution was purified by ultracentrifugation (3 times from 6.0 mL to 0.5 mL; washed with water; molecular cutoff: 10kDa). The product in 1 ml $H_2O$ was lyophilized within 4 hours yielding 3.9 mg (45%) Man-PLL$^{100}$ **1a.** as a white solid (loading percentage based

on $^1$HNMR: 44 %).

### Synthesis of Man-PLL$^{250}$ 1b

To a solution of chloroacetylated L-polylysine$^{250}$ (**3b**, 3.6 mg, 17.59 μmol) in DMF (200 μL) a solution of **4** [26.8 μL (2.68 mg, 6.1μmol) of an aqueous stock solution (8.0 mg in water 80 μL] was added under Argon. Then 1,8-diazabicyclo(5.4.0)undec-7-ene (2.6 μL, 17.59 μmol) was added. The reaction mixture was shaken in Eppendorf tube at 25°C for 1 h with Eppendorf thermomixer at 300 rpm. Then, thioglycerol (4.6 μL, 52.77 μmol) and $Et_3N$ (7.4 μL, 52.77 μmol) were added and the mixture was shaken overnight. The reaction mixture was dropped into a stirred solution of ethyl acetate/ethanol (1:1; 3.0 mL), leading to precipitation of the product. The precipitate was collected by centrifugation, washed with ethanol (3.0 mL), and then dissolved in water (3.0 mL). The aqueous solution was purified by ultracentrifuge (3 times from 6.0 mL to 0.5 mL; washed with water; molecular cutoff: 10kDa). The product in 1 ml of water was lyophilized within 4 hours yielding 4.3 mg (64%) Man-PLL$^{250}$ **1b** as a white solid (loading percentage based on $^1$HNMR: 46 %).

### Synthesis of Man-PLL$^{400}$ 1c

To a solution of chloroacetylated L-polylysine400 (**3c**, 5.7 mg, 27.85 μmol) in DMF (200 μL) was added a solution of **4** (3.3 mg, 9.74 μmol) in water (45 μL) under Argon. Then 1,8-dDiazabicyclo(5.4.0)undec-7-ene (4.2 μL, 27.85 μmol) was added. The reaction mixture was shaken in an Eppendorf tube at 25°C for 1 h with Eppendorf thermomixer at 300 rpm. Then, thioglycerol (7.2 μL, 83.55 μmol) and $Et_3N$ (11.7 μL, 83.55 μmol) were added and the mixture was shaken overnight. Next, the reaction mixture was dropped into a stirred solution of ethyl acetate/ethanol (1:1; 3.0 mL), leading to precipitation of the product.. The precipitate was collected by centrifugation, washed with ethanol (3.0 mL), and then dissolved in water (3.0 mL). The solution was purified by ultracentrifuge (4 times from 20.0 mL to 0.5 mL; washed with water; molecular cutoff: 10kDa). The product was lyophilized from 1-2 mL aqueous solution within 5.5 hours yielding 7.0 mg (68%) Man-PLL$^{400}$ **1c** as a white solid (loading percentage based on $^1$HNMR: 39 %).

### Synthesis of Man-PLL$^{400}$ 1d

To a solution of chloroacetylated L-polylysine$^{400}$ (**3c**, 4.2 mg, 20.0 μmol) in DMF (200 μL) a solution of **4** [45 μL (3.13 mg, 9.2 μmol) of an aqueous stock solution (14.6 mg in 200 μL of water], were added under Argon. Then, 1,8-diazabicyclo(5.4.0)undec-7-ene (3.0 μL, 20.0 μmol) was added. The reaction mixture was shaken in Eppendorf tube at 25°C for 1 h with Eppendorf thermomixer at 300 rpm. Then, thioglycerol (5.2 μL, 60.0 μmol) and $Et_3N$ (8.4 μL, 60.0 μmol) were added and the mixture was shaken overnight. Next, the reaction mixture was dropped into a stirred solution of ethyl acetate/ethanol (1:1; 3.0 mL), leading to precipitation of the product. The precipitate was collected by centrifugation, washed with ethanol (3.0 mL), and then dissolved in water (3.0 mL). The aqueous solution was purified by ultracentrifuge (4 times from 20.0 mL to 0.5 mL; washed with water; molecular cutoff: 10kDa). The product was lyophilized from 1 mL aqueous solution within 5.5 hours yielding 4.3 mg (51%) of Man-PLL400 1d as a white solid (loading percentage based on $^1$HNMR: 62%).

### Synthesis of fluorescently labeled polymers 13 and 14

### Synthesis of Rhodamine 6G precursor 12

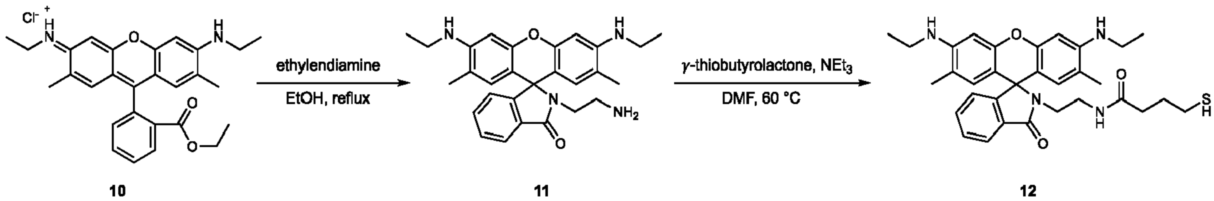


**10** **11** **12**

Synthetic route toward Rhodamine 6G precursor **12**

### Rhodamine 6G derivative 11

Rhodamine 6G (**10**, 500 mg, 1.04 mmol, 1 eq) and ethylenediamine (1.25 g, 20.8 mmol, 20 eq) were dissolved

in EtOH and refluxed for 5 h. The solvent was removed under reduced pressure, and the crude product purified by silica gel chromatography (DCM/MeOH 10:1) to yield the product **11** as a slightly pink solid (428 mg, 0.94 mmol, 90%). $^1$H NMR (500 MHz, $CDCl_3$) δ 7.96 - 7.89 (m, 1H, $H7_{isoindoline\text{-}1\text{-}one}$), 7.49 - 7.40 (m, 2H, $H5/6_{isoindoline\text{-}1\text{-}one}$), 7.09 - 7.01 (m, 1 H, $H4_{isoindoline\text{-}1\text{-}one}$), 6.34 (s, 2H, $H4/5_{9H\text{-}xanthene}$), 6.22 (d, *J* = 0.9 Hz, 2H, $H1/8_{9H\text{-}xanthene}$), 3.53 - 3.45 (m, 2H, Ar-N*H*$CH_2CH_3$), 3.25 - 3.17 (m, 4H, Ar-NHC$H_2$$CH_3$), 3.15 (t, *J* = 6.7 Hz, 2H, $R_2NCH_2CH_2NH_2$), 2.35 (t, *J* = 6.7 Hz, 2H, $R_2NCH_2CH_2NH_2$), 1.89 (s, 6H, Ar-C$H_3$), 1.32 (t, *J* = 7.2 Hz, 6H, Ar-NHC$H_2$C$H_3$). $^{13}$C NMR (126 MHz, $CDCl_3$) δ 168.6 (*C*O$NH_2$), 153.5 ($C1a_{isoindoline\text{-}1\text{-}one}$), 151.7 ($C4a/5a_{9H\text{-}xanthene}$), 147.5 ($C3/6_{9H\text{-}xanthene}$), 132.5 ($C5_{isoindoline\text{-}1\text{-}one}$), 131.2 ($C3a_{isoindoline\text{-}1\text{-}one}$), 128.3 ($C1/8_{9H\text{-}xanthene}$), 128.1 ($C6_{isoindoline\text{-}1\text{-}one}$), 123.8 ($C4_{isoindoline\text{-}1\text{-}one}$), 122.8 ($C7_{isoindoline\text{-}1\text{-}one}$), 118.0 ($C2/7_{9H\text{-}xanthene}$), 106.2 ($C1a/8a_{9H\text{-}xanthene}$), 96.5 ($C4/5_{9H\text{-}xanthene}$), 65.0 ($C9_{9H\text{-}xanthene}$), 44.0 ($R_2NCH_2CH_2NH_2$), 40.8 ($R_2NCH_2CH_2NH_2$), 38.4 (Ar-NH*C*$H_2CH_3$), 16.7 (Ar-*C*$H_3$), 14.7 (Ar-NH$CH_2$*C*$H_3$). ESI-MS m/z calculated for $C_{23}H_{33}N_4O_2$ [M+H]+: 457.60; found: 457.19.

**Rhodamin 6G precursor 12**

Rhodamin 6G derivative **11** (100 mg, 0.22 mmol, 1 eq) and γ-thiobutyrolactone (224 mg, 2.19 mmol, 10 eq) were dissolved under Argon in dry DMF. The resulting mixture was heated to 60 °C for 5 h, then the solvent removed under reduced pressure, and the crude product purified by silica gel chromatography (DCM/MeOH 10:1) to yield the free thiol **12** as a red oil (40 mg, 0.07 mmol, 33%) and the corresponding disulfide as a red oil (57 mg, 0.05 mmol, 46%). $^1$H NMR (500 MHz, $CDCl_3$) δ 7.99 - 7.86 (m, 1H, $H7_{isoindoline\text{-}1\text{-}one}$), 7.51 - 7.43 (m, 2H, $H5/6_{isoindoline\text{-}1\text{-}one}$), 7.12 - 6.98 (m, 1H, $H4_{isoindoline\text{-}1\text{-}one}$), 6.92 - 6.72 (m, 1H, CON*H*), 6.34 (s, 2H, $H4/5_{9H\text{-}xanthene}$), 6.26 - 6.14 (m, 2H, $H1/8_{9H\text{-}xanthene}$), 3.55 (s, 2H, Ar-N*H*$CH_2CH_3$), 3.30 - 3.24 (m, 2H, Ar-NHC$H_2$$CH_3$), 3.21 (q, *J* = 7.2 Hz, 4H, Ar-NHC$H_2$$CH_3$), 3.02 - 2.89 (m, 2H, $R_2NCH_2CH_2NH$), 2.60 - 2.46 (m, 2H, $HSCH_2CH_2CH_2CONH$), 2.19 (t, *J* = 7.3 Hz, 2H, $HSCH_2CH_2CH_2CONH$), 2.03 - 1.77 (m, 8H, $HSCH_2CH_2CH_2CONH$/Ar-C$H_3$), 1.33 (t, *J* = 7.2 Hz, 6H, Ar-NH$CH_2$C$H_3$). $^{13}$C NMR (126 MHz, $CDCl_3$) δ 172.7 (CONH), 170.1 ($CONR_2$), 153.9 ($C1a_{isoindoline\text{-}1\text{-}one}$), 151.9 ($C4a/5a_{9H\text{-}xanthene}$), 147.8 ($C3/6_{9H\text{-}xanthene}$), 133.0 ($C5_{isoindoline\text{-}1\text{-}one}$), 130.4 ($C3a_{isoindoline\text{-}1\text{-}one}$), 128.4 ($C1/8_{9H\text{-}xanthene}$), 128.1 ($C6_{isoindoline\text{-}1\text{-}one}$), 124.1 ($C4_{isoindoline\text{-}1\text{-}one}$), 123.0 ($C7_{isoindoline\text{-}1\text{-}one}$), 118.4 ($C2/7_{9H\text{-}xanthene}$), 105.4 ($C1a/8a_{9H\text{-}xanthene}$), 96.8 ($C4/5_{9H\text{-}xanthene}$), 65.7 ($C9_{9H\text{-}xanthene}$), 40.7 ($R_2NCH_2CH_2NH$), 40.1 ($R_2NCH_2CH_2NH$), 38.5 (Ar-NH*C*$H_2CH_3$), 35.0 ($HSCH_2CH_2CH_2CONH$), 29.9 ($HSCH_2CH_2CH_2CONH$), 24.3 ($HSCH_2CH_2CH_2CONH$), 16.9 (Ar-*C*$H_3$), 14.9 (Ar-NH$CH_2$*C*$H_3$). ESI-MS m/z calculated for $C_{32}H_{39}N_4O_3S$ $[M+H]^+$: 559.75; found: 559.38.

**Synthesis of Rhodamine 6G labeled mannosylated polymer 13**

The chloroacetylated $PLL_{400}$ (**3c**, 5 mg, 61 nmol, 1 eq), the rhodamine derivative **12** (0.41mg, 732 nmol, 12 eq), and DBU (3.72 mg, 24.4 μmol, 400 eq) were dissolved in DMF under Argon and protected from light. The resulting mixture was stirred at rt for 15 min. Then, a solution of mannoside **4** (3.31 mg, 9.76 μmol, 160 eq) in water (30 μL) was added and stirring continued for additional 45 min. Thioglycerol (7.4 mg, 68.3 μmol, 1120 eq) and $Et_3N$ (6.9 mg, 68.3 μmol, 1120 eq) were added and the solution was stirred for another 16 h at rt. The mixture was poured on 4 mL of a stirred 1:1 mixture of EtOH and $Et_2O$. After precipitation the product was collected by centrifugation (5 min, 200 rpm, 25 °C) and washed three times with 3 ml EtOH. The resulting solid was dissolved in 5 mL water and purified by ultrafiltration (Vivaspin 6, 50 kDa MWCO, 4000 rpm, 25 °C, three times from 5 mL to 0.5 mL). The purified product **13** was lyophilized to obtain a colorless solid (4.8 mg, 31 nmol, 51%) with a mannose loading of 47% (by H-NMR).

**Synthesis of Rhodamine 6G labeled non-mannosylated polymer 14**

The chloroacetylated $PLL_{400}$ **3c** (5 mg, 61 nmol, 1 eq), the rhodamine derivative **12** (0.41mg, 732 nmol, 12 eq), and DBU (3.72 mg, 24.4 μmol, 400 eq) were dissolved in DMF under Argon and protected from light. The resulting mixture was stirred at rt for 15 min. After the addition of 30 μL water stirring was continued for 45 min. Then, thioglycerol (7.4 mg, 68.3 μmol, 1120 eq) and $Et_3N$ (6.9 mg, 68.3 μmol, 1120 eq) were added and the solution was stirred for 16 h at rt. The mixture was poured into 4 mL of a vigourously stirred 1:1 mixture of EtOH and $Et_2O$. The precipitated product was collected by centrifugation (5 min, 200 rpm, 25 °C) and washed three times with 3 ml EtOH. The resulting solid was suspended in 5 mL water and 1 M HCl was added until complete dissolution of the solid material. The product was purified by ultrafiltration (Vivaspin 6, 50 kDa MWCO, 4000 rpm, 25 °C, three times from 5 mL to 0.5 mL) and lyophilized to obtain a pink solid (5.8 mg, 50 nmol, 82%).

**Weight average molecular weight determination by SEC-MALS**

SEC-MALS measurements were performed on an Agilent 1260 HPLC coupled to an Agilent multi-wavelength absorbance detector, a Wyatt Heleos II 8+ MALS detector, and a Wyatt Optilab rEX differential refractive index detector

(dRI), using a 1000 Å silica column (WTC1000N5, Silica SEC column, 4.6x300 mm, 5 micron beads, 1000 angstrom pore size, Wyatt Technology, United Kingdom). The column was equilibrated in PBS to stabilize the baseline signals of the detectors. The alignment, band broadening and detector normalization was performed using pullulan standard p100 ($M_w$=111000 Da, PSS Polymers).

**Characterization of polymer loading with the compound according to formula (I) for compounds 1a-d of the present invention by NMR**

Polymer loading with the compound according to formula (I) (referred to as polymer loading for brevity) was calculated from the ratio of integrals A and C' in the $^1$H-NMR spectra of polymers **1a-d** (Figure 6) following the formula below:

$$polymer\ loading = \frac{\frac{I_A}{N_A}}{\frac{I_A}{N_A}+\frac{I_{C'}}{N_{C'}}} \times 100\%$$

wherein *I* is the integral of signal A or C' and N is the number of corresponding protons contributing to each signal. Polymer loading may also be referred to as x%.

**Protein expression and purification**

*E. coli* BL21(DE3) cells were transfected with Novagen pET15b plasmids encoding for a recombinant DC-SIGN ECD linked to a thrombin cleavage site and an N-terminal His-Tag. Cells were initially cultivated overnight in 20 mL Luria Bertani medium substituted with 0.1 mg/mL ampicillin at 37 °C and then transferred into 1 L Terrific Broth medium substituted with 0.1 mg/mL ampicillin. Cells were incubated for 8 h at 37 °C and DC-SIGN expression was induced by addition of 0.5 mM IPTG. After 16 h, cells were harvested by centrifugation (4,000 rpm, 20 min, 4 °C), resuspended in lysis buffer (50 mM Tris-HCl, 10 mM $MgCl_2$, 0.1% Triton X100), and lysed by sonication or addition of lysozyme and DNAse I. The cell lysate was centrifuged (11000 rpm, 20 min, 4 °C), the supernatant discarded, and the precipitated material was washed three times with washing buffer (50 mM Tris-HCl, pH 8.0, 4 M urea, 500 mM NaCl, 1 mM EDTA). The purified inclusion bodies were dissolved in 20 mL of denaturation buffer (6 M guanidine hydrochloride, 100 mM Tris-HCl, pH 8.0, 1 mM DTT) for 1 h at 37 °C. After ultracentrifugation (22000 rpm, 30 min, 4 °C), the denatured protein was refolded by slow dilution into 100 mL refolding buffer (100 mM Tris-HCl, pH 8.0, 1 M l-arginine, 150 mM NaCl, 120 mM sucrose). The mixture was stirred for 2 d at 4 °C and dialyzed against binding buffer (20 mM Tris-HCl, 500 mM NaCl, 25 mM $CaCl_2$, pH 7.8). Precipitated protein was removed by ultracentrifugation (22000 rpm, 30 min, 4 °C) and the refolded soluble protein was purified by affinity chromatography on a mannose-sepharose column (elution buffer: 20 mM TRIS, 500 mM NaCl, 2 mM EDTA, pH 7.8).

**Mammalian cell culture, flow cytometry, and fluorescence microscopy**

The following reagent was obtained through the NIH AIDS Reagent Program, Division of AIDS, NIAID, NIH: B-THP-1 DC-SIGN+ cells from Drs. Li Wu and Vineet N. KewalRamani (cat# 9941, Wu, L.; Martin, T. D.; Carrington, M.; KewalRamani, V. N. Raji B Cells, Misidentified as THP-1 Cells, Stimulate DC-SIGN-Mediated HIV Transmission. Virology 2004, 318 (1), 17-23), HIV-1 JR-CSF Fc-gp120 Recombinant Protein (Cat#11556) from Aymeric de Parseval and Dr. John H. Elder (Binley, J. M.; Ngo-Abdalla, S.; Moore, P.; Bobardt, M.; Chatterji, U.; Gallay, P.; Burton, D. R.; Wilson, I. A.; Elder, J. H.; de Parseval, A. Inhibition of HIV Env Binding to Cellular Receptors by Monoclonal Antibody 2G12 as Probed by Fc-Tagged Gp120. Retrovirology 2006, 3, 39.). Recombinant Ebola glycoprotein was obtained from R&D Systems (cat# 9016-EB-100), recombinant SARS-CoV-2 spike glycoprotein S1 subunit was purchased from Creative Biomart (cat# Spike-191V). Murine alveolar macrophage cell line MH-S (ATCC CRL-2019) and THP-1 monocytes (ATCC TIB-202) were obtained from American Type Culture Collection.

B-THP-1 cells were maintained in RPMI 1640 medium supplemented with 10 % FCS and 1% antibiotic-antimycotic solution at 37 °C, 5% $CO_2$. MH-S cells were maintained in RPMI supplemented with 10% FCS, 0.05 mM β-mercaptoethanol, and 1% antibiotic-antimycotic solution at 37 °C, 5% $CO_2$. THP-1 cells were maintained in RPMI 1640 medium supplemented with 10% FCS, 1% antibiotic-antimycotic solution, 10 mM HEPES buffer, 1% sodium pyruvate, and 0.05 mM β-mercaptoethanol at 37 °C, 5% $CO_2$. For IC50 measurements, 50000 cells were seeded in 50 μL complete medium in a well of a 96-well plate. A serial dilution of the investigated ligand was prepared and mixed with an equal volume of Cy5-labeled glycoprotein. This mixture was added to the cell suspension to a final volume of 100 μL and a

final glycoprotein concentration of 10 nM (gp120), 10 nM (SARS-CoV-2 spike S1), or 50 nM (EBOV-gp). After incubation for 30 min (37°C, 5 % $CO_2$), the cells were centrifuged (5 min, 100g) and washed with warm PBS. Data were collected on a Cytoflex flow cytometer (Beckman Coulter, Indianapolis, USA) and analyzed with FlowJo (FlowJo LLC). Mean fluorescence intensities were plotted and a nonlinear regression analysis employing a four parameter Hill model was performed with Prism 8 (Graphpad, San Diego, USA). For receptor blocking analysis, 50000 B-THP-1 DC-SIGN+ cells were seeded and incubated with 100 nM 1d for 0 min to 6h, before washing and incubation with Cy5-labeled recombinant SARS-CoV-2 spike glycoprotein S1 subunit according to the protocol of the IC50 measurements. Additionally, B-THP-1 DC-SIGN+ cells were incubated with 100 nM 1d for 30 min, washed, and incubated with Cy5-labeled recombinant SARS-CoV-2 spike glycoprotein S1 subunit after 0 min, 10 min, 30 min, 1h, 3h, and 6h. Internalization experiments were performed with B-THP-1 DC-SIGN+ cells, control B-THP-1 cells, unpolarized MH-S cells, M2 polarized (20 ng/ml IL-4, 6h) MH-S cells, and PMA differentiated (100 nM, 72h) THP-1 control cells. 50000 cells were incubated with 100 nM **7** and 100 nM **8** for 30 min, 1h, 3h, 6h, and 24h, washed with PBS, and analyzed by flow cytometry. For microscopic analysis cells were additionally incubated with 5 μg/ml CellMask Deep Red Plasma membrane stain (C10046, Invitrogen, Thermo Fisher Scientific, Switzerland) for 10 min. Images were recorded with the Leica SP8 confocal point scanning microscope using a HC PL Apo CS 40x (NA 1.1) objective (Leica Microsystems, Germany). Comparisons between the conditions were performed using one-way ANOVA with Dunnett's multiple comparison posttest with a 0.05 confidence level accepted for statistical significance (*$P \leq 0.05$, **$P \leq 0.01$, ***$P \leq 0.001$, ****$P \leq 0.0001$).

**Example 1 - Dynamic light scattering**

The size characteristics of Man-PLL **1a-d** were analyzed by DLS with a Malvern Zetasizer Nano instrument (Malvern Panalytical, Malvern, UK). Samples containing 10 μM **1a** (0.38 mg $mL^{-1}$) or 2 μM **1b-d** (0.02-0.34 mg $mL^{-1}$) were allowed to equilibrate at 20 °C for two minutes prior to the experiments. Experiments were performed in triplicate and experimental data were analyzed with the manufacturer supplied software to obtain hydrodynamic diameters (Z-average), polydispersity indices, and size distribution data.

The size distribution profiles of Man-PLL **1a-d** in solution were elucidated by dynamic light scattering (DLS) experiments (Table 2, Figure 1). The measured hydrodynamic diameters ($D_h$) of **1a-d** reflect the degree of polymerization. A $D_h$ of 9.2 nm was found for Man-$PLL_{100}$ (**1a**), which is roughly doubled for the 250-mer **1b** (19.8 nm) and tripled for the larger 400-mers **1c** and **1d** (30 nm). The polydispersity indices (Đ) fall in a moderate range of 0.3 to 0.4, indicating the presence of multiple species in the polymer samples, as expected for the polydisperse PLL scaffolds.

**Table 2.** Biophysical characterization of Man-PLL **1a-d** by DLS.[a] Z-Average values are reported as hydrodynamic radii. [b] The standard deviation from three independent experiments is given as an estimation of experimental error. $D_h$, hydrodynamic diameter; Đ, polydispersity index.

| Man-PLL | DLS | |
|---|---|---|
| | $D_h$ [nm][a,b] | Đ |
| **1a** | 9.2 ± 0.4 | 0.4 ± 0.01 |
| **1b** | 19.8 ± 0.3 | 0.4 ± 0.05 |
| **1c** | 32.0 ± 0.4 | 0.3 ± 0.02 |
| **1d** | 27.2 ± 0.7 | 0.4 ± 0.02 |

**Example 2: Analytical ultracentrifugation**

Sedimentation velocity experiments were performed for 410 μl samples at concentrations of 0.8 mg $ml^{-1}$ (22 μM) for Man-PLL **1a**, 0.2 mg $ml^{-1}$ (2 μM) for Man-PLL **1b**, 1.3 mg $ml^{-1}$ (9 μM) for Man-PLL **1c**, and 0.2 mg $ml^{-1}$ (1 μM) for Man-PLL **1d** at 20 °C in 20 mM HEPES, 150 mM NaCl, 1 mM CaCl2, pH 7.4. Centrifugation was performed at 42,000 rpm (128,000 x g) using a Beckman XL-I Analytical Ultracentrifuge with an An-60 Ti rotor, and sedimentation was monitored using the interference optics with 12 mm double-sector charcoal-epon centerpieces. The data were analysed by fitting diffusion-deconvoluted differential sedimentation coefficient distributions (c(s) distributions) using the program SEDFIT (Schuck, P. (2000). Size-distribution analysis of macromolecules by sedimentation velocity ultracentrifugation and Lamm equation modeling. Biophysical Journal, 78(3), 1606-1619.), best-fit values of the frictional ratio ($f/f_0$), meniscus and bottom positions, and time- and radially-invariant noise profiles were determined for each data set. Values of buffer density and viscosity were obtained from Sednterp (Laue TM, Shah BD, Ridgeway TM, Pelletier SL (1992) in Analytical Ultracentrifugation in Biochemistry and Polymer Science, eds Harding SE, Rowe AJ, Horton JC (The Royal Society of

Chemistry, Cambridge), pp 90-125). Values of partial specific volume were calculated for individual components of Man-PLLs **1a-d**, namely lysine conjugated to mannose (0.797 ml $g^{-1}$) and for lysine conjugated to thioglycerol (0.772 ml $g^{-1}$) using the method of Durchschlag and Zipper (Durchschlag, H., & Zipper, P. (1994). Calculation of the partial volume of organic compounds and polymers. In Ultracentrifugation (pp. 20-39). Springer.). Partial specific volumes were then calculated for different polymers according to estimated mass fraction of mannose or thioglycerol conjugated lysine in the polymer (0.787 ml $g^{-1}$ for Man-PLL **1a**, 0.787 ml $g^{-1}$ for Man-PLL **1b**, 0.786 ml $g^{-1}$ for Man-PLL **1c**, and 0.790 ml $g^{-1}$ for Man-PLL **1d**. Signal-weighted average S values were calculated from c(s) distributions using the program GUSSI (Brautigam, C. A. (2015). Calculations and Publication-Quality Illustrations for Analytical Ultracentrifugation Data. Methods Enzymol, 562, 109-133). Values of $R_h$ were calculated from diffusion coefficients, which in turn were calculated from signal-weighted average S values and $f/f_0$ according to a standard scaling law (Brown, P. H., Balbo, A., & Schuck, P. (2008). Characterizing Protein-Protein Interactions by Sedimentation Velocity Analytical Ultracentrifugation. Current Protocols in Immunology, 81(1), 18.15.1-18.15.39.).

Sedimentation coefficients (s) were determined by analytical ultracentrifugation (AUC) (Table 3, Figure 2). Based on AUC data, the frictional ratio parameters ($f/f_0$) were calculated as well, providing information about the shape of macromolecules in solution. Whereas a value for $f/f_0$ of 1 corresponds to a circular shape, values >2.0 indicate a highly extended conformation. Based on sedimentation velocity data, a narrow and fairly simple distribution with an average sedimentation coefficient of 2.21 S was obtained for Man-$PLL_{100}$ (**1a**). Its fitted molecular weight of 39.6 kDa correlates well with the theoretical value of 37.8 kDa. For Man-$PLL_{100}$ (**1a**), the recorded frictional ratio of 1.4 suggests a globular shape, comparable to a folded protein. The hydrodynamic radius ($R_h$) calculated from the average sedimentation coefficient is 3.3 nm. On the other hand, experimental data for the larger glycopolymers **1b-d** calculations resulted in more complex distributions, revealing the presence of multiple species with sedimentation coefficients between 2 and 15 S. Molecular weight estimations for these species correlate not only to polymer monomers, but also dimers and higher order aggregates. The calculated $R_h$ from the average sedimentation coefficients are between 10.47 nm and 12.17 nm, a sign for larger particle sizes for the longer polymers compared with **1a**, which corroborates DLS data. Finally, based on the average frictional ratios with values between 2.37 and 2.52 a highly extended conformations of the polymers can be assumed.

**Table 3.** Biophysical characterization of Man-PLL **1a-d** by AUC. $^c$ Weight-Averaged over Range 1-20 S. f/f0, frictional ratio; $R_h$, hydrodynamic radius.

| Man-PLL | AUC | | |
|---|---|---|---|
| | Sedimentation coefficient [S]$^c$ | f/f0 | $R_h$ [nm] |
| **1a** | 2.21 | 1.41 | 3.3 |
| **1b** | 4.04 | 2.50 | 10.5 |
| **1c** | 5.08 | 2.37 | 10.8 |
| **1d** | 5.24 | 2.52 | 12.2 |

**Example 3: Competitive inhibition of viral glycoprotein binding to DC-SIGN$^+$ cells**

The potential of Man-PLL **1a-d** to inhibit the attachment of viral glycoproteins from SARS-CoV-2 (spike glycoprotein S1 subunit), Ebola (EBOV glycoprotein), or HIV (gp120 envelope glycoprotein) to DC-SIGN$^+$ B-THP-1 cells was investigated using a flow-cytometric assay (Figure 3). DC-SIGN$^+$ cells or isotype controls were incubated with the above glycoproteins labeled with the cyanine dye Cy5 in the presence of the glycopolymers **1a-d** or buffer. The amount of fluorescently labeled glycoprotein bound to the cell surface was then determined by the mean fluorescence intensity. The shortest Man-$PLL_{100}$ (**1a**) showed an $IC_{50}$ value of 43 nM for SARS-CoV-2 spike S1 and 36 nM for EBOV-gp. In contrast, the affinities for the intermediate length Man-$PLL_{250}$ (**1b**) are in the sub-nanomolar range (789 pM and 637 pM), which corresponds to an affinity gain by approximately a factor of 50. Further elongation of the polymer backbone (**1b** → **1c**) and increase in mannose loading (**1c** → **1d**) resulted in a less pronounced increase of the binding affinities by only a factor of two. With $IC_{50}$ values around 200 pM, **1d** is a highly potent inhibitor of the interaction between SARS-CoV-2 spike S1 and EBOV-gp glycoproteins with DC-SIGN. Compared with the monovalent epitope methyl α-D-mannoside (MeMan, $IC_{50}$ = 6.5 mM), the multivalent affinity enhancement is in the order of $10^7$. For the glycoprotein HIV gp120, an identical trend in affinity was observed. The absolute $IC_{50}$ values, however, differed by a factor of five, resulting in an $IC_{50}$ value of 1.0 nM for the most potent Man-$PLL_{400}$ (**1d**). The less potent inhibition of the gp120/DC-SIGN interaction is probably linked to a higher binding affinity of the heavily glycosylated gp120 compared with the other glycoproteins.

### Example 4: Blocking of DC-SIGN receptors

Prolonged incubation of DC-SIGN⁺ B-THP-1 cells with the most potent inhibitor **1d** effectively blocked the DC-SIGN receptors, making them unavailable for binding to SARS-CoV-2 spike S1 for up to 6 h (Figure 4A). An effect could still be observed after only a 30 min exposure of the cells (Figure 4B). Whereas no complete inhibition was achieved compared to a prolonged exposure to Man-$PLL_{400}$ (**1d**), the binding of SARS-CoV-2 spike S1was still significantly decreased for up to 6 h post incubation.

### Example 5: Uptake of Man-PLL 1a-d into target cells

To study endocytosis into acidic cell compartments by flow cytometry and fluorescence microscopy, Man-$PLL_{400}$ was labeled with the pH-sensitive fluorescent dye rhodamine 6G to yield **13.** As negative control, the fluorescently labeled $PLL_{400}$ **14** containing no mannose modification was used. Both polymers were incubated with DC-SIGN⁺ B-THP-1 and control B-THP-1 cells. Man-$PLL_{400}$ **13** was internalized highly specifically by DC-SIGN expressing B-THP-1 cells (Figure 4A) and the fluorescent signal increased over 6 h and diminished after 24 h hours, highlighting a complete removal of the compound as previously described by Hoppe and Lee (Hoppe, C. A.; Lee, Y. C. Accumulation of a Nondegradable Mannose Ligand within Rabbit Alveolar Macrophages. Receptor Reutihzation Is Independent of Ligand Degradation. Biochemistry 1984, 23 (8), 1723-1730.). Meanwhile no uptake in control B-THP-1 cells and only minor uptake of non-mannosylated control $PLL_{400}$ **14** into both cell types was observed (Figure 5)

### Example 6: Uptake of Man-PLL 1a-d into macrophages

To study endocytosis into acidic compartments of alveolar macrophages by flow cytometry and fluorescence microscopy, Man-$PLL_{400}$ was labeled with the pH-sensitive fluorescent dye rhodamine 6G (**13**, Man loading: 47% by NMR,). As negative control, the fluorescently labeled $PLL_{400}$ **14** containing no mannose modification was used. Both polymers were incubated with either unpolarized M0 or M2 polarized (20 ng/ml IL-4, 6h) MH-S alveolar macrophages showing different expression levels of mannose receptor 1 (Mrc1 or CD206), or control macrophages (phorbol 12-myristate 13-acetate differentiated THP-1 macrophages) lacking mannose receptor 1. Man-$PLL_{400}$ **13** was internalized highly specifically by CD206⁺ MH-S alveolar macrophages, without showing uptake by control THP-1 macrophages. The fluorescent signal increased time dependently and diminished after 24h highlighting a degradation of the compound in acidic cell compartments of the specialized phagocytic cells. Only minor non-specific uptake of non-mannosylated control $PLL_{400}$ **14** was observed in all cell types.

These observations further elucidate the mode of action of the Man-PLL, which, by multivalently binding to DC-SIGN on the cell surface, effectively inhibits binding of viral glycoproteins. Additionally, the compound is readily endocytosed via DC-SIGN. As a result, the availability of DC-SIGN on the cell surface is substantially reduced, and the glycopolymers are efficiently metabolized.

Further embodiments of the invention are disclosed in the following numbered items:

1. A compound according to the formula:

$$OS\text{-}A\text{-}B\text{-}(CH_2)_q\text{-}SH \qquad (I)$$

or a tautomer, pharmaceutically acceptable salt, hydrate, solvate or polymorph thereof, wherein:

OS is a glycan moiety comprising up to 9 mannose monomers, wherein OS is attached to A preferably through the reducing end of one of its monomers, preferably through an O, NH, S, or $CH_2$ group;
A is $C_1$-$C_7$-alkylene, $C_1$-$C_4$-alkylene-$(OCH_2CH_2)_p$O-$C_1$-$C_4$-alkylene, or $C_1$-$C_7$-alkylene-$R^b$-, wherein -$R^b$- is an optionally substituted arylene or an optionally substituted heteroarylene, and wherein p is 0 to 6, preferably p is 1,2, or 3, and more preferably p is 1;
B is -NHC(O)-, -S-, -$CH_2$-, -O-, -NH- or

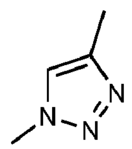
,

and

q is 0 to 6, preferably q is 1, 2, 3 or 4, and further preferably q is 1 or 2.

2. The compound of item 1, wherein the OS moiety is selected from:

α/β-D-mannopyranosyl,
α-D-mannopyranosyl-(1→2)-α/β-D-mannopyranosyl,
α-D-mannopyranosyl-(1→3)-α/β-D-mannopyranosyl,
α-D-mannopyranosyl-(1→4)-α/β-D-mannopyranosyl,
α-D-mannopyranosyl-(1→6)-α/β-D-mannopyranosyl,
α-D-mannopyranosyl-(1→2)-α-D-mannopyranosyl-(1→2)-α/β-D-mannopyranosyl,
α-D-mannopyranosyl-(1→2)-α-D-mannopyranosyl-(1→3)-α/β-D-mannopyranosyl,
α-D-mannopyranosyl-(1→2)-α-D-mannopyranosyl-(1→6)-α/β-D-mannopyranosyl,
α-D-mannopyranosyl-(1→3)-α-D-mannopyranosyl-(1→6)-α/β-D-mannopyranosyl,
α-D-mannopyranosyl-(1→3)-[α-D-mannopyranosyl-(1→6)]-α/β-D-mannopyranosyl,
α-D-mannopyranosyl-(1→2)-α-D-mannopyranosyl-(1→2)-α-D-mannopyranosyl-(1→3)-α/β-D-mannopyranosyl,
α-D-mannopyranosyl-(1→2)-α-D-mannopyranosyl-(1→3)-α-D-mannopyranosyl-(1→6)-α/β-D-mannopyranosyl,
α-D-mannopyranosyl-(1→2)-α-D-mannopyranosyl-(1→6)-α-D-mannopyranosyl-(1→6)-α/β-D-mannopyranosyl,
α-D-mannopyranosyl-(1→2)-α-D-mannopyranosyl-(1→6)-[α-D-mannopyranosyl-(1→3)]-α/β-D-mannopyranosyl,
α-D-mannopyranosyl-(1→2)-α-D-mannopyranosyl-(1→3)-[α-D-mannopyranosyl-(1→6)]-α/β-D-mannopyranosyl,
α-D-mannopyranosyl-(1→6)-α-D-mannopyranosyl-(1→6)-[α-D-mannopyranosyl-(1→3)]-α/β-D-mannopyranosyl,
α-D-mannopyranosyl-(1→3)-α-D-mannopyranosyl-(1→6)-[α-D-mannopyranosyl-(1→3)]-α/β-D-mannopyranosyl, and
α-D-mannopyranosyl-(1→6)-[α-D-mannopyranosyl-(1→3)]-α-D-mannopyranosyl-(1→6)-α/β-D-mannopyranosyl.

3. The compound of item 1 or 2, according to the formula:

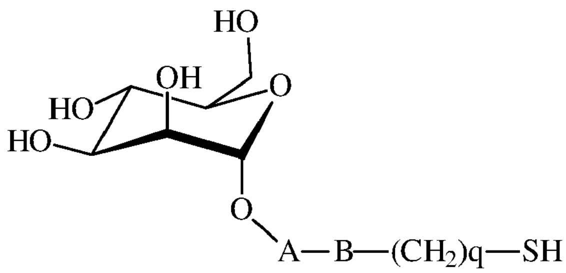

or an anomer, tautomer, pharmaceutically acceptable salt, hydrate, solvate or polymorph thereof, wherein:

A is $C_1$-$C_7$-alkylene, $C_1$-$C_4$-alkylene-$(OCH_2CH_2)_pO$-$C_1$-$C_4$-alkylene, or $C_1$-$C_7$-alkylene-$R^b$-, wherein -$R^b$- is an optionally substituted arylene or an optionally substituted heteroarylene, and wherein p is 0 to 6, preferably p is 1,2, or 3, and more preferably p is 1;
B is -NHC(O)-, -S-, -$CH_2$-, -O-, -NH- or

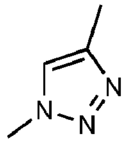,

and
q is 0 to 6, preferably q is 1, 2, 3 or 4, and further preferably q is 1 or 2.

4. The compound of any one of items 1 to 3, wherein A is $C_1$-$C_7$-alkylene, preferably $-CH_2CH_2CH_2-$.

5. The compound of any one of items 1 to 4, wherein B is -NHC(O)-.

6. The compound of any one of items 1 to 5, wherein q is 3.

7. The compound of any one of items 1 to 6, according to the formula:

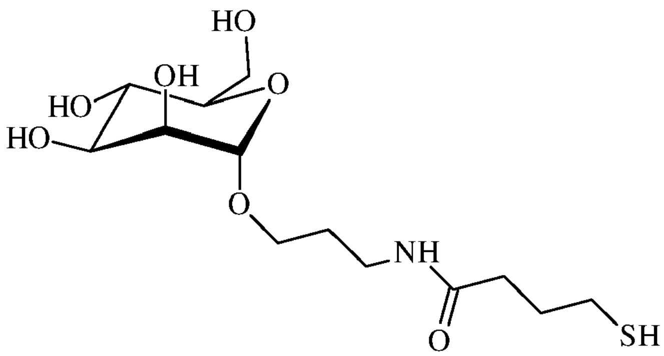

or an anomer, tautomer, pharmaceutically acceptable salt, hydrate, solvate or polymorph thereof.

8. A polymer comprising a polymer backbone and a multitude of compounds of any one of items 1 to 7, wherein said compounds are connected to the polymer backbone through the -SH group.

9. The polymer of item 8, wherein the polymer backbone is an α-amino acid polymer, an acrylic acid or methacrylic acid polymer or copolymer, an N-vinyl-2-pyrrolidone-vinyl alcohol copolymer, a chitosan polymer, or a polyphosphazene polymer.

10. The polymer of item 8 or 9, wherein the polymer backbone is an α-amino acid polymer, and wherein said α-amino acid is lysine, ornithine, glutamic acid, aspartic acid, histidine, cysteine, or serine, and wherein the polymer is optionally PEGylated.

11. The polymer of any one of items 8 to 10, wherein the polymer backbone is poly-lysine.

12. The polymer of any one of items 8 to 11, wherein the weight average molecular weight of the polymer is at least 30000 Da, preferably wherein the weight average molecular weight of the polymer is at least 90 000 Da, more preferably wherein the weight average molecular weight of the polymer is between 90 000 Da and 500 000 Da.

13. The polymer of any one of items 8 to 12, wherein the percentage of loading of the compound according to any one of items 1 to 7 onto the polymer backbone is between 10 and 90%, preferably between 20 and 80%, more preferably between 30 and 70%.

14. The polymer of any one of items 8 to 13, wherein the polymer exhibits a hydrodynamic radius of at least 3 nm as measured by analytical ultracentrifugation, preferably wherein the polymer exhibits a hydrodynamic radius of at least 10 nm as measured by analytical ultracentrifugation,
and/or
wherein the polymer exhibits a hydrodynamic diameter of at least 9 nm as measured by dynamic light scattering, preferably wherein the polymer exhibits a hydrodynamic diameter of at least 19 nm as measured by dynamic light scattering.

15. The polymer of any one of items 8 to 14, comprising x repeating units of the following formula:

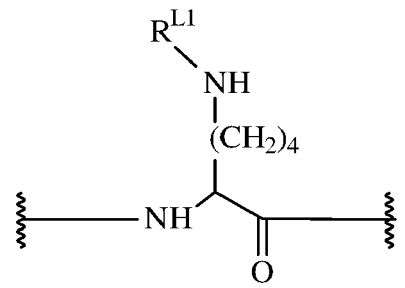

or a tautomer, pharmaceutically acceptable salt, hydrate, solvate or polymorph thereof,
wherein $R^{L1}$ is:

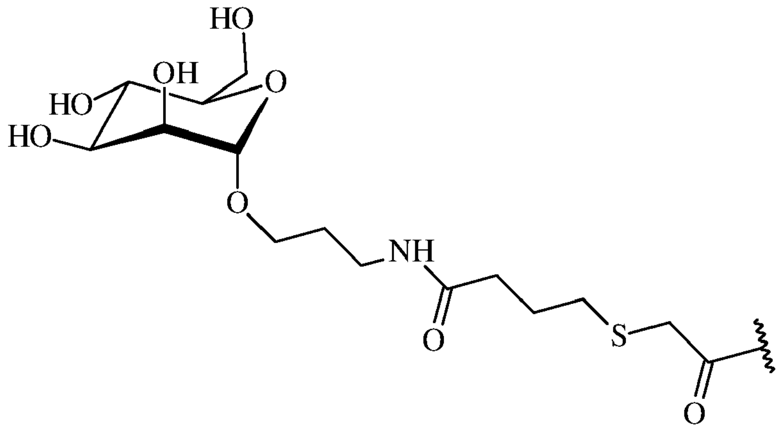

wherein the total number of repeating units n is at least 100, preferably n is at least 250, more preferably n is in the range from 250 to 800; and
wherein x/n is between 10 and 90%, preferably between 20 and 80%, more preferably between 30 and 70%.

16. A pharmaceutical composition comprising the compound of any one of items 1 to 7, or a polymer according to any one of items 8 to 15, and at least one pharmaceutically acceptable carrier.

17. The compound of any one of items 1 to 7, the polymer of any one of items 8 to 15, or the pharmaceutical composition of item 16 for use as a medicament.

18. The compound of any one of items 1 to 7, the polymer of any one of items 8 to 15, or the pharmaceutical composition of item 16 for use in the treatment and/or prevention and/or alleviation of a viral infection.

19. The compound for use, the polymer for use, or the pharmaceutical composition for use of item 18, wherein the viral infection is caused by a virus characterized by a mannose-comprising glycan epitopes on envelope glycoproteins.

20. The compound for use, the polymer for use, or the pharmaceutical composition for use of item 18 or 19, wherein the viral infection is caused by a virus selected from SARS-Coronavirus 2, SARS-Coronavirus, MERS-Coronavirus, HIV, influenza A virus, hepatitis C virus, zika virus, dengue virus, ebola virus, west-Nile virus, Marburg virus, chikungunya virus, and Japanese encephalitis virus.

21. The compound for use, the polymer for use, or the pharmaceutical composition for use of any one of items 18 to 20, wherein the compound, the polymer, or the pharmaceutical composition interferes with DC-SIGN-mediated attachment and internalization of viral particles.

22. The compound for use, the polymer for use, or the pharmaceutical composition for use of any one of items 18 to 21, wherein the compound, the polymer, or the pharmaceutical composition is administered through pulmonary administration route or intravenous administration route.

**Claims**

1. A compound according to the formula:

$$OS\text{-}A\text{-}B\text{-}(CH_2)_q\text{-}SH \qquad (I)$$

or a tautomer, pharmaceutically acceptable salt, hydrate, solvate or polymorph thereof, wherein:

OS is a glycan moiety comprising up to 9 mannose monomers, wherein OS is attached to A preferably through the reducing end of one of its monomers, preferably through an O, NH, S, or $CH_2$ group;
A is $C_1$-$C_7$-alkylene, $C_1$-$C_4$-alkylene-$(OCH_2CH_2)_p$O-$C_1$-$C_4$-alkylene, or $C_1$-$C_7$-alkylene-$R^b$-, wherein -$R^b$- is an optionally substituted arylene or an optionally substituted heteroarylene, and wherein p is 0 to 6, preferably p is 1,2, or 3, and more preferably p is 1;
B is -NHC(O)-, -S-, -$CH_2$-, -O-, -NH- or

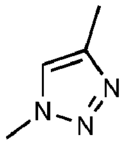
,

and
q is 0 to 6, preferably q is 1, 2, 3 or 4, and further preferably q is 1 or 2.

2. The compound of claim 1, wherein the OS moiety is selected from:

α/β-D-mannopyranosyl,
α-D-mannopyranosyl-(1→2)-α/β-D-mannopyranosyl,
α-D-mannopyranosyl-(1→3)-α/β-D-mannopyranosyl,
α-D-mannopyranosyl-(1→4)-α/β-D-mannopyranosyl,
α-D-mannopyranosyl-(1→6)-α/β-D-mannopyranosyl,
α-D-mannopyranosyl-(1→2)-α-D-mannopyranosyl-(1→2)-α/β-D-mannopyranosyl,
α-D-mannopyranosyl-(1→2)-α-D-mannopyranosyl-(1→3)-α/β-D-mannopyranosyl,
α-D-mannopyranosyl-(1→2)-α-D-mannopyranosyl-(1→6)-α/β-D-mannopyranosyl,
α-D-mannopyranosyl-(1→3)-α-D-mannopyranosyl-(1→6)-α/β-D-mannopyranosyl,
α-D-mannopyranosyl-(1→3)-[α-D-mannopyranosyl-(1→6)]-α/β-D-mannopyranosyl,
α-D-mannopyranosyl-(1→2)-α-D-mannopyranosyl-(1→2)-α-D-mannopyranosyl-(1→3)-α/β-D-mannopyranosyl,
α-D-mannopyranosyl-(1→2)-α-D-mannopyranosyl-(1→3)-α-D-mannopyranosyl-(1→6)-α/β-D-mannopyranosyl,
α-D-mannopyranosyl-(1→2)-α-D-mannopyranosyl-(1→6)-α-D-mannopyranosyl-(1→6)-α/β-D-mannopyranosyl,
α-D-mannopyranosyl-(1→2)-α-D-mannopyranosyl-(1→6)-[α-D-mannopyranosyl-(1→3)]-α/β-D-mannopyranosyl,
α-D-mannopyranosyl-(1→2)-α-D-mannopyranosyl-(1→3)-[α-D-mannopyranosyl-(1→6)]-α/β-D-mannopyranosyl,
α-D-mannopyranosyl-(1→6)-α-D-mannopyranosyl-(1→6)-[α-D-mannopyranosyl-(1→3)]-α/β-D-mannopyranosyl,
α-D-mannopyranosyl-(1→3)-α-D-mannopyranosyl-(1→6)-[α-D-mannopyranosyl-(1→3)]-α/β-D-mannopyranosyl, and
α-D-mannopyranosyl-(1→6)-[α-D-mannopyranosyl-(1→3)]-α-D-mannopyranosyl-(1→6)-α/β-D-mannopyranosyl.

3. The compound of claim 1 or 2, according to the formula:

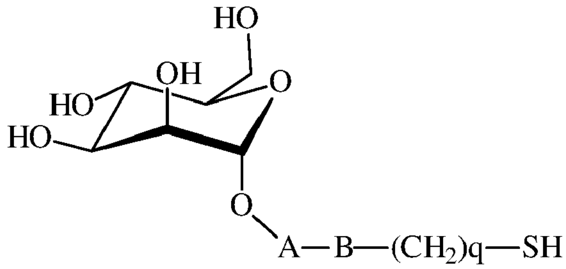

or an anomer, tautomer, pharmaceutically acceptable salt, hydrate, solvate or polymorph thereof, wherein:

A is $C_1$-$C_7$-alkylene, $C_1$-$C_4$-alkylene-$(OCH_2CH_2)_pO$-$C_1$-$C_4$-alkylene, or $C_1$-$C_7$-alkylene-$R^b$-, wherein -$R^b$- is an optionally substituted arylene or an optionally substituted heteroarylene, and wherein p is 0 to 6, preferably p is 1,2, or 3, and more preferably p is 1;
B is -NHC(O)-, -S-, -$CH_2$-, -O-, -NH- or

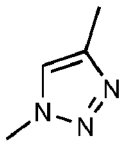,

and
q is 0 to 6, preferably q is 1, 2, 3 or 4, and further preferably q is 1 or 2.

**4.** The compound of any one of claims 1 to 3, wherein A is $C_1$-$C_7$-alkylene, preferably -$CH_2CH_2CH_2$-, and/or wherein B is -NHC(O)-, and/or wherein q is 3.

**5.** The compound of any one of claims 1 to 4, according to the formula:

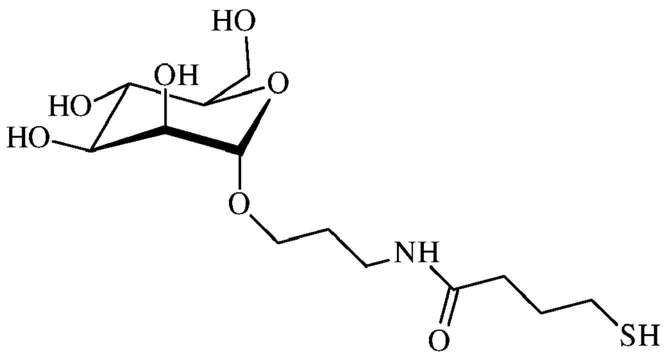

or an anomer, tautomer, pharmaceutically acceptable salt, hydrate, solvate or polymorph thereof.

**6.** A polymer comprising a polymer backbone and a multitude of compounds of any one of claims 1 to 5, wherein said compounds are connected to the polymer backbone through the -SH group.

**7.** The polymer of claim 6, wherein the polymer backbone is an $\alpha$-amino acid polymer, an acrylic acid or methacrylic acid polymer or copolymer, an N-vinyl-2-pyrrolidone-vinyl alcohol copolymer, a chitosan polymer, or a polyphosphazene polymer.

**8.** The polymer of claim 6 or 7, wherein the polymer backbone is an $\alpha$-amino acid polymer, and wherein said $\alpha$-amino acid is lysine, ornithine, glutamic acid, aspartic acid, histidine, cysteine, or serine, and wherein the polymer is optionally PEGylated, preferably

**9.** The polymer of any one of claims 6 to 8, wherein the polymer backbone is poly-lysine.

**10.** The polymer of any one of claims 6 to 9, wherein the weight average molecular weight of the polymer is at least 30000 Da, preferably wherein the weight average molecular weight of the polymer is at least 90 000 Da, more preferably wherein the weight average molecular weight of the polymer is between 90 000 Da and 500 000 Da, and/or
wherein the percentage of loading of the compound according to any one of claims 1 to 7 onto the polymer backbone is between 10 and 90%, preferably between 20 and 80%, more preferably between 30 and 70%,
and/or
wherein the polymer exhibits a hydrodynamic radius of at least 3 nm as measured by analytical ultracentrifugation, preferably wherein the polymer exhibits a hydrodynamic radius of at least 10 nm as measured by analytical ultracentrifugation,
and/or
wherein the polymer exhibits a hydrodynamic diameter of at least 9 nm as measured by dynamic light scattering, preferably wherein the polymer exhibits a hydrodynamic diameter of at least 19 nm as measured by dynamic light scattering.

**11.** The polymer of any one of claims 6 to 10, comprising x repeating units of the following formula:

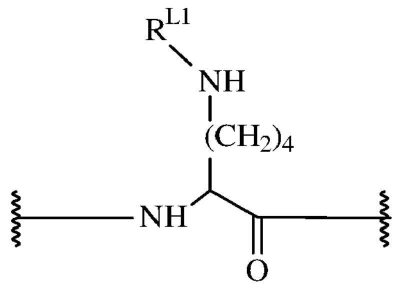

or a tautomer, pharmaceutically acceptable salt, hydrate, solvate or polymorph thereof,
wherein $R^{L1}$ is:

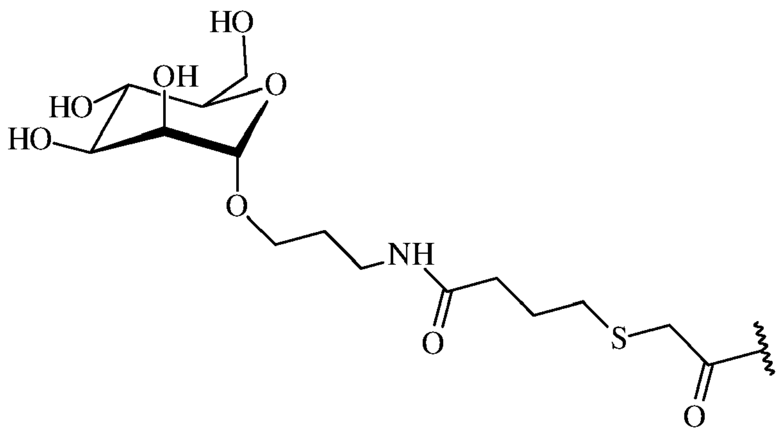

wherein the total number of repeating units n is at least 100, preferably n is at least 250, more preferably n is in the range from 250 to 800; and
wherein x/n is between 10 and 90%, preferably between 20 and 80%, more preferably between 30 and 70%.

**12.** A pharmaceutical composition comprising the compound of any one of claims 1 to 5, or a polymer according to any one of claims 6 to 11, and at least one pharmaceutically acceptable carrier.

**13.** The compound of any one of claims 1 to 5, the polymer of any one of claims 6 to 11, or the pharmaceutical composition of claim 12 for use as a medicament.

**14.** The compound of any one of claims 1 to 5, the polymer of any one of claims 6 to 11, or the pharmaceutical composition of claim 12 for use in the treatment and/or prevention and/or alleviation of a viral infection.

**15.** The compound for use, the polymer for use, or the pharmaceutical composition for use of claim 14, wherein the viral infection is caused by a virus **characterized by** a mannose-comprising glycan epitopes on envelope glycoproteins, and/or
wherein the viral infection is caused by a virus selected from SARS-Coronavirus 2, SARS-Coronavirus, MERS-Coronavirus, HIV, influenza A virus, hepatitis C virus, zika virus, dengue virus, ebola virus, west-Nile virus, Marburg virus, chikungunya virus, and Japanese encephalitis virus,
and/or
wherein the compound, the polymer, or the pharmaceutical composition interferes with DC-SIGN-mediated attachment and internalization of viral particles,
and/or
wherein the compound, the polymer, or the pharmaceutical composition is administered through pulmonary administration route or intravenous administration route.

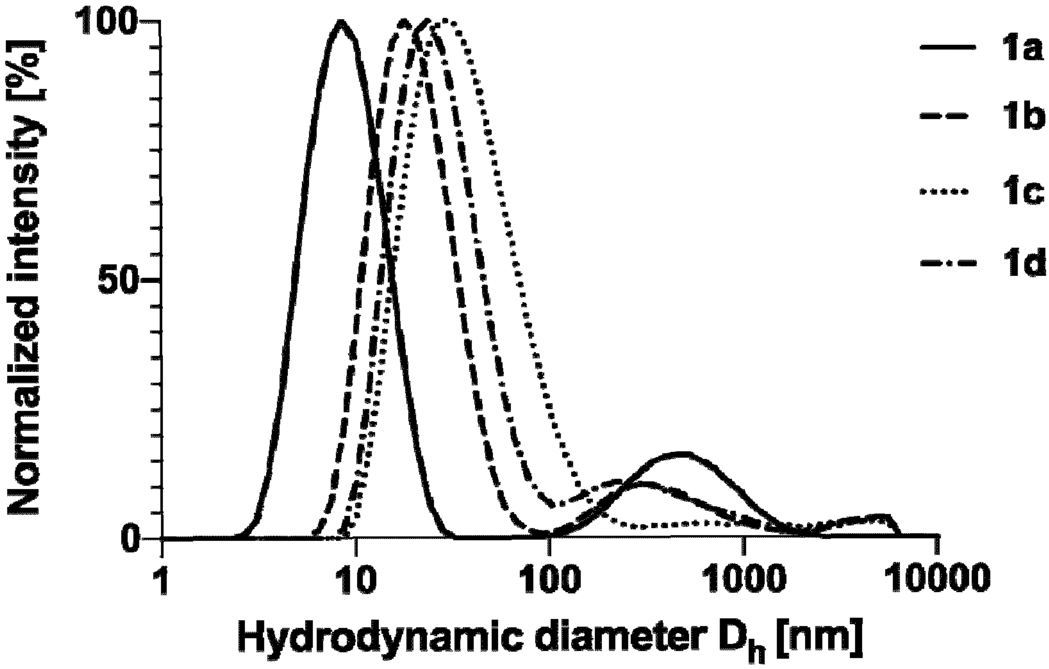
100
50
0
1
10
100
1000
10000
Normalized intensity [%]
Hydrodynamic diameter D$_h$ [nm]
1a
1b
1c
1d

Figure 1

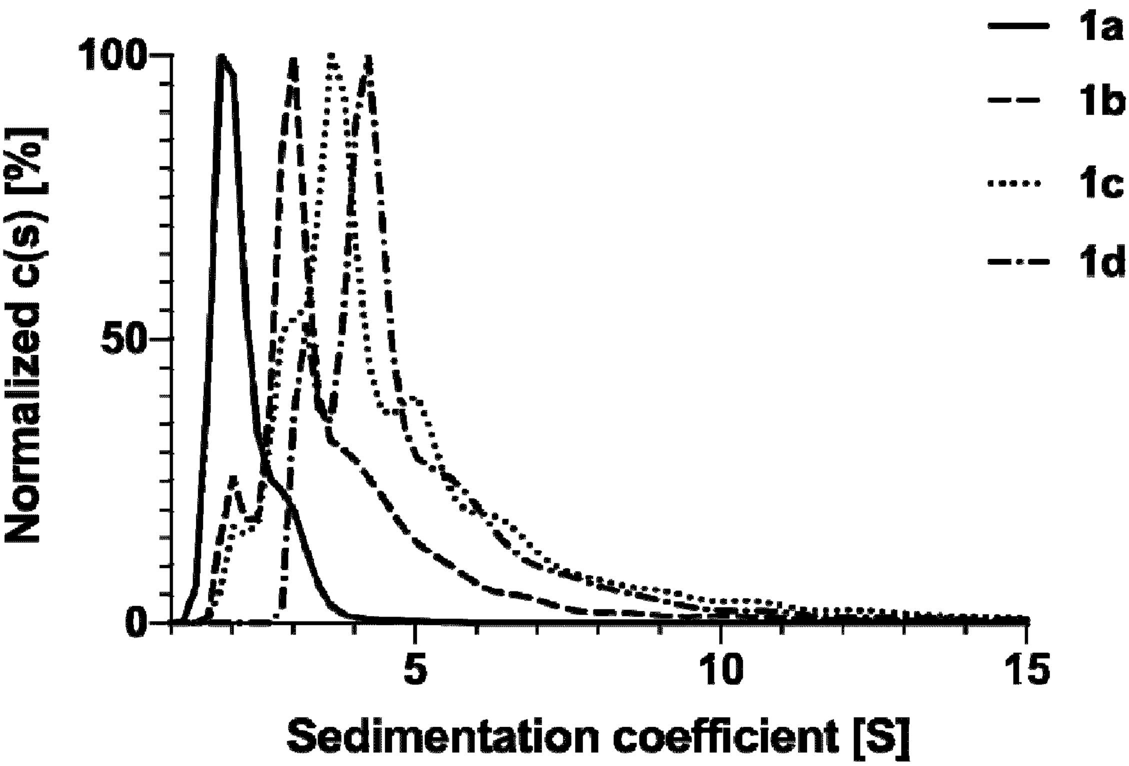
1a
1b
1c
1d
100
50
0
5
10
15
Normalized c(s) [%]
Sedimentation coefficient [S]

Figure 2

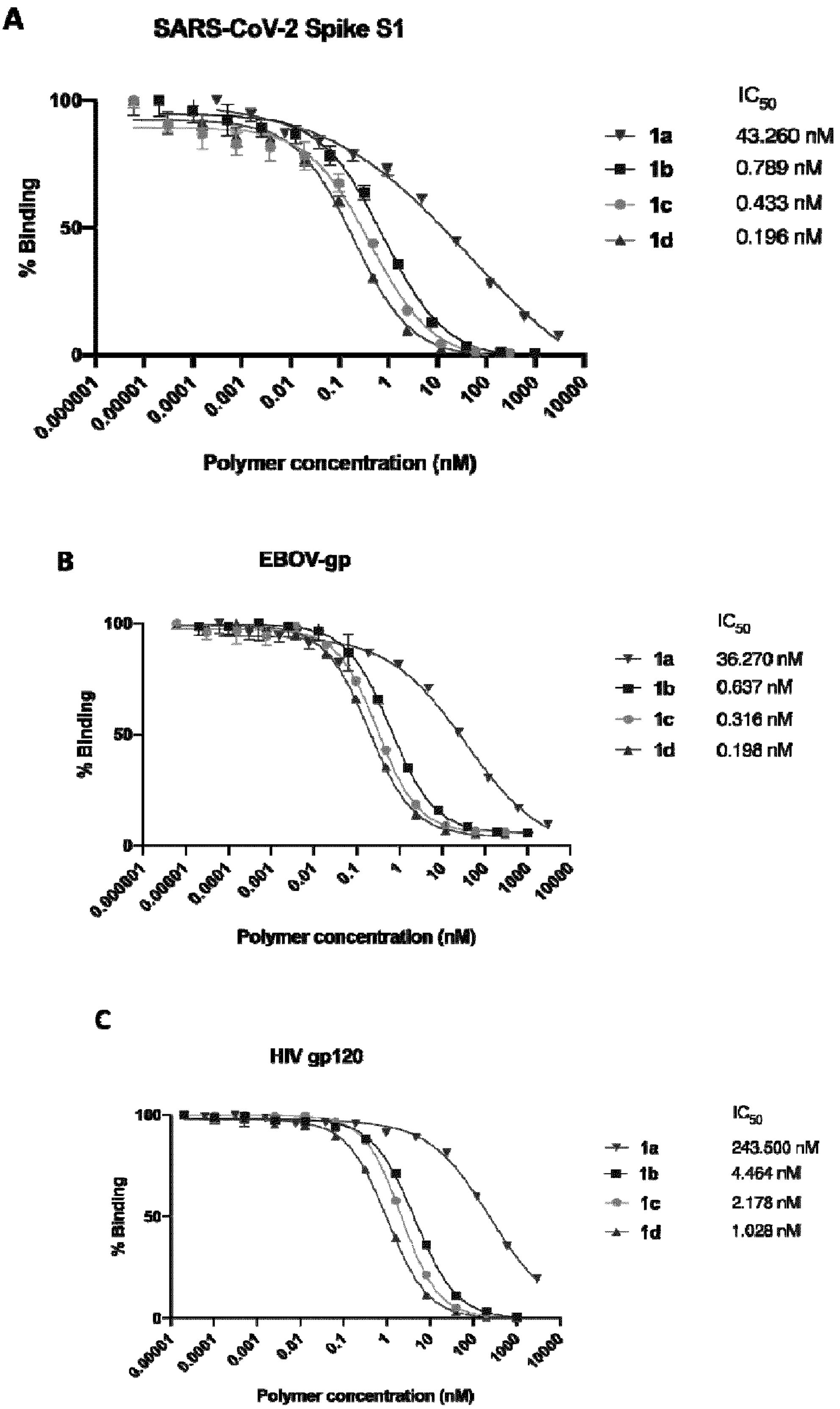
A
SARS-CoV-2 Spike S1
IC50
1a 43.260 nM
1b 0.789 nM
1c 0.433 nM
1d 0.196 nM
% Binding
Polymer concentration (nM)
B
EBOV-gp
IC50
1a 36.270 nM
1b 0.637 nM
1c 0.316 nM
1d 0.198 nM
% Binding
Polymer concentration (nM)
C
HIV gp120
IC50
1a 243.500 nM
1b 4.464 nM
1c 2.178 nM
1d 1.028 nM
% Binding
Polymer concentration (nM)

Figure 3

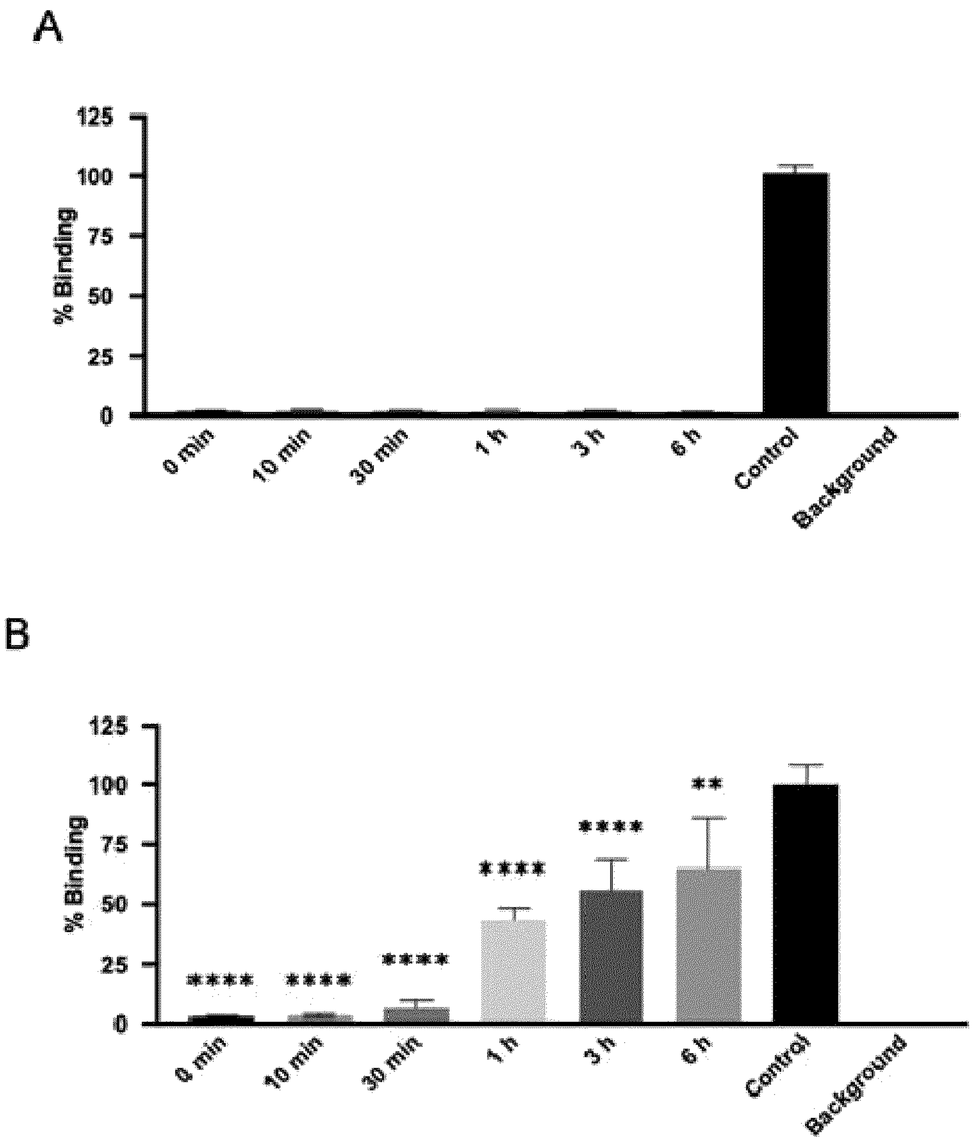
A
125
100
75
50
25
0
% Binding
0 min
10 min
30 min
1 h
3 h
6 h
Control
Background
B
125
100
75
50
25
0
% Binding
****
****
****
****
****
**
0 min
10 min
30 min
1 h
3 h
6 h
Control
Background

Figure 4

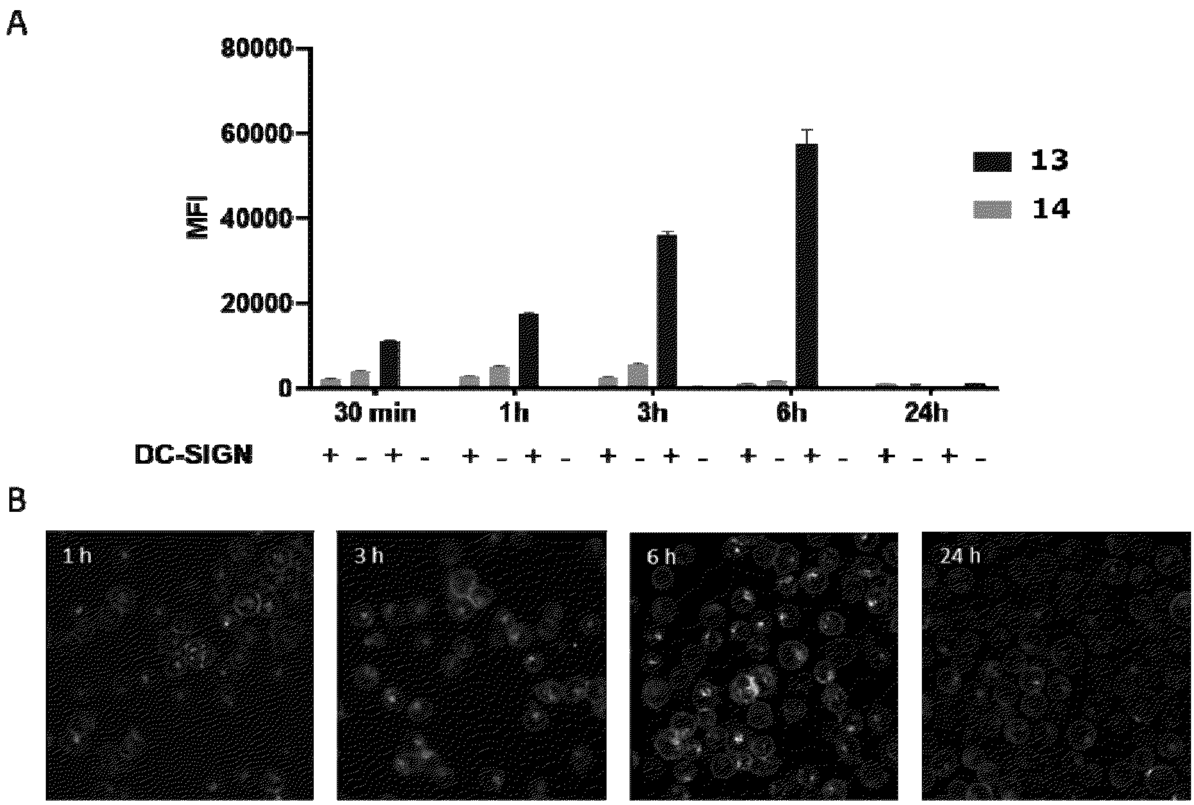
A
80000
60000
40000
20000
0
MFI
13
14
30 min
1h
3h
6h
24h
DC-SIGN
+ - + - + - + - + - + - + - + - + - + -
B
1 h
3 h
6 h
24 h

Figure 5

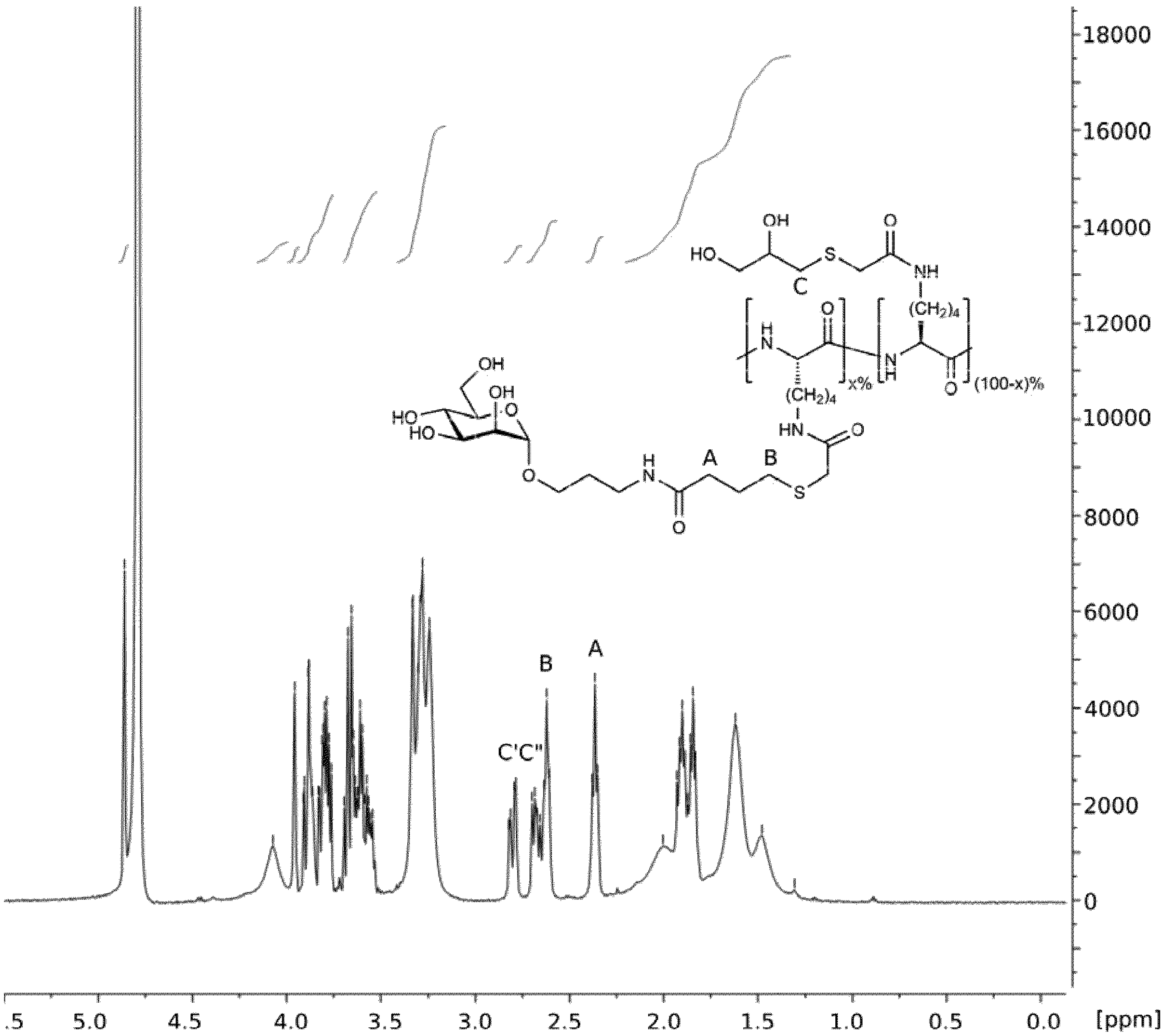
18000
16000
14000
12000
10000
8000
6000
4000
2000
0
B
A
C'C"
.5
5.0
4.5
4.0
3.5
3.0
2.5
2.0
1.5
1.0
0.5
0.0
[ppm]

Figure 6A.

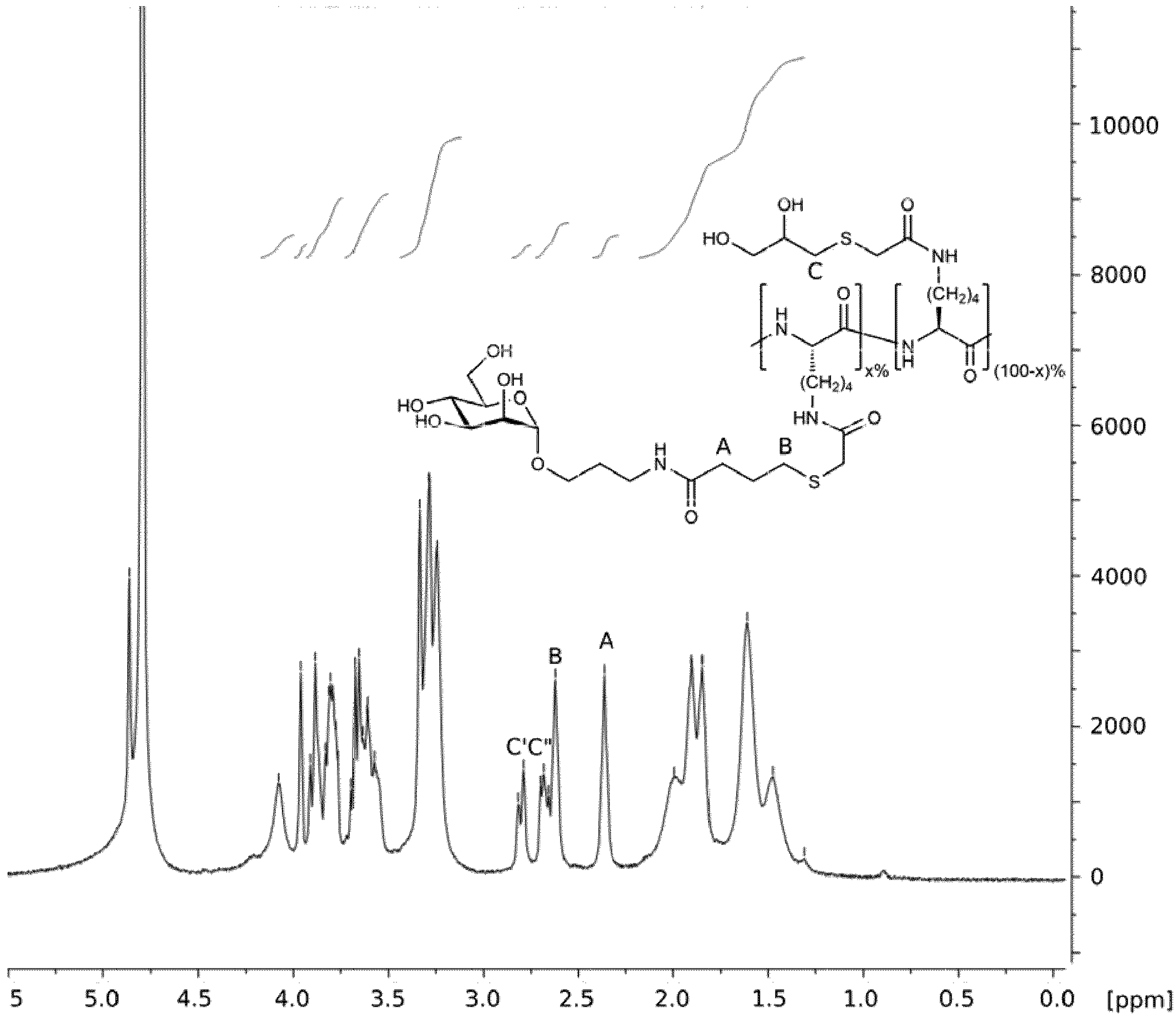
10000
8000
6000
4000
2000
0
B
A
C'C''
5
5.0
4.5
4.0
3.5
3.0
2.5
2.0
1.5
1.0
0.5
0.0
[ppm]

Figure 6B.

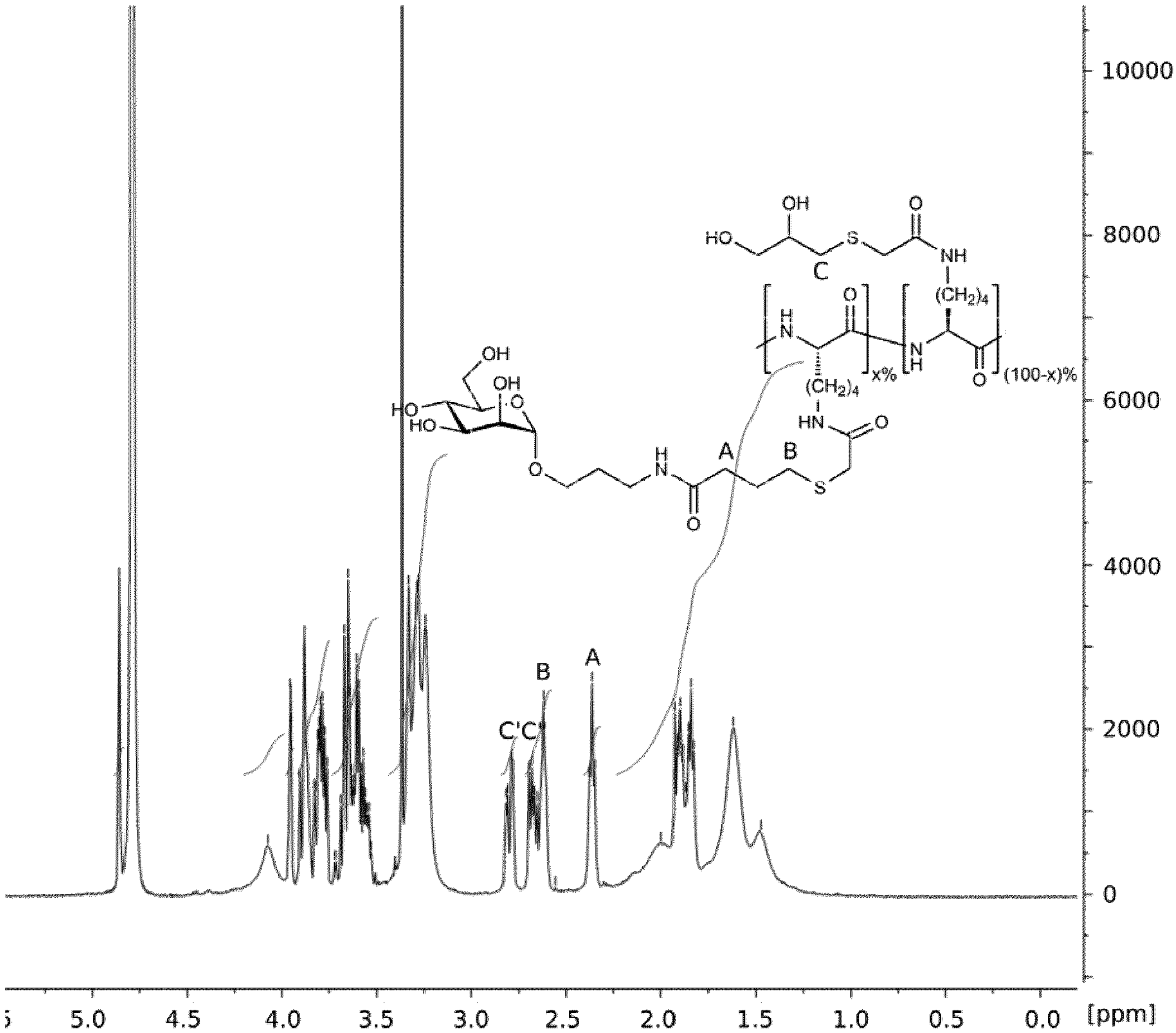


Figure 6C.

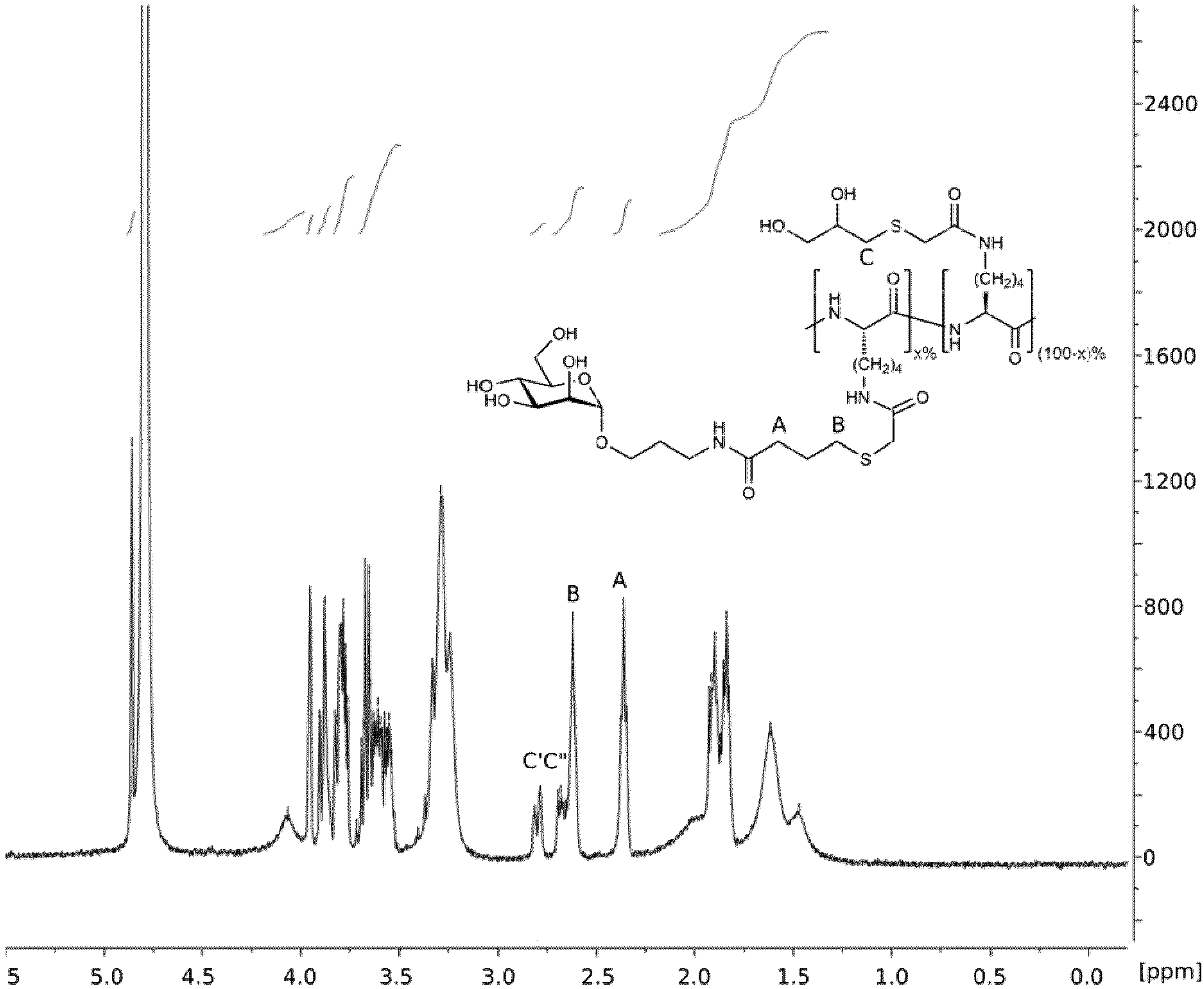
2400
2000
1600
1200
800
400
0
B
A
C'C"
5
5.0
4.5
4.0
3.5
3.0
2.5
2.0
1.5
1.0
0.5
0.0
[ppm]

Figure 6D.

Europäisches Patentamt
European Patent Office
Office européen des brevets

# EUROPEAN SEARCH REPORT

Application Number
EP 20 20 0905

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/093524 A2 (GEN HOSPITAL CORP [US]; MASSACHUSETTS INST TECHNOLOGY [US] ET AL.) 8 September 2006 (2006-09-08) * figure 1 * ----- | 1-4,6 | INV. C07H15/26 A61P31/12 A61P31/16 A61P31/18 |
| X | RATNER DANIEL M. ET AL: "Probing Protein-Carbohydrate Interactions with Microarrays of Synthetic Oligosaccharides", CHEMBIOCHEM, vol. 5, no. 3, 5 March 2004 (2004-03-05), pages 379-383, XP055788593, ISSN: 1439-4227, DOI: 10.1002/cbic.200300804 * page 381 * ----- | 1-4,6 | |
| X | KATARZYNA GORSKA ET AL: "DNA-Templated Homo- and Heterodimerization of Peptide Nucleic Acid Encoded Oligosaccharides that Mimick the Carbohydrate Epitope of HIV", ANGEWANDTE CHEMIE INTERNATIONAL EDITION, vol. 48, no. 41, 28 September 2009 (2009-09-28), pages 7695-7700, XP055248598, ISSN: 1433-7851, DOI: 10.1002/anie.200903328 * the whole document * ----- -/-- | 1-4,6 | TECHNICAL FIELDS SEARCHED (IPC) C07H A61P |

The present search report has been drawn up for all claims

2

| Place of search | Date of completion of the search | Examiner |
|---|---|---|
| Munich | 26 March 2021 | Klein, Didier |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

Europäisches Patentamt
European Patent Office
Office européen des brevets

# EUROPEAN SEARCH REPORT

Application Number
EP 20 20 0905

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TAKAHASHI DAISUKE ET AL: "The synthesis of carbohydrate microarrays by S-alkylation of the glass-supported 2-bromoacetamides", CHEMISTRY LETTERS, CHEMICAL SOCIETY OF JAPAN,NIPPON KAGAKUKAI, JP, vol. 37, no. 12, 1 January 2008 (2008-01-01), pages 1252-1253, XP009167089, ISSN: 0366-7022, DOI: 10.1246/CL.2008.1252 * compound 4 * ----- | 1-4,6 | |
| X | CHRISTIAN WENDELN ET AL: "Rapid Preparation of Multifunctional Surfaces for Orthogonal Ligation by Microcontact Chemistry", CHEMISTRY - A EUROPEAN JOURNAL, vol. 18, no. 19, 7 May 2012 (2012-05-07), pages 5880-5888, XP055117433, ISSN: 0947-6539, DOI: 10.1002/chem.201103422 * figure 3; compound 5 * ----- | 1-4,6 | TECHNICAL FIELDS SEARCHED (IPC) |
| X | GÖTZE SEBASTIAN ET AL: "Diagnosis of toxoplasmosis using a synthetic glycosylphosphatidylinositol glycan", ANGEWANDTE CHEMIE (INTERNATIONAL EDITION), WILEY - VCH VERLAG GMBH & CO, DE, vol. 53, no. 50, 8 December 2014 (2014-12-08), pages 13701-13705, XP002782558, ISSN: 1521-3773, DOI: 10.1002/ANIE.201406706 * figure 3 * ----- -/-- | 1-4 | |

The present search report has been drawn up for all claims

2

| Place of search | Date of completion of the search | Examiner |
|---|---|---|
| Munich | 26 March 2021 | Klein, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 20 0905

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YONGJUN GAO ET AL: "Efficient Preparation of Glycoclusters from Silsesquioxanes", ORGANIC LETTERS, vol. 6, no. 20, 9 September 2004 (2004-09-09), pages 3457-3460, XP055370633, US ISSN: 1523-7060, DOI: 10.1021/ol040043a * compound 12 * ----- | 1-4 | |
| X | BHATTARAI JAY K ET AL: "Electrochemical impedance spectroscopy study of Concanavalin A binding to self-assembled monolayers of mannosides on gold wire electrodes", JOURNAL OF ELECTROANALYTICAL CHEMISTRY, ELSEVIER, AMSTERDAM, NL, vol. 780, 28 September 2016 (2016-09-28), pages 311-320, XP029780131, ISSN: 1572-6657, DOI: 10.1016/J.JELECHEM.2016.09.045 * chart 1 * ----- | 1-4 | TECHNICAL FIELDS SEARCHED (IPC) |
| X | EP 3 492 481 A1 (VAXXILON AG [CH]) 5 June 2019 (2019-06-05) * page 18 - page 33; compounds i'a/b/c/e/f-9 * ----- | 1-4 | |
| X | US 2017/199185 A1 (MIYAZAWA YUTA [JP] ET AL) 13 July 2017 (2017-07-13) * page 18, paragraph 0351 * ----- | 1-4 | |
| X | WO 2007/033329 A1 (UNIV MARYLAND BIOTECH INST [US]; WANG LAI-XI [US] ET AL.) 22 March 2007 (2007-03-22) * the whole document * ----- -/-- | 1-15 | |

The present search report has been drawn up for all claims

2

| Place of search | Date of completion of the search | Examiner |
|---|---|---|
| Munich | 26 March 2021 | Klein, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 5

Europäisches Patentamt
European Patent Office
Office européen des brevets

# EUROPEAN SEARCH REPORT

Application Number
EP 20 20 0905

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 2 594 575 A1 (UNIV STRASBOURG [FR]; CENTRE NAT RECH SCIENT [FR]) 22 May 2013 (2013-05-22) * the whole document * ----- | 1-15 | |
| A | TABARANI G ET AL: "Mannose hyperbranched dendritic polymers interact with clustered organization of DC-SIGN and inhibit gp120 binding", FEBS LETTERS, ELSEVIER, AMSTERDAM, NL, vol. 580, no. 10, 1 May 2006 (2006-05-01), pages 2402-2408, XP028030480, ISSN: 0014-5793, DOI: 10.1016/J.FEBSLET.2006.03.061 [retrieved on 2006-05-01] * the whole document * ----- | 1-15 | |
| A | FRISON N ET AL: "Oligolysine-based Oligosaccharide Clusters", JOURNAL OF BIOLOGICAL CHEMISTRY, AMERICAN SOCIETY FOR BIOCHEMISTRY AND MOLECULAR BIOLOGY, vol. 278, no. 26, 27 June 2003 (2003-06-27), pages 23922-23929, XP008124617, ISSN: 0021-9258, DOI: 10.1074/JBC.M302483200 [retrieved on 2003-04-14] * the whole document * ----- | 1-15 | TECHNICAL FIELDS SEARCHED (IPC) |

-/--

The present search report has been drawn up for all claims

| Place of search | Date of completion of the search | Examiner |
|---|---|---|
| Munich | 26 March 2021 | Klein, Didier |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches Patentamt
European Patent Office
Office européen des brevets

# EUROPEAN SEARCH REPORT

Application Number
EP 20 20 0905

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | Cramer Jonathan ET AL: "Poly-L-lysine Glycoconjugates Inhibit DC-SIGN-mediated Attachment of Pandemic Viruses", ChemRxiv (2020) 1-32, 2020, 9 October 2020 (2020-10-09), pages 1-32, XP055788892, DOI: 10.26434/chemrxiv.13072025.v1 Retrieved from the Internet: URL:https://s3-eu-west-1.amazonaws.com/itempdf74155353254prod/13072025/Poly-L-lysine_Glycoconjugates_Inhibit_DC-SIGN-mediated_Attachment_of_Pandemic_Viruses_v1.pdf [retrieved on 2021-03-23] ----- | | |

TECHNICAL FIELDS SEARCHED (IPC)

The present search report has been drawn up for all claims

2

| Place of search | Date of completion of the search | Examiner |
|---|---|---|
| Munich | 26 March 2021 | Klein, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 5 of 5

## ANNEX TO THE EUROPEAN SEARCH REPORT ON EUROPEAN PATENT APPLICATION NO. EP 20 20 0905

This annex lists the patent family members relating to the patent documents cited in the above-mentioned European search report. The members are as contained in the European Patent Office EDP file on
The European Patent Office is in no way liable for these particulars which are merely given for the purpose of information.

26-03-2021

| Patent document cited in search report | Publication date | Patent family member(s) | Publication date |
|---|---|---|---|
| WO 2006093524 A2 | 08-09-2006 | US 2009004218 A1 | 01-01-2009 |
| | | WO 2006093524 A2 | 08-09-2006 |
| EP 3492481 A1 | 05-06-2019 | AU 2018375986 A1 | 16-07-2020 |
| | | CA 3082951 A1 | 06-06-2019 |
| | | CN 111448204 A | 24-07-2020 |
| | | EP 3492481 A1 | 05-06-2019 |
| | | EP 3717498 A1 | 07-10-2020 |
| | | JP 2021504438 A | 15-02-2021 |
| | | KR 20200106033 A | 10-09-2020 |
| | | WO 2019106200 A1 | 06-06-2019 |
| US 2017199185 A1 | 13-07-2017 | JP 5960374 B2 | 02-08-2016 |
| | | JP WO2015182770 A1 | 20-04-2017 |
| | | US 2017199185 A1 | 13-07-2017 |
| | | WO 2015182770 A1 | 03-12-2015 |
| WO 2007033329 A1 | 22-03-2007 | CA 2663633 A1 | 22-03-2007 |
| | | EP 1937308 A1 | 02-07-2008 |
| | | US 2009117154 A1 | 07-05-2009 |
| | | WO 2007033329 A1 | 22-03-2007 |
| EP 2594575 A1 | 22-05-2013 | CN 104039804 A | 10-09-2014 |
| | | EP 2594575 A1 | 22-05-2013 |
| | | EP 2780352 A1 | 24-09-2014 |
| | | ES 2657752 T3 | 06-03-2018 |
| | | JP 6295202 B2 | 14-03-2018 |
| | | JP 2015501796 A | 19-01-2015 |
| | | US 2014349952 A1 | 27-11-2014 |
| | | WO 2013072355 A1 | 23-05-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- Remington's Pharmaceutical Sciences. Mack Publishing Company, 1990, 1445
- **Z. KADLECOVA et al.** *Biomacromolecules,* 2012, vol. 13, 3127-3137
- **G. THOMA ; J. T. PATTON ; J. L. MAGNANI ; B. ERNST ; R. OHRLEIN ; R. O. DUTHALER.** *J. Am. Chem. Soc.,* 1999, vol. 121, 5919-5929
- **WU, L. ; MARTIN, T. D. ; CARRINGTON, M. ; KEWALRAMANI, V. N.** Raji B Cells, Misidentified as THP-1 Cells, Stimulate DC-SIGN-Mediated HIV Transmission. *Virology,* 2004, vol. 318 (1), 17-23
- **BINLEY, J. M. ; NGO-ABDALLA, S. ; MOORE, P. ; BOBARDT, M. ; CHATTERJI, U. ; GALLAY, P. ; BURTON, D. R. ; WILSON, I. A. ; ELDER, J. H. ; DE PARSEVAL, A.** Inhibition of HIV Env Binding to Cellular Receptors by Monoclonal Antibody 2G12 as Probed by Fc-Tagged Gp120. *Retrovirology,* 2006, vol. 3, 39
- **SCHUCK, P.** Size-distribution analysis of macromolecules by sedimentation velocity ultracentrifugation and Lamm equation modeling. *Biophysical Journal,* 2000, vol. 78 (3), 1606-1619
- **LAUE TM ; SHAH BD ; RIDGEWAY TM ; PELLETIER SL.** Analytical Ultracentrifugation in Biochemistry and Polymer Science. The Royal Society of Chemistry, 1992, 90-125
- Calculation of the partial volume of organic compounds and polymers. **DURCHSCHLAG, H. ; ZIPPER, P.** Ultracentrifugation. Springer, 1994, 20-39
- **BRAUTIGAM, C. A.** Calculations and Publication-Quality Illustrations for Analytical Ultracentrifugation Data. *Methods Enzymol,* 2015, vol. 562, 109-133
- **BROWN, P. H. ; BALBO, A. ; SCHUCK, P.** Characterizing Protein-Protein Interactions by Sedimentation Velocity Analytical Ultracentrifugation. *Current Protocols in Immunology,* 2008, vol. 81 (1), 18.15.1-18.15.39
- **HOPPE, C. A. ; LEE, Y. C.** Accumulation of a Nondegradable Mannose Ligand within Rabbit Alveolar Macrophages. Receptor Reutihzation Is Independent of Ligand Degradation. *Biochemistry,* 1984, vol. 23 (8), 1723-1730